# EUROPÄISCHE PATENTSCHRIFT

(11) **EP 1 727 799 B1**
(45) Veröffentlichungstag und Bekanntmachung des Hinweises auf die Patenterteilung: **07.01.2009**
(21) Anmeldenummer: 05715811.5
(22) Anmeldetag: 05.03.2005
(51) Int. Cl.: C07D 209/34, C07D 401/12, C07D 405/06, C07D 405/14, C07D 403/06, C07D 401/06, A61K 31/501, A61P 25/28

(54) **NEUE ALKYL-HALTIGE 5-ACYLINDOLINONE, DEREN HERSTELLUNG UND DEREN VERWENDUNG ALS ARZNEIMITTEL**
NOVEL ALKYL-CONTAINING 5-ACYLINDOLINONES, THEIR PREPARATION AND THEIR USE AS PHARMACEUTICAL PRODUCTS
NOUVELLES 5-ACYLINDOLINONES A TENEUR EN ALKYLE, LEUR PREPARATION ET LEUR UTILISATION COMME PRODUITS PHARMACEUTIQUES

(30) Priorität: 12.03.2004 DE 102004012068
(43) Veröffentlichungstag der Anmeldung: 06.12.2006
(73) Patentinhaber: Boehringer Ingelheim International GmbH, 55216 Ingelheim am Rhein (DE); Boehringer Ingelheim Pharma GmbH & Co. KG, 55216 Ingelheim am Rhein (DE)
(72) Erfinder: HECKEL, Armin, 88400 BIBERACH (DE); ROTH, Gerald Jürgen, 88400 BIBERACH (DE); KLEY, Jörg, 88441 Mittelbiberach (DE); HOERER, Stefan, D-88416 OCHSENHAUSEN (DE); UPHUES, Ingo, 88444 UMMENDORF (DE)
(74) Vertreter: Hammann, Heinz
(86) Internationale Anmeldenummer: PCT/EP2005/002406
(87) Internationale Veröffentlichungsnummer: WO 2005/087727

(56) Entgegenhaltungen:
- WO-A-01/27080

## Beschreibung

Gegenstand der vorliegenden Erfindung sind neue alkyl-haltige 5-Acylindolinone der allgemeinen Formel 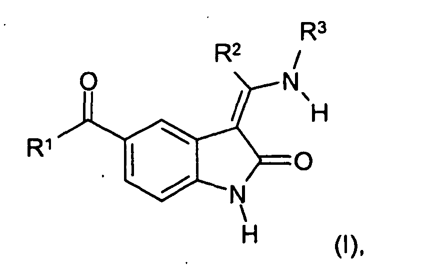 deren Tautomere, deren Enantiomere, deren Diastereomere, deren Gemische und deren Salze, insbesondere deren physiologisch verträgliche Salze mit anorganischen oder organischen Säuren oder Basen, welche wertvolle pharmakologische Eigenschaften aufweisen, beispielsweise eine Hemmwirkung auf Proteinkinasen, insbesondere eine Hemmwirkung auf die Aktivität der Glykogen-Synthase-Kinase (GSK-3) deren Herstellung, deren Verwendung zur Prävention oder Behandlung von Krankheiten oder Zuständen, die in Zusammenhang mit einer veränderten GSK-3 Aktivität stehen insbesondere von Diabetes mellitus Typ I und Typ II, Diabetes assoziierte Störungen wie diabetische Neuropathie, degenerativer neurologischer Erkrankungen wie Alzheimers Erkrankung, Schlaganfall, neurotraumatische Verletzungen, bipolare Störungen, die eine Verbindung der allgemeinen Formel (I) oder ein physiologisch verträgliches Salz davon enthaltenden Arzneimittel sowie Verfahren zu deren Herstellung.

In 5-Stellung substituierte Indolinonderivate, welche die Proliferation von Tumorzellen hemmen, werden in der WO 01/27080 beschrieben.

In der obigen Formel I bedeuten
R¹ eine lineare oder verzweigte C₁₋₅-Alkylgruppe, in der die Wasserstoffatome ganz oder teilweise durch Fluoratome ersetzt sein können, oder eine gegebenenfalls durch ein Fluor-, Chlor- oder Bromatom substiuierte Arylgruppe,
wobei unter einer Arylgruppe eine Phenyl- oder Naphthylgruppe zu verstehen ist,
R² eine C₁₋₇-Alkyl- oder C₃₋₇-Cycloalkylgruppe,
eine gegebenenfalls durch ein oder zwei Fluor-, Chlor-, Brom- oder Iodatome oder ein oder zwei Nitro-, Cyano-, Amino-, C₁₋₃-Alkyl- oder C₁₋₃-Alkoxygruppen substituierte 5- oder 6-gliedrige Heteroarylgruppe mit ein bis drei Heteroatomen ausgewählt aus der Gruppe N, S und O, wobei sowohl die Heteroatome als auch die Substituenten gleich oder verschieden sein können,
eine Phenylgruppe, in der zwei benachbarte Kohlenstoffatome über eine Methylendioxy-, Ethylendioxy- oder Difluormethylendioxygruppe miteinander verknüpft sind,
eine Phenylgruppe, an die ein weiterer Phenylring oder ein 5- oder 6-gliedriger heteroaromatischer Ring mit ein bis drei Heteroatomen ausgewählt aus der Gruppe N, S und O, wobei die Heteroatome gleich oder verschieden sein können, und wobei der Bicyclus durch ein oder zwei Fluor-, Chlor-, Brom- oder Iodatome oder ein oder zwei Nitro-, Cyano-, Amino-, C₁₋₃-Alkyl- oder C₁₋₃-Alkoxygruppen substituiert sein kann und die Substituenten gleich oder verschieden sein können, anneliert ist, oder
eine Phenylgruppe, die durch ein bis drei Fluor-, Chlor-, Brom- oder Iodatome oder durch eine bis drei C₁₋₃-Alkyl-, Nitro-, Cyano-, Amino-, Di-(C₁₋₃-alkyl)-amino-, C₁₋₃-Alkyl-carbonylamino-, Phenylcarbonylamino-, C₁₋₃-Alkylsulfonylamino-, Arylsulfonylamino-, Trifluormethyl-, C₁₋₃Alkylsulfonyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylaminocarbonyl-, Hydroxycarbonyl-C₁₋₃-alkyl-aminocarbonyl-, C₁₋₃-Alkoxycarbonyl-C₁₋₃-alkyl-aminocarbonyl-, Di-(C₁₋₃-alkyl)-amino-C₁₋₃-alkylaminocarbonyl-, Di-(C₁₋₃-alkyl)-amino-carbonyl-C₁₋₃-alkoxy-, C₁₋₃-Alkyl-amino-carbonyl-C₁₋₃-alkoxy-, Carboxy-C₁₋₃-alkoxy-, C₁₋₃-Alkyloxy-carbonyl-C₁₋₃-alkoxy-, Carboxy-C₁₋₃-alkyl-, C₁₋₃Alkoxy-carbonyl-C₁₋₃-alkyl, C₁₋₃-Alkoxy-carbonylamino-C₁₋₃-alkyl-, Amino-C₁₋₃-alkyl-, Di-(C₁₋₃-alkyl)-amino-C₁₋₃-alkyl-, C₁₋₃-Alkyl-carbonylamino-C₁₋₃-alkyl-, Phthalimido-, Pyrrolyl- oder Mono- oder Di-(C₁₋₃-alkyl)-pyrrolylgruppen substituiert sei n kann, wobei die Substituenten gleich oder verschieden sind, und
R³ ein Wasserstoffatom,
eine lineare oder verzweigte C₁₋₆-Alkylgruppe, die durch eine bis drei Carboxy-, C₁₋₄-Alkoxy-carbonyl-, Aminocarbonyl-, C₁₋₃-Alkylaminocarbonyl-, Di-(C₁₋₃-alkyl)-aminocarbonyl-, Di-(C₁₋₃-alkyl)-amino-(C₁₋₃-alkyl)-amino-carbonyl-, N-[Di-(C₁₋₃-alkyl)-amino-(C₁₋₃-alkyl)]-N-(C₁₋₃-alkyl)-amino-carbonyl-, Arylaminocarbonyl-, Heteroarylaminocarbonyl-, Aryl-C₁₋₃-alkyl-aminocarbonyl-, Heteroaryl-C₁₋₃-alkyl-aminocarbonyl-, N-(Aryl)-N-(C₁₋₃-alkyl)-aminocarbonyl-, N-(Heteroaryl)-N-(C₁₋₃-alkyl)-aminocarbonyl-, Di-(ω-hydroxy-C₂₋₃-alkyl)-aminocarbonyl-, Aryl- oder Heteroarylgruppen oder durch eine 5 bis 7-gliedrige Cycloalkylenimino- oder Cycloalkyleniminocarbonylgruppe substituiert sein kann,
wobei in der oben erwähnten Cycloalkylenimino- oder Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
eine lineare oder verzweigte C₂₋₆-Alkylgruppe, die ab Position 2 durch eine bis drei Hydroxy-, C₁₋₃-Alkoxy-, Aryloxy-, Heteroaryloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, ω-Hydroxy-C₂₋₃-alkoxy-, Amino-, C₁₋₃-Alkylamino-, Di-(C₁₋₃-alkyl)-amino-, C₁₋₃-Alkyl-carbonylamino-, Heteroaryl-carbonylamino-, N-(C₁₋₃-Alkyl)-N-(C₁₋₃-alkyl-carbonyl)-amino-, N-(C₁₋₃-Alkyl)-N-(heteroaryl-carbonyl)-amino-, C₁₋₄-Alkoxy-carbonylamino-, N-(C₁₋₃-Alkyl)-N-(C₁₋₄-alkoxy-carbonyl)-amino-, Arylcarbonylamino-, N-(C₁₋₃-Alkyl)-N-(arylcarbonyl)-amino-, (ω-Hydroxy-C₂₋₃-alkyl)-amino-, Di-(ω-hydroxy-C₂₋₃-alkyl)-amino-, Arylamino-, Heteroarylamino-, C₁₋₃-Alkylsulfonylamino-, N-(C₁₋₃-Alkyl)-N-(C₁₋₃-alkylsulfonyl)-amino-, C₁₋₃-Alkoxy-carbonyl-C₁₋₃-alkyl-carbonylamino-, (C₁₋₃-Alkyl-amino)-carbonyl-amino-, [Di-(C₁₋₃-alkyl)-amino]-carbonyl-amino-, (C₁₋₃-Alkyl-amino)-C₁₋₃-alkyl-carbonyl-amino-, [Di-(C₁₋₃-alkyl)-amino]-C₁₋₃-alkyl-carbonyl-amino-, N-(Aryl-C₁₋₃-alkyl-carbonyl)-N-(C₁₋₃-alkyl)-amino-, N-(C₁₋₃-Alkyl)-N-[(C₁₋₃-alkyl-amino)-carbonyl]-amino- oder N-(C₁₋₃-Alkyl)-N-{[di-(C₁₋₃-alkyl)-amino]-carbonyl}-aminogruppe substituiert ist und gegebenenfalls zusätzlich ab Position 1 durch eine Carboxy-, C₁₋₄-Alkoxy-carbonyl-, C₁₋₃-Alkylaminocarbonyl-, Di-(C₁₋₃-alkyl)-aminocarbonyl-, Arylaminocarbonyl-, Heteroarylaminocarbonyl-, Di-(ω-hydroxy-C₂₋₃-alkyl)-aminocarbonyl-, Aryl- oder Heteroarylgruppen oder durch eine 5 bis 7-gliedrige Cycloalkylenimino- oder Cycloalkyleniminocarbonylgruppe substituiert sein kann,
wobei in der oben erwähnten Cycloalkylenimino- oder Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
eine C₂₋₄-Alkenyl- oder C₂₋₄-Alkinylgruppe, wobei sich zwischen dem Stickstoffatom, an das R³ gebunden ist, und der Mehrfachbindung mindestens ein sp³-hybridisiertes Kohlenstoffatom befindet und die Alkenyl- oder Alkinylgruppe durch eine bis drei C₁₋₃-Alkylgruppen substituiert sein kann,
eine C₁₋₅-Alkylgruppe, in der Methylengruppe, die dem Stickstoffatom, an das R³ gebunden ist, benachbart ist, durch eine -NH-, oder eine -N(C₁₋₃-Alkyl)- Gruppe oder durch ein Sauerstoffatom ersetzt sein können,
eine C₁₋₄-Alkyloxygruppe oder
eine Aminogruppe, die durch eine oder zwei C₁₋₃-Alkyl-, Aryl-, Aryl-C₁₋₃-alkyl-, C₁₋₃-Alkyl-carbonyl-, C₁₋₄-Alkyloxy-carbonyl-,Arylcarbonyl- oder Aryl-C₁₋₃-alkyl-carbonylgruppen substituiert sein kann, wobei die Substituenten gleich oder verschieden sein können,
wobei die vorstehend erwähnten Alkylgruppen geradkettig oder verzweigt sein können,
deren Tautomere, Enantiomere, Diastereomere, deren Gemische und deren Salze.

Soweit nichts anderes erwähnt wurde, ist unter einer Heteroarylgruppe oder einem heteroaromatischen Ring bevorzugterweise eine Furanyl-, Thiophenyl-, Pyrrolyl-, Pyrazolyl-, Thiazolyl-, Imidazolyl-, Oxazolyl-, Triazolyl-, Thiadiazolyl-, Pyridinyl-, Pyrimidinyl- oder Pyrazinylgruppe zu verstehen, welche durch ein Fluor-, Chlor-, Brom- oder lodatom oder eine Amino-, Nitro-, Cyano-, C₁₋₃-Alkyl-, C₁₋₃-Alkyloxy-, Di-(C₁₋₃-alkyl)-amino-C₁₋₃-alkyloxy-, oder Trifluormethylgruppe mono-, di- oder trisubstituiert sein kann, wobei die Substituenten gleich oder verschieden sind, und an welche über zwei benachbarte Atome ein Phenylring ankondensiert sein kann.

Unter einer Arylgruppe, ist, soweit nicht anderes erwähnt wird, eine Phenyl- oder Naphthylgruppe zu verstehen; bevorzugt ist die Phenylgruppe. Sofern nichts anderes erwähnt wurde, kann eine solche Arylgruppe durch eine Nitro-, Cyano-, C₁₋₃-Alkyloxy-, Di-(C₁₋₃-alkyl)-amino-C₁₋₃-alkyloxy-, Amino-C₁₋₃₋alkyl-, C₁₋₃-Alkyl-amino-C₁₋₃-alkyl-, Di-(C₁₋₃-alkyl)-amino-C₁₋₃-alkyl- oder C₁₋₄-Alkoxy-carbonylamino-C₁₋₃-alkylgruppe substituiert sein kann.

Bevorzugt sind diejenigen Verbindungen der allgemeinen Formel I, in denen
R² und R³ wie vorstehend erwähnt definiert sind und
R¹ eine Methyl-, Ethyl-, n-Propyl-, Isopropyl-, n-Pentyl-, Trifluormethyl- oder Phenylgruppe bedeutet,
deren Tautomere, Enantiomere, Diastereomere, deren Gemische und deren Salze.

Besonders bevorzugt sind diejenigen Verbindungen der allgemeinen Formel I, in denen
R¹ eine Methyl- oder Ethylgruppe,
R² eine Ethyl-, Propyl-, Butyl- oder Pentylgruppe,
eine Pyridinyl-, Furanyl- oder Pyrazinylgruppe,
eine Phenylgruppe, in der zwei benachbarte Kohlenstoffatome über eine Methylendioxy-, Ethylendioxy- oder Difluormethylendioxygruppe miteinander verknüpft sind, oder
eine Phenylgruppe, die durch ein oder zwei Fluor-, Chlor-, Brom- oder lodatome oder durch eine oder zwei C₁₋₃-Alkyl-, Nitro-, Cyano-, Amino-, C₁₋₃-Alkylcarbonylamino-, Phenylcarbonylamino-, C₁₋₃-Alkylsulfonylamino-, Trifluormethyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylaminocarbonyl-, Hydroxycarbonyl-C₁₋₃-alkyl-aminocarbonyl-, C₁₋₃-Alkoxycarbonyl-C₁₋₃-alkylaminocarbonyl-, Di-(C₁₋₃-alkyl)-amino-C₁₋₃-alkylaminocarbonyl-, Carboxy-C₁₋₃-alkyl-, C₁₋₃-Alkoxy-carbonyl-C₁₋₃-alkyl-, Amino-C₁₋₃-alkyl- oder C₁₋₃-Alkyl-carbonylamino-C₁₋₃-alkylgruppen substituiert sein kann, wobei die Substituenten gleich oder verschieden sind, und
R³ ein Wasserstoffatom,
eine lineare oder verzweigte C₁₋₆-Alkylgruppe, die durch eine Carboxy-, C₁₋₄-Alkoxycarbonyl-, Phenyl-, Pyridinyl-, Indolyl-, Imidazolyl-, C₁₋₃-Alkylaminocarbonyl-, Di-(C₁₋₃-alkyl)-aminocarbonyl-, Phenylaminocarbonyl-, Pyridinylami nocarbonyl-, Di-(w-hydroxy-C₂₋₃-alkyl)-aminocarbonyl- oder durch eine 5 bis 7-gliedrige Cycloalkylenimino- oder Cycloalkyleniminocarbonylgruppe substituiert sein kann,
wobei die oben erwähnte Phenylgruppe gegebenenfalls durch Nitro-, Cyano-, C₁₋₃-Alkyloxy-, [Di-(C₁₋₃-alkyl)-amino]-C₁₋₃-alkyloxy-, eine Amino-C₁₋₃-alkyl- oder C₁₋₄-Alkoxy-carbonylamino-C₁₋₃-alkylgruppe substituiert sein kann und
wobei in der oben erwähnten Cycloalkylenimino- und Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
eine lineare oder verzweigte C₂₋₆-Alkylgruppe, die ab Position 2 durch eine Hydroxy-, C₁₋₃-Alkoxy-, ω-Hydroxy-C₂₋₃-alkoxy-, Amino-, C₁₋₃-Alkylamino-, Di-(C₁₋₃-alkyl)-amino-, C₁₋₃-Alkyl-carbonylamino-, N-(C₁₋₃-Alkyl)-N-(C₁₋₃-alkyl-carbonyl)-amino-, C₁₋₄-Alkoxycarbonylamino-, N-(C₁₋₃-Alkyl)-N-(C₁₋₄-alkoxy-carbonyl)-amino-, Phenylcarbonylamino-, N-(C₁₋₃-Alkyl)-N-(phenylcarbonyl)-amino-, Di-(ω-hydroxy-C₂₋₃-alkyl)-amino-, Pyridinylamino-, Nitro-pyridinyl-amino-, Chlor-trifluormethyl-pyridinylamino-, C₁₋₃-Alkylsulfonylamino-, N-(C₁₋₃-Alkyl)-N-(C₁₋₃-alkylsulfonyl)-amino- oder C₁₋₃-Alkoxycarbonyl-C₁₋₃-alkyl-carbonylaminogruppe substiuiert ist und gegebenenfalls zusätzlich ab Position 1 durch eine Carboxy-, C₁₋₄-Alkoxy-carbonyl-, Phenyl-, Pyridinyl-, Imidazolyl-, C₁₋₃-Alkylaminocarbonyl-, Di-(C₁₋₃-alkyl)-aminocarbonyl-, Phenylaminocarbonyl-, Di-(ω-hydroxy-C₂₋₃-alkyl)-aminocarbonyl- oder durch eine 5 bis 7-gliedrige Cycloalkylenimino- oder Cycloalkyleniminocarbonylgruppe substituiert sein kann,
wobei in der oben erwähnten Cycloalkylenimino- oder Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
und wobei die oben erwähnten Phenylgruppen gegebenenfalls durch eine Amino-C₁₋₃-alkyl- oder C₁₋₄-Alkoxy-carbonylamino-C₁₋₃-alkylgruppe substituiert sein können, oder
oder eine Propenyl- oder Propinylgruppe bedeuten,
insbesondere jedoch diejenigen Verbindungen, in denen
R¹ eine Methylgruppe,
R² eine Ethyl-, Propyl-, Butyl- oder Pentylgruppe,
eine Phenylgruppe, in der zwei benachbarte Kohlenstoffatome über eine Methyl endioxy-, Ethylendioxy- oder Difluormethylendioxygruppe miteinander verknüpft sind, oder
eine Phenylgruppe, die durch ein oder zwei Fluor-, Chlor- oder Bromatome oder durch eine oder zwei Trifluormethyl-, Nitro-, Cyano-, C₁₋₃-Alkoxy-, Carboxy-C₁₋₃-alkyl-, C₁₋₃-Alkoxy-carbonyl-C₁₋₃-alkyl-, Amino-C₁₋₃-alkyl- oder C₁₋₃-Alkyl-carbonylamino-C₁₋₃-alkyl-gruppen substituiert sein kann, wobei die Substituenten gleich oder verschieden sind, und
R³ eine lineare oder verzweigte C₁₋₅-Alkylgruppe, die durch eine Carboxy-, C₁₋₄-Alkoxy-carbonyl-, C₁₋₃-Alkylaminocarbonyl-, Di-(C₁₋₃-alkyl)-aminocarbonyl-, Phenylaminocarbonyl-, Pyridinylaminocarbonyl-, Di-(2-hydroxy-ethyl)-aminocarbonyl-, Piperazinylcarbonyl-, 4-(C₁₋₃-Alkyl)-piperazinyl-carbonyl-, 4-(C₁₋₄-Alkoxy-carbonyl)-piperazinyl-carbonyl-, Phenyl-, Pyridinyl- oder Imidazolylgruppe substituiert sein kann,
wobei die Phenylgruppe gegebenenfalls durch eine Nitro-, Cyano-, C₁₋₃-Alkyloxy-, [Di-(C₁₋₃-alkyl)-amino]-C₁₋₃-alkyloxy-, Amino-C₁₋₃-alkyl- oder C₁₋₄-Alkoxy-carbonylamino-C₁₋₃-alkylgruppe substituiert sein kann,
eine C₂₋₅-Alkylgruppe, die endständig durch eine Hydroxy-, C₁₋₃-Alkoxy-, Phenyloxy-, 2-Hydroxy-ethoxy-, Amino-, C₁₋₃-Alkylamino-, Di-(C₁₋₃-alkyl)-amino-, C₁₋₃-Alkylcarbonylamino-, N-(C₁₋₃-Alkyl)-N-(C₁₋₃-alkyl-carbonyl)-amino-, C₁₋₄-Alkoxycarbonylamino-, N-(C₁₋₃-Alkyl)-N-(C₁₋₄-alkoxy-carbonyl)-amino-, Phenylcarbonylamino-, N-(C₁₋₃-Alkyl)-N-(phenylcarbonyl)-amino-, Pyridinylamino-, Nitro-pyridinylamino-, Di-(2-hydroxy-ethyl)-amino-, 3-Chlor-5-trifluormethyl-pyridin-2-yl-amino-, C₁₋₃-Alkylsulfonylamino-, N-(C₁₋₃-Alkyl)-N-(C₁₋₃-alkylsulfonyl)-amino-, C₁₋₃-Alkoxy-carbonyl-C₁₋₃-alkyl-carbonylamino-, Indolyl-, Pyrrolidinyl-, 2-Oxo-pyrrolidinyl-, Morpholinyl-, Piperazinyl- oder 4-(C₁₋₃-Alkyl)-piperazinylgruppe substituiert ist und gegebenenfalls zusätzlich ab Position 1 durch eine Carboxy-, C₁₋₄-Alkoxy-carbonyl-, C₁₋₃-Alkylamino-carbonyl-, Di-(C₁₋₃-alkyl)-aminocarbonyl-, Phenylaminocarbonyl-, Di-(2-hydroxyethyl)-aminocarbonyl-, Piperazinylcarbonyl-, 4-(C₁₋₃-Alkyl)-piporazinyl-carbonyl-, 4-(C₁₋₄-Alkoxy-carbonyl)-piperazinyl-carbonyl-, Phenyl-, Pyridinyl-oder Imidazolylgruppe substituiert sein kann, oder
eine Aminogruppe, die durch eine oder zwei C₁₋₃-Alkyl-, Aryl-, Aryl-C₁₋₃-alkyl-, C₁₋₃-Alkyl-carbonyl-, C₁₋₄-Alkyloxy-carbonyl-,Arylcarbonyl- oder Aryl-C₁₋₃-alkyl-carbonylgruppen substituiert sein kann, wobei die Substituenten gleich oder verschieden sein können, bedeuten,
wobei die vorstehend erwähnten Alkylgruppen geradkettig oder verzweigt sein können,
deren Tautomere, Enantiomere, Diastereomere, deren Gemische und deren Salze.

Ganz besonders bevorzugt sind diejenigen Verbindungen der allgemeinen Formel I, in denen
R¹ eine Methylgruppe,
R² eine Ethyl-, Propyl-, Butyl- oder Pentylgruppe,
eine Phenylgruppe, in der zwei benachbarte Kohlenstoffatome über eine Methylendioxy- oder Ethylendioxygruppe miteinander verknüpft sind, oder
eine Phenylgruppe und
R³ eine C₁₋₄-Alkylgruppe, die endständig durch eine C₁₋₄-Alkoxy-carbonylgruppe substituiert sein kann, oder
eine C₂₋₄-Alkylgruppe, die endständig durch eine C₁₋₃-Alkyl-carbonylamino-, Phenylcarbonylamino-, Di-(C₁₋₃-alkyl)-amino-, Phenyl-, Pyridinyl- oder C₁₋₃-Alkylsulfonylaminogruppe substituiert ist, bedeuten,
wobei die vorstehend erwähnten Alkylgruppen geradkettig oder verzweigt sein können,
deren Tautomere, Enantiomere, Diastereomere, deren Gemische und deren Salze;
insbesondere sind folgende Verbindungen der allgemeinen Formel I zu nennen:
(a) 5-Acetyl-3-[3-(methoxycarbonylpropylamino)-(benzo-[1,3]dioxol-5-yl)-methyliden]-2-indolinon 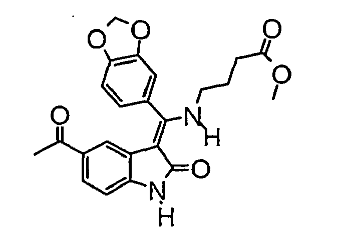
(b) 5-Acetyl-3-[isopropylamino-phenyl-methyliden]-2-indolinon 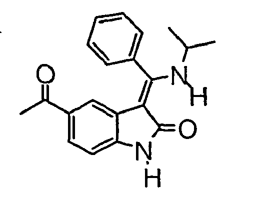
(c) 5-Acetyl-3-[propylamino-phenyl-methyliden]-2-indolinon 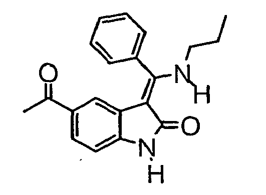
(d) 5-Acetyl-3-[(3-methoxycarbonyl-propylamino)-(2,3-dihydro-benzo[1,4]dioxin-6-yl)-methyliden]-2-indolinon 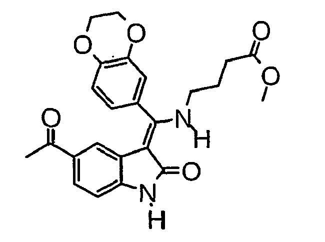
(e) 5-Acetyl-3-[(benzo[1,3]dioxol-5-yl)-(3-(benzoylamino-propylamino)-methyliden]-2-indolinon 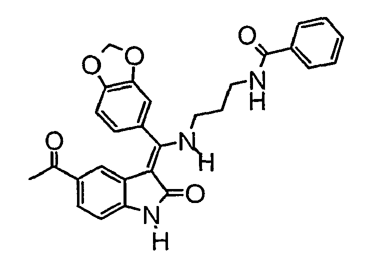
(f) 5-Acetyl-3-[(benzo[1,3]dioxol-5-yl)-(3-(butyrylamino-propylamino)-methyliden]-2-indolinon 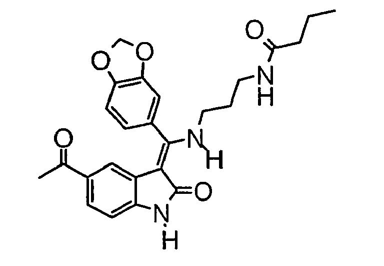
(g) 5-Acetyl-3-[(3-methansulfonylamino-propylamino)-phenyl-methyliden]-2-indolinon 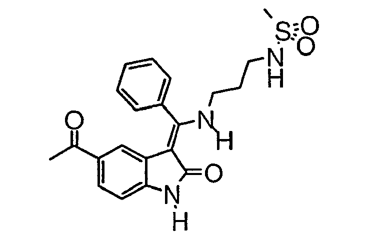
(h) 5-Acetyl-3-[1-(3,4-difluorphenyl)-1-(N',N'-dimethylhydrazino)-methyliden]-2-indolinon 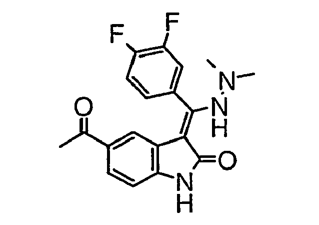
(i) 5-Acetyl-3-[1-(4-dimethylamino-butyl)-butenyliden]-2-indolinon 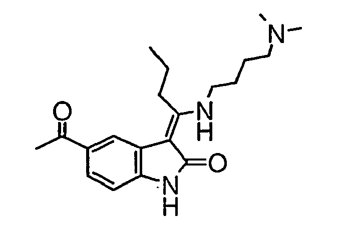
(j) 5-Acetyl-3-[1-phenyl-1-(3-pyridin-3-yl-propylamino)-methyliden]-2-indolinon 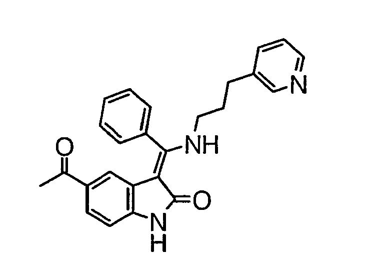
sowie deren Tautomere, Enantiomere, Diastereomere, deren Gemische und deren Salze.

Erfindungsgemäß erhält man die Verbindungen der allgemeinen Formel I nach an sich bekannten Verfahren, beispielsweise nach folgenden Verfahren:
a) Umsetzung einer Verbindung der allgemeinen Formel 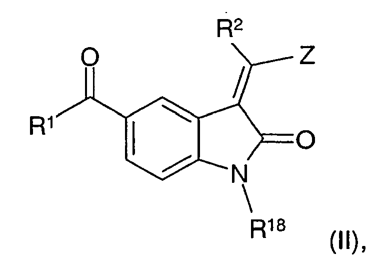 in der R¹ und R² wie eingangs erwähnt definiert sind,
   R¹⁸ ein Wasserstoffatom oder eine Schutzgruppe für das Stickstoffatom der Lactamgruppe und
   Z eine Austrittsgruppe wie etwa ein Halogenatom, eine Hydroxy-, Alkoxy-, Alkylsulfonyl-, Trialkylsilyloxy-, Alkyl-arylsulfonyl-, oder Aryl-alkoxygruppe, z.B. ein Chlor- oder Bromatom, eine Methoxy-, Ethoxy-, Methansulfonyl-, Trimethylsilyloxy-, Toluolsulfonyl- oder Benzyloxygruppe, bedeuten,
   mit einem Amin der allgemeinen Formel

   R³-NH₂ (III),

   in der R³ wie eingangs erwähnt definiert ist,
   wobei eventuell in den Resten R² und/oder R³ enthaltene Hydroxy-, Amino- oder Iminogruppen vorübergehend durch geeignete Schutzgruppen geschützt werden können.
   Als Schutzgruppe für das Stickstoffatom der Lactamgruppe kommt beispielsweise eine Acetyl-, Benzoyl-, Ethoxycarbonyl-, tert.Butyloxycarbonyl- oder Benzyloxycarbonylgruppe in Betracht.
   Die Umsetzung wird zweckmäßigerweise in einem Lösungsmittel wie Dimethylformamid, Toluol, Acetonitril, Tetrahydrofuran, Dimethylsulfoxid, Methylenchlorid oder deren Gemischen gegebenenfalls in Gegenwart einer inerten Base wie Triethylamin, N-Ethyl-diisopropylamin oder Natriumhydrogencarbonat bei Temperaturen zwischen 20 und 175°C durchgeführt, wobei eine verwendete Schutzgruppe infolge Umamidierung gleichzeitig abgespalten werden kann.
   Bedeutet Z in einer Verbindung der allgemeinen Formel II ein Halogenatom, dann wird die Umsetzung vorzugsweise in Gegenwart einer inerten Base bei Temperaturen zwischen 20 und 120°C, durchgeführt.
   Bedeutet Z in einer Verbindung der allgemeinen Formel II eine Hydroxy-, Alkoxy- oder Arylalkoxygruppe, dann wird die Umsetzung vorzugsweise bei Temperaturen zwischen 20 und 200°C, durchgeführt.
   Die gegebenenfalls erforderliche anschließende Abspaltung einer verwendeten Schutzgruppe wird zweckmäßigerweise entweder hydrolytisch in einem wäßrigen oder alkoholischen Lösungsmittel, z.B. in Methanol/Wasser, Ethanol/Wasser, Isopropanol/Wasser, Tetrahydrofuran/Wasser, Dioxan/Wasser, Dimethylformamid/Wasser, Methanol oder Ethanol in Gegenwart einer Alkalibase wie Lithiumhydroxid, Natriumhydroxid oder Kaliumhydroxid bei Temperaturen zwischen 0 und 100°C, vorzugsweise bei Temperaturen zwischen 10 und 50°C,
   oder vorteilhafterweise durch Umamidierung mit einer organischen Base wie Ammoniak, Butylamin, Dimethylamin oder Piperidin in einem Lösungsmittel wie Methanol, Ethanol, Dimethylformamid und deren Gemischen oder in einem Überschuß des eingesetzten Amins bei Temperaturen zwischen 0 und 100°C, vorzugsweise bei Temperaturen zwischen 10 und 50°C, durchgeführt.
b) zur Herstellung einer Verbindung der Formel I, die eine Aminocarbonylgruppe enthält: Umsetzung einer Verbindung, die eine Carboxygruppe enthält, mit dem entsprechenden Amin zur entsprechenden Aminocarbonylverbindung;
c) zur Herstellung einer Verbindung der Formel I, die eine Carbonylaminogruppe enthält: Umsetzung einer Verbindung, die eine Aminogruppe enthält, mit dem entsprechenden Säurechlorid zur entsprechenden Carbonylaminoverbindung;
d) zur Herstellung einer Verbindung der Formel I, die eine Aminomethylgruppe enthält: Hydrierung einer Verbindung, die eine Cyanogruppe enthält, zu dem entsprechenden Aminomethylderivat;
e) zur Herstellung einer Verbindung der Formel I, die eine Aminogruppe enthält: Reduktion einer Verbindung, die eine Nitrogruppe enthält.

Anschließend können gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.

Ferner können die erhaltenen Verbindungen der allgemeinen Formel I, wie bereits eingangs erwähnt wurde, in ihre Enantiomeren und/oder Diastereomeren aufgetrennt werden. So können beispielsweise cis-/trans-Gemische in ihre cis- und trans-Isomere, und Verbindungen mit mindestens einem optisch aktiven Kohlenstoffatom in ihre Enantiomeren aufgetrennt werden.

So lassen sich beispielsweise die erhaltenen cis-/trans-Gemische durch Chromatographie in ihre cis- und trans-Isomeren, die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestens 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-O-p-toluoyl-weinsäure, Äpfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise (+)-oder (-)-Menthyloxycarbonyl in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Die als Ausgangsstoffe verwendeten Verbindungen der allgemeinen Formeln II bis III sind entweder literaturbekannt oder man erhält diese nach an sich literaturbekannten Verfahren (siehe Beispiele I bis VIII).

Wie bereits eingangs erwähnt, weisen die erfindungsgemäßen Verbindungen der allgemeinen Formel I und ihre physiologisch verträglichen Salze wertvolle pharmakologische Eigenschaften auf, insbesondere eine Hemmwirkung auf das Enzym GSK-3.

Glykogen Synthase Kinase-3 (GSK-3) ist eine Serin/Threonin-Kinase die in zwei Isoformen, GSK-3α und GSK-3β, existiert. GSK-3 phosphoryliert und inaktiviert die Glykogensynthase, ein Schlüsselenzym der insulinabhängigen Regulation der Glykogensynthese (Embi et al., Eur. J. Biochem. 107, 519-527, (1980)), aber in vitro auch eine Vielzahl weiterer regulatorischer Proteine. Zu diesen Proteinen gehören das Mikrotubulus assoziierte Protein Tau, Elongation initiation factor 2b (eIF2b), β-Catenin, Axin, ATP-Citratlyase, Heat-shock-factor 1, c-Jun, c-myc, c-myb, CREB und CEBPα. Diese verschiedenen Substrate implizieren eine Rolle für GSK-3 in vielen Bereichen des zellulären Metabolismus, der Proliferation, der Differenzierung und der Entwicklung.

Typ 2 Diabetes wird durch eine Insulinresistenz in verschiedenen Geweben wie Skelettmuskel, Leber und Fettgewebe und durch eine veränderte Insulinsekretion der Bauchspeicheldrüse gekennzeichnet. Die Speicherung von Glykogen in Leber und Muskel ist von großer Bedeutung für die Aufrechterhaltung des Glukosegleichgewichts. Im Typ 2 Diabetes ist die Aktivität der Glykogensynthase vermindert und somit die Rate der Glykogensynthese reduziert. Außerdem konnte gezeigt werden, dass GSK-3 im Typ 2 diabetischen Muskel verstärkt exprimiert wird und dass somit eine vermehrte GSK-3 Aktivität mit einer verminderten Glykogensyntheserate einhergeht (Nikoulina et al., Diabetes 49, 263-271, (2000)). Eine Hemmung der GSK-3 Aktivität stimuliert die Glykogensynthase, verstärkt somit die Glykogensynthese und führt letztlich zu einer Reduktion der Glukosespiegel. Eine GSK-3 Hemmung ist daher von therapeutischer Relevanz für die Behandlung von Typ 1 und Typ 2 Diabetes sowie von diabetischer Neuropathie.

Die Alzheimersche Erkrankung ist dadurch gekennzeichnet, dass das Mikrotubulus assoziierte Protein Tau übermäßig stark phosphoryliert vorliegt (Cohen & Frame, Nature Reviews: Molecular Cell Biology, 2, 1-8, (2001)). GSK-3 phosphoryliert viele dieser Phosphorylierungsstellen von Tau in vitro, wodurch die Bindung an Mikrotubuli verhindert wird. Hierdurch steht Tau für eine vermehrte Filamentassemblierung zur Verfügung, die in der Alzheimerschen Erkrankung und anderen neurologischen Erkrankungen der neuronalen Degeneration zugrunde liegt. Es konnte gezeigt werden dass GSK-3 Inhibitoren, wie Insulin oder Lithium eine partielle Dephosphorylierung von Tau in neuronalen Zellen bewirken (Cross et al., J. Neurochem. 77, 94-102 (2001)). Eine GSK-3 Hemmung kann daher von therapeutischer Relevanz für die Behandlung degenerativer neurologischer Erkrankungen wie der Alzheimerschen Erkrankung sein.

Inhibitoren der GSK-3 Aktivität können somit therapeutisch und /oder präventiv für eine Reihe von Erkrankungen nützlich sein, bei denen eine GSK-3 Hemmung hilfreich ist, wie bei Diabetes und Diabetes assoziierten Erkrankungen, chronisch neurodegenerativen Erkrankungen und Demenzen, wie der Alzheimerschen Erkrankung, beim Parkinson Syndrom, Pick's-Erkrankungen, Demenz bei subkortikaler arteriosklerotischer Enzephalopathie (SAE), Chorea Huntington, multiple Sklerose, infektiöse Erkrankungen (Meningoenzephalitiden, Lues, Hirnabszeß, Creutzfeldt-Jakob-Krankheit, AIDS), Demenzkomplex mit Lewy-Körperchen, neurotraumatischen Erkrankungen wie akuter Schlaganfall, Schizophrenie, manischer Depression, Hirnblutung, Haarausfall, Adipositas, atherosklerotische kardiovaskuläre Erkrankungen, Bluthochdruck, PCO-Syndrom, Metabolisches Syndrom, Ischämie, Krebs, Leukopenie, Down Syndrom, Entzündungen, Immundefizienz.

Eine neue Studie (Sato, N. et al., Nature Medicine 10, 55-63 (2004)) zeigt, dass GSK-3 Inhibitoren die Pluripotenz von Stammzellen erhalten können, was neue Anwendungsmöglichkeiten im Rahmen regenerativer Therapien durch Stammzellen ermöglichen kann.

### Bestimmung der GSK-3 Aktivität

Die Wirkung von Substanzen auf die GSK-3 Aktivität wurde nach folgendem Versuchsprotokoll durchgeführt, das auf der Phosphorylierung eines 26mer Peptids (YRRAAVPPSPSLSRHSSFHQpSEDEEE) aus der Glykogensynthase beruht, dessen Sequenz die Phosphorylierungsstellen für GSK-3 aufweist und dessen Vorphosphorylierung mit (pS) gekennzeichnet ist.

Die Testsubstanz wird in DMSO/Wasser gelöst. GSK3β (Universität Dundee, UK) gelöst in 10 mM MOPS (Morpholinopropansulfonsäure), 0.05 mM EDTA, 0.005% Brij, 2.5 % Glycerin, 0.05 % Mercaptoethanol, pH 7.0, wird mit 10 µM [³³P]-ATP, 0.25 µM 26mer Peptid versetzt und mit der gelösten Substanz in 50 mM Tris, 10 mM MgCl₂, 0.1 % Mercaptoethanol, pH 7.5, bei Raumtemperatur inkubiert. Die Reaktion wurde durch Zugabe von 75 mM Phosphorsäure gestoppt. Der Reaktionsansatz wurde auf Phosphocellulose Filterplatten (Millipore) transferiert und bis zur trockne gefiltert und zweimal mit 75 mM Phosphorsäure gewaschen. Die Phosphorylierung wurde durch die Messung der Radioaktivität auf dem Filter in einem Szintillationscounter (Topcount, Packard) bestimmt. Die Fähigkeit einer Substanz zur GSK-3 Hemmung wird durch den Vergleich des Signals eines Reaktionsansatzes mit verschiedenen Substanzkonzentrationen mit dem Signal des Reaktionsansatzes ohne Substanz bestimmt. Die IC₅₀-Werte werden durch nicht-lineare Regressionsanalyse mit Hilfe der GraphPad Prism Software berechnet.
Typische IC₅₀-Werte für die untersuchten Substanzen lagen zwischen 0.0001 µM und 1 µM.

### Bestimmung der Glykogensynthese

Dieser Test dient dazu, die Wirkung von Testsubstanzen auf die Glykogensynthese in Zellen zu untersuchen.
C3A hepatoma Zellen (ATCC) werden mit einer Dichte von 100000 Zellen/ml in 96well Platten ausgesät und als Monolayer im Medium bis zur Konfluenz kultiviert. Das Medium wird entfernt und die Zellen werden mehrmals mit PBS gewaschen und anschließend in KRBH-Puffer (134 mM NaCl, 3.5 mM KCI, 1.2 mM KH₂PO₄, 0.5 mM MgSO₄, 1.5 mM CaCl₂, 5 mM NaHCO₃, 10 mM HEPES, pH 7.4) mit 0.1 % BSA und 0.5 mM Glukose für 60 min bei 37°C inkubiert. Testsubstanz und 0.2 µCi D-[U¹⁴C]-Glukose (Amersham) werden zugefügt und die Zellen für weitere 60 min unter den gleichen Bedingungen inkubiert. Nach Entfernung des Inkubationspuffers werden die Zellen mehrmals mit kaltem PBS gewaschen und dann 10 min bei 37°C und 10 min bei Raumtemperatur mit 1 M NaOH lysiert. Die Zell-Lysate werden auf Filterplatten transferiert und das Gkykogen wird durch 2 h Inkubation mit kaltem Ethanol (70%) auf Eis präzipitiert. Die Präzipitate werden mehrmals mit Ethanol gewaschen und bis zur trockne filtriert. Das synthetisierte Glykogen wird durch die Messung der Radioaktivität (eingebaute 14C-Glucose) auf den Filterplatten in einem Szintillationscounter (Topcount, Packard) bestimmt.
Die Fähigkeit einer Substanz zur Stimulation der Glykogensynthese wird durch den Vergleich des Signals eines Reaktionsansatzes mit verschiedenen Substanzkonzentrationen mit dem Signal des Reaktionsansatzes ohne Substanz bestimmt.

### Oraler Glukose Toleranztest

7 - 9 Wochen alte gefastete db/db-Mäuse (Janvier, Frankreich) werden gewogen und Blut wird aus der Schwanzspitze entnommen. Dieses Blut dient der erste Glukosemessung auf Grund derer die Tiere randomisiert und in Gruppen eingeteilt werden. Die zu überprüfende Testsubstanz kann entweder oral oder i.p. als Suspension in 0.5 % Natrosol verabreicht werden. 30 Minuten nach Substanzgabe wird den Tieren oral 2 g/kg Glukose in einem Volumen von 0,1 ml/100 g Körpergewicht gelöst in NaCl-Lösung verabreicht. Im Anschluss werden aus Schwanzblut in bestimmten Zeitabständen [30, 60, 120 und 180 Minuten nach oraler Glukosegabe] die Glukosewerte mit einem Glukometer (Ultra OneTouch, Lifescan) bestimmt. Beispielsweise zeigt Verbindung 1.009 eine deutliche Wirkung im oralen Glukosetoleranztest.

Die erfindungsgemäß hergestellten Verbindungen sind gut verträglich, da beispielsweise nach oraler Gabe von 10 mg/kg der Verbindung des Beispiels 1.009 an Mäusen keine Änderungen im Verhalten der Tiere beobachtet werden konnten.

Die erfindungsgemäßen Verbindungen können auch in Kombination mit anderen Wirkstoffen verwendet werden. Zu den zu einer solchen Kombination geeigneten Therapeutika gehören z.B. Antidiabetika, wie etwa Metformin, Sulfonylharnstoffe (z.B. Glibenclamid, Tolbutamid, Glimepiride), Nateglinide, Repaglinide, Thiazolidindione (z.B. Rosiglitazone, Pioglitazone), PPAR-gamma-Agonisten (z.B. GI 262570) und -Antagonisten, PPAR-gamma/alpha Modulatoren (z.B. KRP 297), alpha-Glucosidasehemmer (z.B. Acarbose, Voglibose), DPP-IV-Inhibitoren, alpha2-Antagonisten, Insulin und Insulinanaloga, GLP-1 und GLP-1 Analoga (z.B. Exendin-4) oder Amylin.

Daneben SGLT2-Inhibitoren wie T-1095, Inhibitoren der Proteintyrosinphosphatase 1, Substanzen, die eine deregulierte Glucoseproduktion in der Leber beeinflussen, wie z.B. Inhibitoren der Glucose-6-phosphatase, oder der Fructose-1,6-bisphosphatase, der Glycogenphosphorylase, Glucagonrezeptorantagonisten und Inhibitoren der Phosphoenolpyruvatcarboxykinase, der Pyruvatdehydrokinase, Lipidsenker, wie etwa HMG-CoA-Reduktasehemmer (z.B. Simvastatin, Atorvastatin), Fibrate (z.B. Bezafibrat, Fenofibrat), Nikotinsäure und deren Derivate, PPAR-alpha agonisten, PPAR-delta-agonisten, ACAT-Inhibitoren (z.B. Avasimibe) oder Cholesterolresorptionsinhibitoren wie zum Beispiel Ezetimibe, gallensäurebindende Substanzen wie zum Beispiel Colestyramin, Hemmstoffe des ilealen Gallensäuretransportes, HDLerhöhende Verbindungen wie zum Beispiel Inhibitoren von CETP oder Regulatoren von ABC1 oder Wirkstoffe zur Behandlung von Obesitas, wie etwa Sibutramin oder Tetrahydrolipstatin, Dexfenfluramin, Axokine, Antagonisten des Cannbinoid1 Rezeptors, MCH-1 Rezeptorantagonisten, MC4 Rezeptor Agonisten, NPY5 oder NPY2 Antagonisten oder β₃-Agonisten wie SB-418790 oder AD-9677 ebenso wie Agonisten des 5HT2c Rezeptors.

Daneben ist eine Kombination mit Medikamenten zur Beeinflussung des Bluthochdrucks wie z.B. All Antagonisten oder ACE Inhibitoren, Diuretika, β-Blocker, Ca-Antagonisten und anderen oder Kombinationen daraus geeignet.

Im Allgemeinen können GSK-3-Inhibitoren auf verschiedenen Wegen dargereicht werden: oral, transdermal, intranasal, parenteral oder unter besonderen Umständen intrarectal. Die bevorzugte Darreichungsform ist die tägliche orale Gabe, die mehrmals am Tag erfolgen kann. GSK-3-Inhibitoren sind über einen weiten Dosisbereich wirksam. So kann die Dosierung beispielsweise zwischen 0.001 und 100 mg/kg liegen.

Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

### Herstellung der Ausgangsverbindungen:

### Beispiel I

### 5-Acetyl-2-indolinon

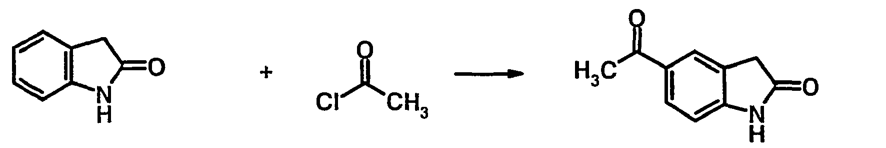

171 g (1,28 mol) Aluminiumchlorid werden in 500 ml 1,2-Dichlorethan im Eisbad gekühlt. Dann werden 78 g (1,1 mol) Acetylchlorid zugetropft, so dass die Temperatur 10°C nicht überschreitet. Nach 1 h werden 71,3 g (0,53 mol) 2-Indolinon (1,3-Dihydro-indol-2-on) in 4 Portionen zugegeben und die Temperatur bei 10-12°C gehalten. Das Reaktionsgemisch lässt man über Nacht langsam auf Raumtemperatur erwärmen. Anschließend wird die Lösung langsam und unter starkem Rühren zu 1 kg Eis gegeben. Der Brei wird mit 1 I Wasser verdünnt und noch 30 min nachgerührt. Dann wird der Niederschlag abgesaugt.

Ausbeute: 80,9 g (86,3 % der Theorie)
R_{f} = 0,36 (Kieselgel, Essigester/Cyclohexan/Methanol 9:9:2)
C₁₀H₉NO₂ (MG = 175,19)
Massenspektrum: m/z = 174 (M-H)⁻

Analog Beispiel I werden folgende Verbindungen hergestellt:
(1) 5-Propionyl-2-indolinon
   Hergestellt aus 2-Indolinon und Propionylchlorid
   Ausbeute: 72 % der Theorie
   R_{f} = 0,44 (Kieselgel, Methylenchlorid/Methanol 9:1)
   C₁₁H₁₁NO₂(MG = 189,22)
   Massenspektrum: m/z = 188 (M-H)⁻
(2) 5-Butyryl-2-indolinon
   Hergestellt aus 2-Indolinon und Buttersäurechlorid (Butyrylchlorid)
   Ausbeute: 68 % der Theorie
   C₁₂H₁₃NO₂ (MG = 203,24)
   Massenspektrum: m/z = 202 (M-H)⁻
(3) 5-Isobutyryl-2-indolinon
   Hergestellt aus 2-Indolinon und Isobutyrylchlorid
   Ausbeute: 13 % der Theorie
   C₁₂H₁₃NO₂ (MG = 203,24)
   Massenspektrum: m/z = 202 (M-H)⁻
(4) 5-Hexanoyl-2-indolinon
   Hergestellt aus 2-Indolinon und Hexansäurechlorid
   Ausbeute: 88 % der Theorie
   R_{f} = 0,51 (Kieselgel, Essigester/Cyclohexan/Methanol 9:9:2)
   C₁₄H₁₇NO₂ (MG = 231,30)
   Massenspektrum: m/z = 230 (M-H)⁻
(5) 5-Benzoyl-2-indolinon
   Hergestellt aus 2-Indolinon und Benzoesäurechlorid
   Ausbeute: 80 % der Theorie
   R_{f} = 0,46 (Kieselgel, Methylenchlorid/Methanol 9:1)
   C₁₅H₁₁NO₂ (MG = 237,26)
   Massenspektrum: m/z = 236 (M-H)⁻

### Beispiel II

### 1,5-Diacetyl-2-indolinon

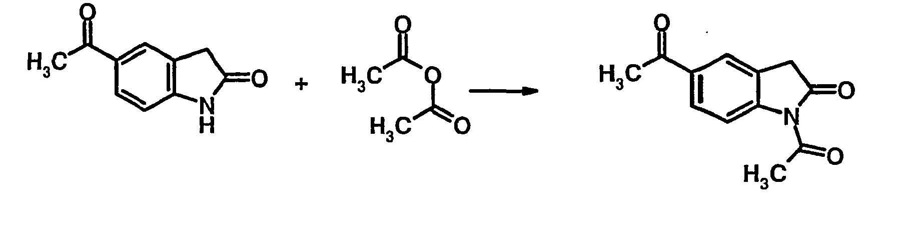

48,9 g (0,279 mol) 5-Acetyl-2-indolinon werden in 400 ml Essigsäureanhydrid 2 h in einem Ölbad bei 140 °C gerührt. Dabei löst sich das Ausgangsmaterial auf. Anschließend lässt man das Reaktionsgemisch abkühlen, engt ein, saugt den Niederschlag ab, wäscht ihn mit Ether und trocknet das Produkt.
Ausbeute: 56,0 g (92,4 % der Theorie)
R_{f} = 0,41 (Kieselgel, Methylenchlorid/Methanol 50:1)
C₁₂H₁₁NO₃ (MG = 217,223)
Massenspektrum: m/z = 216 (M-H)⁻

Analog Beispiel II werden folgende Verbindungen hergestellt:
(1) 1-Acetyl-5-propionyl-2-indolinon
   Hergestellt aus 5-Propionyl-2-indolinon und Essigsäureanhydrid
   Ausbeute: 79 % der Theorie
   R_{f} = 0,68 (Kieselgel, Methylenchlorid/Methanol 9:1)
   C₁₃H₁₃NO₃ (MG = 231 ,25)
   Massenspektrum: m/z = 232 (M+H)⁺
(2) 1-Acetyl-5-benzoyl-2-indolinon
   Hergestellt aus 5-Benzoyl-2-indolinon und Essigsäureanhydrid
   Ausbeute: 89 % der Theorie
   R_{f} = 0,60 (Kieselgel, Methylenchlorid/Methanol 30:1)
   C₁₇H₁₃NO₃ (MG = 279,294)
   Massenspektrum: m/z = 278 (M-H)⁻
(3) 1-Acetyl-5-hexanoyl-2-indolinon
   Hergestellt aus 5-Hexanoyl-2-indolinon und Essigsäureanhydrid
   R_{f} = 0,74 (Kieselgel, Methylenchlorid/Methanol 30:1)
   C₁₆H₁₉NO₃ (MG = 273,33)
   Massenspektrum: m/z = 272 (M-H)⁻

### Beispiel III

### 1,5-Diacetyl-3-(ethoxy-phenyl-methyliden)-2-indolinon

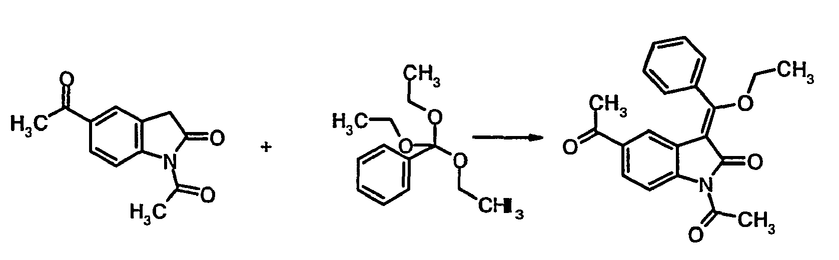

32,6 g (150 mmol) 1,5-Diacetyl-2-indolinon werden in 100 ml Orthobenzoesäuretriethylester suspendiert und mit 150 ml Essigsäureanhydrid bei 110 °C über Nacht gerührt. Dann werden nochmals 50 ml Orthobenzoesäuretriethylester zugegeben und weitere 24 h gerührt. Anschließend wird eingeengt und der entstehende Niede r-schlag abgesaugt, gewaschen und getrocknet.
Ausbeute: 38 g (72,5 % der Theorie)
R_{f} = 0,60 (Kieselgel, Methylenchlorid/Methanol 30:1)
C₂₁H₁₉NO₄ (MG = 349,384)
Massenspektrum: m/z = 350 (M+H)⁺

Analog Beispiel III werden folgende Verbindungen hergestellt:
(1) 1-Acetyl-5-hexanoyl-3-(ethoxy-phenyl-methyliden)-2-indolinon
   Hergestellt aus 1-Acetyl-5-hexanoyl-2-indolinon und Orthobenzoesäuretriethylester
   Ausbeute: 29 % der Theorie
   R_{f} = 0,72 (Kieselgel, Methylenchlorid/Methanol 30:1)
   C₂₅H₂₇NO₄ (MG = 405,491)
   Massenspektrum: m/z = 428 (M+Na)⁺
(2) 1-Acetyl-5-benzoyl-3-(ethoxy-phenyl-methyliden)-2-indolinon
   Hergestellt aus 1-Acetyl-5-benzoyl-2-indolinon und Orthobenzoesäuretriethylester
   Ausbeute: 65 % der Theorie
   R_{f} = 0,72 (Kieselgel, Methylenchlorid/Methanol 30:1)
   C₂₆H₂₁NO₄ (MG = 411,455)
   Massenspektrum: m/z = 412 (M+H)⁺
(3) 1,5-Diacetyl-3-(1-methoxy-propyliden)-2-indolinon
   Hergestellt aus 1,5-Diacetyl-2-indolinon und Orthopropionsäuretrimethylester
   Ausbeute: 80 % der Theorie
   R_{f} = 0,50 (Kieselgel, Methylenchlorid/Methanol 50:1)
   C₁₆H₁₇NO₄ (MG = 287,311)
   Massenspektrum: m/z = 288 (M+H)⁺
(4) 1,5-Diacetyl-3-(1-methoxy-butyliden)-2-indolinon
   Hergestellt aus 1,5-Diacetyl-2-indolinon und Orthobuttersäuretrimethylester
   Ausbeute: 71 % der Theorie
   R_{f} = 0,53 (Kieselgel, Methylenchlorid/Methanol 50:1)
   C₁₇H₁₉NO₄ (MG = 301,337)
   Massenspektrum: m/z = 302 (M+H)⁺
(5) 1,5-Diacetyl-3-(1-methoxy-pentyliden)-2-indolinon
   Hergestellt aus 1,5-Diacetyl-2-indolinon und Orthovaleriansäuretrimethylester
   Ausbeute: 66 % der Theorie
   R_{f} = 0,60 (Kieselgel, Methylenchlorid/Methanol 50:1)
   C₁₈H₂₁NO₄ (MG = 315,364)
   Massenspektrum: m/z = 316 (M+H)⁺
(6) 1,5-Diacetyl-3-(1-methoxy-2-methyl-propyliden)-2-indolinon
   Hergestellt aus 1,5-Diacetyl-2-indolinon und 1,1,1-Trimethoxy-2-methylpropan
   Ausbeute: 40 % der Theorie
   R_{f} = 0,71 (Kieselgel, Essigester:Cyclohexan:Methanol 9:9:2)
   C₁₇H₁₉NO₄ (MG = 301,337)
   Massenspektrum: m/z = 302 (M+H)⁺
(7) 1,Acetyl-5-propionyl-3-(1-methoxy-propyliden)-2-indolinon
   Hergestellt aus 1-Acetyl-5-propionyl-2-indolinon und Orthopropionsäuretrimethylester
(8) 1,Acetyl-5-hexanonyl-3-(1-methoxy-propyliden)-2-indolinon
   Hergestellt aus 1-Acetyl-5-hexanoyl-2-indolinon und Orthopropionsäuretrimethylester

### Beispiel IV

### 1-Acetyl-5-butyryl-3-(ethoxy-phenyl-methyliden)-2-indolinon

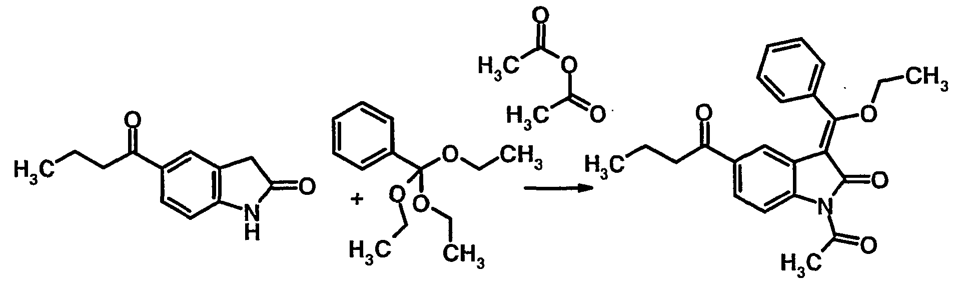

10 g (49 mmol) 5-Butyryl-2-indolinon (Bsp. I.2) werden in 200 ml Essigsäureanhydrid 5 h bei 130 °C gerührt. Dann werden 35 ml Orthobenzoesäuretriethylester zugegeben und weitere 4 h bei 100 °C gerührt. Anschließend wird eingeengt und der entstehende Niederschlag abgesaugt, gewaschen und getrocknet.
Ausbeute: 11,5 g (62 % der Theorie)
R_{f} = 0,79 (Kieselgel, Essigester/Cyclohexan/Methanol 9:9:2)
C₂₃H₂₃NO₄ (MG = 377,438)
Massenspektrum: m/z = 378 (M+H)⁺

Analog Beispiel IV werden folgende Verbindungen hergestellt:
(1) 1-Acetyl-5-isobutyryl-3-(ethoxy-phenyl-methyliden)-2-indolinon
   Hergestellt aus 5-Isobutyryl-2-indolinon, Essigsäureanhydrid und Orthobenzoesäuretriethylester
   R_{f} = 0,55 (Kieselgel, Essigester/Cyclohexan/Methanol 9:9:2)
(2) 1,5-Diacetyl-3-[1-methoxy-ethyliden]-2-indolinon 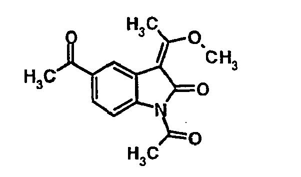 Hergestellt aus 5-Acetyl-2-indolinon, Essigsäureanhydrid und Orthoessigsäuretrimethylester
   R_{f} = 0,40 (Kieselgel, Methylenchlorid/Methanol 50 :1)
(3) 1-Acetyl-5-propionyl-3-(ethoxy-phenyl-methyliden)-2-indolinon 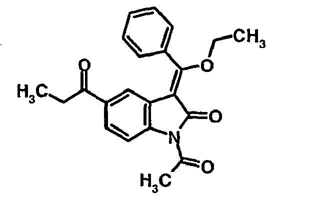 Hergestellt aus 5-Propionyl-2-indolinon, Essigsäureanhydrid und Orthobenzoesäuretriethylester
   R_{f} = 0,79 (Kieselgel, Essigester/Cyclohexan/Methanol 9:9:2)
(4) 1-Acetyl-5-hexanoyl-3-(ethoxy-phenyl-methyliden)-2-indolinon 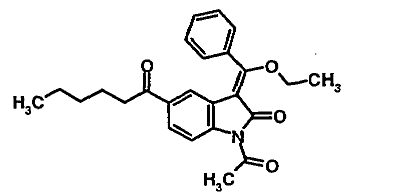 Hergestellt aus 5-Hexanoyl-2-indolinon, Essigsäureanhydrid und Orthobenzoesäuretriethylester
   R_{f} = 0,72 (Methylenchlorid/Methanol 30 :1)
(5) 1-Acetyl-5-butyryl-3-(ethoxy-phenyl-methyliden)-2-indolinon 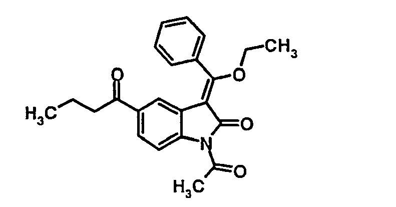 Hergestellt aus 5-butyryl-2-indolinon, Essigsäureanhydrid und Orthobenzoesäuretriethylester
   R_{f} = 0,79 (Kieselgel, Essigester/Cyclohexan/Methanol 9:9:2)

### Beispiel V

### 1,5-Diacetyl-3-[(3,4-dimethoxy-phenyl)-hydroxy-methyliden]-2-indolinon

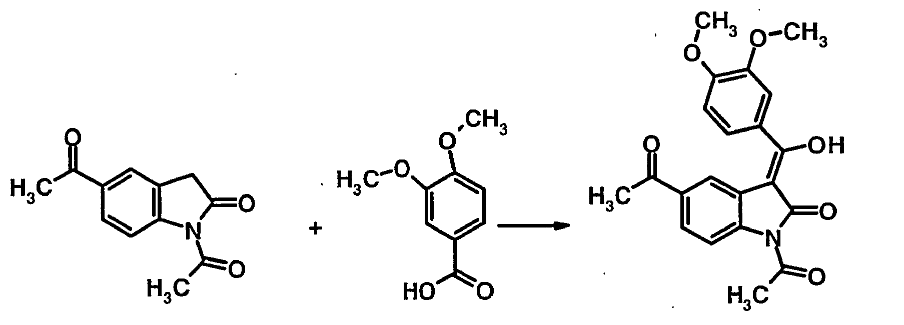

4,3 g (20 mmol) 1,5-Diacetyl-2-indolinon (Bsp. II) werden zusammen mit 4 g 3,4-Dimethoxybenzoesäure, 7,1 g TBTU (O-Benzotriazol-1-yl-N,N,N',N'-tetramethyluroniumtetrafluoroborat) und 14 ml Triethylamin in 80 ml DMF (Dimethylformamid) bei Raumtemperatur über Nacht gerührt. Dann wird der Ansatz auf 300 ml Eiswasser mit 10 ml konz. Salzsäure gegossen und der entstandene Niederschlag abgesaugt. Der Rückstand wird mit wenig Methanol und anschließend mit Ether gewaschen.
Ausbeute: 6,2 g (81,3 % der Theorie)
R_{f} = 0,85 (Kieselgel, Methylenchlorid/Methanol 9:1)
C₂₁H₁₉NO₆ (MG = 381,382)
Massenspektrum: m/z = 381 (M)⁺

Analog Beispiel V werden folgende Verbindungen hergestellt:
(1) 1,5-Diacetyl-3-[(benzo[1,3]dioxol-5-yl)-hydroxy-methyliden]-2-indolinon 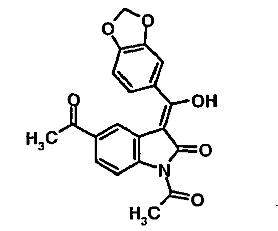 Hergestellt aus 1,5-Diacetyl-2-indolinon und Piperonylsäure (Benzo[1,3]dioxol-5-carbonsäure)
   Ausbeute: 60 % der Theorie
   R_{f} = 0,70 (Kieselgel, Methylenchlorid/Methanol 9:1)
   C₂₀H₁₅NO₆ (MG = 365,339)
   Massenspektrum: m/z = 366 (M+H)⁺
(2) 1,5-Diacetyl-3-[(4-nitro-phenyl)-hydroxy-methyliden]-2-indolinon 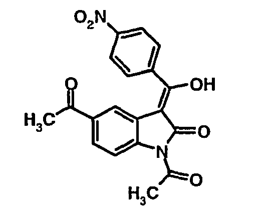 Hergestellt aus 1,5-Diacetyl-2-indolinon und 4-Nitrobenzoesäure
   Ausbeute: 82 % der Theorie
   R_{f} = 0,38 (Kieselgel, Methylenchlorid/Methanol 9:1)
   C₁₉H₁₄N₂O₆ (MG = 366,328)
   Massenspektrum: m/z = 367 (M+H)⁺
(3) 1,5-Diacetyl-3-[(3-nitro-phenyl)-hydroxy-methyliden]-2-indolinon 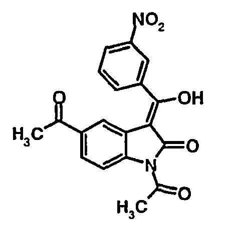 Hergestellt aus 1,5-Diacetyl-2-indolinon und 3-Nitrobenzoesäure
   Ausbeute: 75 % der Theorie
   R_{f} = 0,38 (Kieselgel, Methylenchlorid/Methanol 9:1)
   C₁₉H₁₄N₂O₆ (MG = 366,328)
   Massenspektrum: m/z = 367 (M+H)⁺
(4) 1,5-Diacetyl-3-[(4-methyloxycarbonyl-phenyl)-hydroxy-methyliden]-2-indolinon 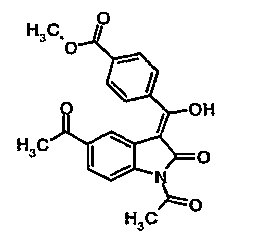 Hergestellt aus 1,5-Diacetyl-2-indolinon und Terephthalsäuremonomethylester Ausbeute: 71 % der Theorie
   R_{f} = 0,41 (Kieselgel, Methylenchlorid/Methanol 30:1)
   C₂₁H₁₇NO₆ (MG = 379,366)
   Massenspektrum: m/z = 380 (M+H)⁺
(5) 1,5-Diacetyl-3-[(4-chloro-phenyl)-hydroxy-methyliden]-2-indolinon 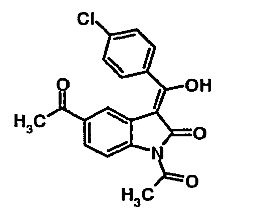 Hergestellt aus 1,5-Diacetyl-2-indolinon und 4-Chlorobenzoesäure
   Ausbeute: 87 % der Theorie
   C₁₉H₁₄ClNO₄ (MG = 355,776)
   Massenspektrum: m/z = 356/358 (M+H)⁺
(6) 1,5-Diacetyl-3-[(3,4-dichlor-phenyl)-hydroxy-methyliden]-2-indolinon 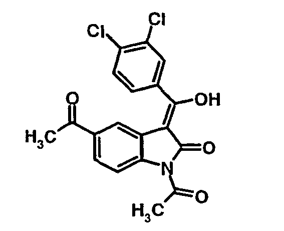 Hergestellt aus 1,5-Diacetyl-2-indolinon und 3,4-Dichlorobenzoesäure
   Ausbeute: 83 % der Theorie
   C₁₉H₁₃Cl₂NO₄ (MG = 390,221)
   Massenspektrum: m/z = 390/392/394 (M+H)⁺
(7) 1,5-Diacetyl-3-[(4-cyano-phenyl)-hydroxy-methyliden]-2-indolinon 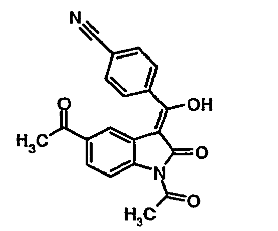 Hergestellt aus 1,5-Diacetyl-2-indolinon und 4-Cyanobenzoesäure
   Ausbeute: 71 % der Theorie
   R_{f} = 0,32 (Kieselgel, Methylenchlorid/Methanol 9:1)
   C₂₀H₁₄N₂O₄ (MG = 346,341)
   Massenspektrum: m/z = 347 (M+H)⁺
(8) 1,5-Diacetyl-3-[(4-trifluoromethyl-phenyl)-hydroxy-methyliden]-2-indolinon 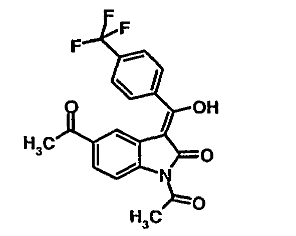 Hergestellt aus 1,5-Diacetyl-2-indolinon und 4-Trifluoromethyl-benzoesäure
   Ausbeute: 83 % der Theorie
   C₂₀H₁₄F₃NO₄ (MG = 389,328)
   Massenspektrum: m/z = 390 (M+H)⁺
(9) 1,5-Diacetyl-3-[(2,3-dihydro-benzo-[1,4]dioxin-6-yl)-hydroxy-methyliden]-2-indolinon 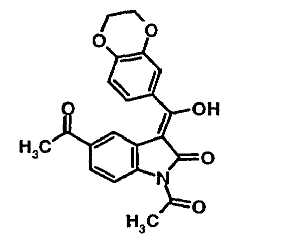 Hergestellt aus 1,5-Diacetyl-2-indolinon und 2,3-Dihydro-1,4-benzodioxin-6-carbonsäure
   Ausbeute: 90 % der Theorie
   R_{f} = 0,75 (Kieselgel, Methylenchlorid/Methanol 9:1)
   C₂₁H₁₇NO₆ (MG = 379,366)
   Massenspektrum: m/z = 380 (M+H)⁺
(10) 1,5-Diacetyl-3-[(3-methoxy-phenyl)-hydroxy-methyliden]-2-indolinon 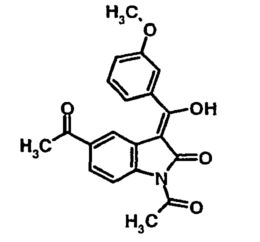 Hergestellt aus 1,5-Diacetyl-2-indolinon und 3-Methoxybenzoesäure
   Ausbeute: 70 % der Theorie
   R_{f} = 0,67 (Kieselgel, Methylenchlorid/Methanol 9:1)
(11) 1,5-Diacetyl-3-[(4-methoxy-phenyl)-hydroxy-methyliden]-2-indolinon 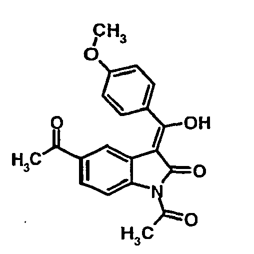 Hergestellt aus 1,5-Diacetyl-2-indolinon und 4-Methoxybenzoesäure
   Ausbeute: 59 % der Theorie
   R_{f} = 0,39 (Kieselgel, Methylenchlorid/Methanol 9:1)
   C₂₀H₁₇NO₅ (MG = 351,356)
   Massenspektrum: m/z = 350 (M-H)⁻
(12) 1-Diacetyl-5-propionyl-3-[(benzo[1,3]dioxol-5-yl)-hydroxy-methyliden]-2-indolinon 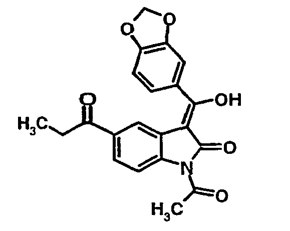 Hergestellt aus 1-Acetyl-5-propionyl-2-indolinon und Piperonylsäure (Benzo[1,3]-dioxol-5-carbonsäure)
   Ausbeute: 67 % der Theorie
   R_{f} = 0,49 (Kieselgel, Methylenchlorid/Methanol 30:1)
   C₂₁H₁₇NO₆ (MG = 379,366)
   Massenspektrum: m/z = 380 (M+H)⁺
(13) 1,5-Diacetyl-3-[(4-bromophenyl)-hydroxy-methyliden]-2-indolinon 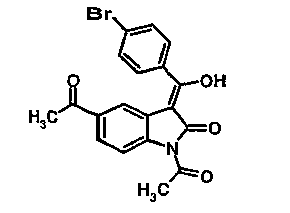 Hergestellt aus 1,5-Diacetyl-2-indolinon und 4-Bromobenzoesäure
   Ausbeute: 89 % der Theorie
   C₁₉H₁₄BrNO₄ (MG = 400,227)
   Massenspektrum: m/z = 400/402 (M+H)⁺
(14) 1,5-Diacetyl-3-[(3,5-dichlor-phenyl)-hydroxy-methyliden]-2-indolinon 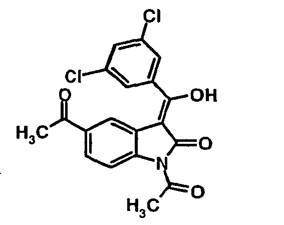 Hergestellt aus 1,5-Diacetyl-2-indolinon und 3,5-Dichlorobenzoesäure
   Ausbeute: 79 % der Theorie
   R_{f} = 0,26 (Kieselgel, Methylenchlorid/Methanol 30:1)
   C₁₉H₁₃Cl₂NO₄ (MG = 390,221)
   Massenspektrum: m/z = 390/392/394 (M+H)⁺
(15) 1,5-Diacetyl-3-[(3,5-dimethoxyphenyl)-hydroxy-methyliden]-2-indolinon 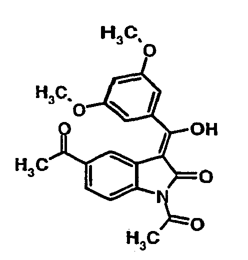 Hergestellt aus 1,5-Diacetyl-2-indolinon und 3,5-Dimethoxybenzoesäure
   Ausbeute: 83 % der Theorie
   R_{f} = 0,37 (Kieselgel, Methylenchlorid/Methanol 30:1)
   C₂₁H₁₉NO₆ (MG = 381,382)
   Massenspektrum: m/z = 382 (M+H)⁺
(16) 1,5-Diacetyl-3-[(2-chloro-phenyl)-hydroxy-methyliden]-2-indolinon 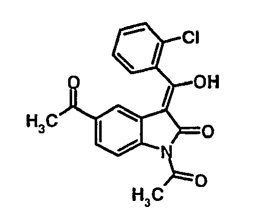 Hergestellt aus 1,5-Diacetyl-2-indolinon und 2-Chlorobenzoesäure
   Ausbeute: 96 % der Theorie
   C₁₉H₁₄ClNO₄ (MG = 355,776)
   Massenspektrum: m/z = 356/358 (M+H)⁺
(17) 1,5-Diacetyl-3-[(2-methoxy-phenyl)-hydroxy-methyliden]-2-indolinon 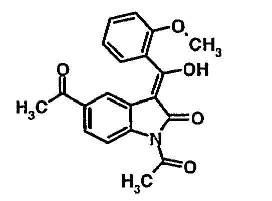 Hergestellt aus 1,5-Diacetyl-2-indolinon und 2-Methoxybenzoesäure
   Ausbeute: 27 % der Theorie
   C₂₀H₁₇NO₅ (MG = 351,356)
   Massenspektrum: m/z = 352 (M+H)⁺
(18) 1,5-Diacetyl-3-[(2,6-difluoro-phenyl)-hydroxy-methyliden]-2-indolinon 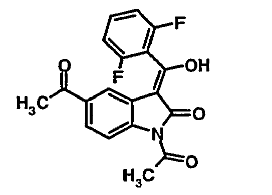 Hergestellt aus 1,5-Diacetyl-2-indolinon und 2,6-Difluorbenzoesäure
   Ausbeute: 52 % der Theorie
   C₁₉H₁₃F₂NO₄ (MG = 357,311)
   Massenspektrum: m/z = 358 (M+H)⁺
(19) 1,5-Diacetyl-3-[(4-fluorphenyl)-hydroxy-methyliden]-2-indolinon 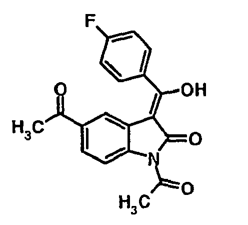 Hergestellt aus 1,5-Diacetyl-2-indolinon und 4-Fluorbenzoesäure
   Ausbeute: 77 % der Theorie
   C₁₉H₁₄FNO₄ (MG = 339,321)
   Massenspektrum: m/z = 338 (M-H)⁻
(20) 1,5-Diacetyl-3-[(3,4-difluor-phenyl)-hydroxy-methyliden]-2-indolinon 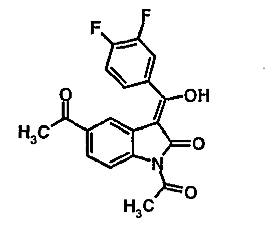 Hergestellt aus 1,5-Diacetyl-2-indolinon und 3,4-Difluorbenzoesäure
   Ausbeute: 91 % der Theorie
(21) 1,5-Diacetyl-3-[(2,2-difluor-benzo[1,3]dioxol-5-yl)-hydroxy-methyliden]-2-indolinon 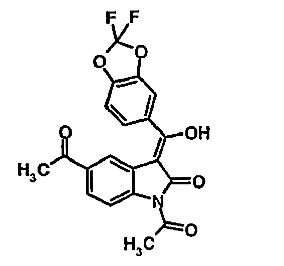 Hergestellt aus 1,5-Diacetyl-2-indolinon und 2,2-Difluor-benzo[1,3]dioxol-5-carbonsäure
   Ausbeute: 69 % der Theorie
   C₂₀H₁₃F₂NO₆ (MG = 401,32)
   Massenspektrum: m/z = 402 (M+H)⁺
(22) 1,5-Diacetyl-3-[(4-(2-methoxycarbonyl-ethyl)-phenyl)-hydroxy-methyliden]-2-indolinon 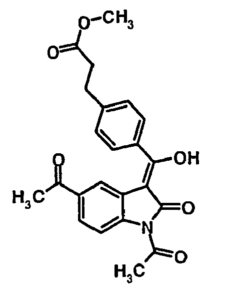 Hergestellt aus 1,5-Diacetyl-2-indolinon und 4-(2-methoxycarbonyl-ethyl)-benzoesäure
   Ausbeute: 23 % der Theorie
   C₂₃H₂₁NO₆ (MG = 407,42)
   Massenspektrum: m/z = 408 (M+H)⁺
(23) 1,5-Diacetyl-3-[(pyrazin-2-yl)-hydroxy-methyliden]-2-indolinon 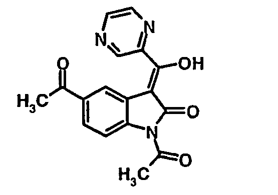 Hergestellt aus 1,5-Diacetyl-2-indolinon und Pyrazin-2-carbonsäure
   Ausbeute: 57 % der Theorie
   C₁₇H₁₃N₃O₄ (MG = 323,311)
   Massenspektrum: m/z = 324 (M+H)⁺
(24) 1,5-Diacetyl-3-[(pyridin-4-yl)-hydroxy-methyliden]-2-indolinon 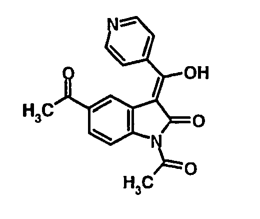 Hergestellt aus 1,5-Diacetyl-2-indolinon und Isonikotinsäure (Pyridin-4-carbonsäure) Ausbeute: 87 % der Theorie
   C₁₈H₁₄N₂O₄ (MG = 322,323)
   Massenspektrum: m/z = 323 (M+H)⁺
(25) 1,5-Diacetyl-3-[(furan-3-yl)-hydroxy-methyliden]-2-indolinon 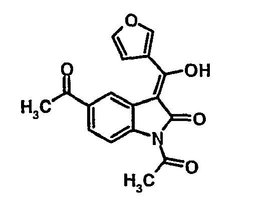 Hergestellt aus 1,5-Diacetyl-2-indolinon und Furan-3-carbonsäure
   Ausbeute: 73 % der Theorie
   C₁₇H₁₃NO₅ (MG = 311,297)
   Massenspektrum: m/z = 312 (M+H)⁺
(26) 1,5-Diacetyl-3-[(4-diethylaminomethyl-phenyl)-hydroxy-methyliden]-2-indolinon 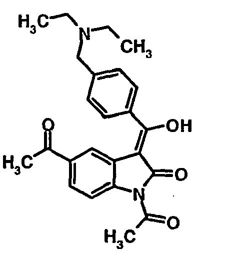 Hergestellt aus 1,5-Diacetyl-2-indolinon und 4-Diethylaminomethyl-benzoesäure
   Ausbeute: 10 % der Theorie
   C₂₄H₂₆N₂O₄ (MG = 406,486)
   Massenspektrum: m/z = 407 (M+H)⁺
(27) 1,5-Diacetyl-3-[(4-methoxycarbonylmethoxy-phenyl)-hydroxy-methyliden]-2-indolinon 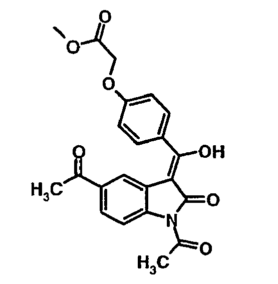 Hergestellt aus 1,5-Diacetyl-2-indolinon und 4-Methoxycarbonyl-methoxybenzoesäure
   Ausbeute: 43 % der Theorie
   C₂₂H₁₉NO₇ (MG = 409,39)
   Massenspektrum: m/z = 410 (M+H)⁺
(28) 1,5-Diacetyl-3-[(4-methylsulfonyl-phenyl)-hydroxy-methyliden]-2-indolinon 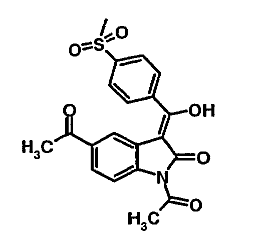 Hergestellt aus 1,5-Diacetyl-2-indolinon und 4-Methylsulfonyl-benzoesäure
   Ausbeute: 25 % der Theorie
   C₂₀H₁₇NO₆S (MG = 399,418)
   Massenspektrum: m/z = 400 (M+H)⁺
(29) 1,5-Diacetyl-3-[(4-(2-diethylamino-ethoxy)-phenyl)-hydroxy-methyliden]-2-indolinon 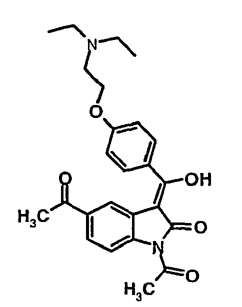 Hergestellt aus 1,5-Diacetyl-2-indolinon und 4-Diethylamino-ethoxy-benzoesäure
   Ausbeute: 27 % der Theorie
   C₂₅H₂₈N₂O₅ (MG = 436,500)
   Massenspektrum: m/z = 437 (M+H)⁺
(30) 1,5-Diacetyl-3-[(3-(2-diethylamino-ethoxy)-phenyl)-hydroxy-methyliden]-2-indolinon 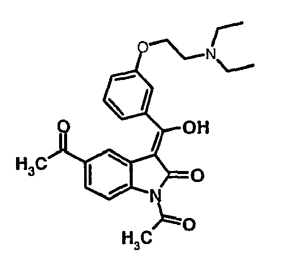 Hergestellt aus 1,5-Diacetyl-2-indolinon und 3-Diethylamino-ethoxy-benzoesäure
   Ausbeute: 43 % der Theorie
   C₂₅H₂₈N₂O₅ (MG = 436,500)
   Massenspektrum: m/z = 437 (M+H)⁺
(31) 1,5-Diacetyl-3-[(3-(2-diethylamino-ethoxy)-phenyl)-hydroxy-methyliden]-2-indolinon 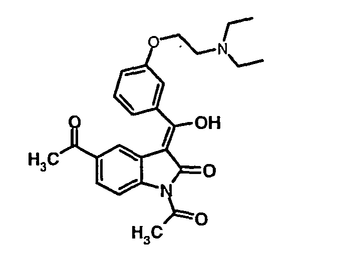 Hergestellt aus 1,5-Diacetyl-2-indolinon und 3-Diethylamino-ethoxy-benzoesäure
   Ausbeute: 43 % der Theorie
   C₂₅H₂₈N₂O₅ (MG = 436,500)
   Massenspektrum: m/z = 437 (M+H)⁺
(31) 1,5-Diacetyl-3-[(3-(2-diethylamino-ethoxy)-phenyl)-hydroxy-methyliden]-2-indolinon 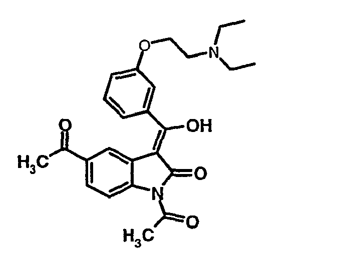 Hergestellt aus 1,5-Diacetyl-2-indolinon und 3-Diethylamino-ethoxy-benzoesäure
   Ausbeute: 43 % der Theorie
   C₂₅H₂₈N₂O₅ (MG = 436,500)
   Massenspektrum: m/z = 437 (M+H)⁺
(32) 1,5-Diacetyl-3- (1-hydroxy-heptyliden)-2-indolinon 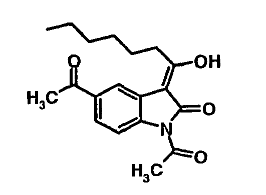 Hergestellt aus 1,5-Diacetyl-2-indolinon und Heptansäure
(33) 1,5-Diacetyl-3- (1-hydroxy-hexyliden)-2-indolinon 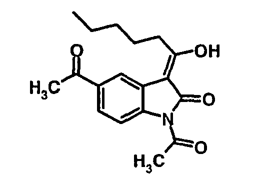 Hergestellt aus 1,5-Diacetyl-2-indolinon und Hexansäure
(34) 1,5-Diacetyl-3-(1-hydroxy-3-methyl-butyliden)-2-indolinon 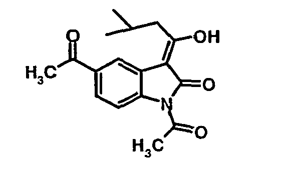 Hergestellt aus 1,5-Diacetyl-2-indolinon und Isovaleriansäure

### Beispiel VI

### 1,5-Diacetyl-3-[(3,4-dimethoxy-phenyl)-methoxy-methyliden]-2-indolinon

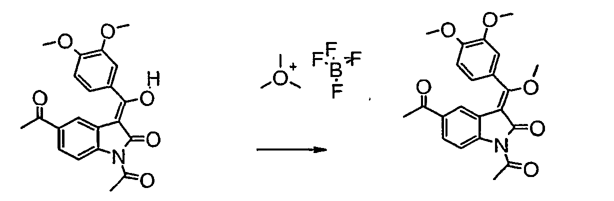

4,0 g (10,5 mmol) 1,5-Diacetyl-3-[(3,4-dimethoxy-phenyl)-hydroxy-methyliden]-2-indolinon (Bsp. V) werden in 100 ml Methylenchlorid suspendiert und mit 3,1 g (21 mmol) Trimethyloxoniumtetrafluorborat sowie 7,2 ml Hünigbase (Ethyldiisopropylamin) bei Raumtemperatur versetzt. Die Lösung wird 3 h gerührt, dann werden nochmals 1,55 g Trimethyloxoniumtetrafluorborat und 3,5 ml Hünigbase zugegeben und über Nacht gerührt. Nachdem nochmals dieselbe Reagenzmenge zugegeben und weitere 5 h gerührt wurde, wird die Umsetzung dreimal mit Wasser gewaschen, die organische Phase über Natriumsulfat getrocknet, filtriert und einrotiert. Der Rückstand wird über eine Kieselgelsäule mit Methylenchlorid/Methanol 9:1 chromatographiert, die entsprechende Fraktionen vereinigt und einrotiert.
Ausbeute: 1,6 g (37 % der Theorie)
R_{f} = 0,78 (Kieselgel, Methylenchlorid/Methanol 50:1)
C₂₂H₂₁NO₆ (MG = 395,409)
Massenspektrum: m/z = 396 (M+H)⁺

Analog Beispiel VI werden folgende Verbindungen hergestellt:
(1) 1,5-Diacetyl-3-[(benzo[1,3]dioxol-5-yl)-methoxy-methyliden]-2-indolinon 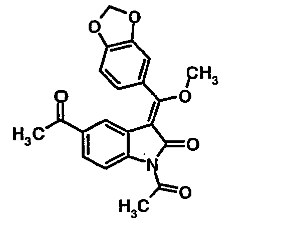 Hergestellt aus 1,5-Diacetyl-3-[(benzo[1,3]dioxol-5-yl)-hydroxy-methyliden]-2-indolinon (Bsp. V.1)
   Ausbeute: 85 % der Theorie
   R_{f} = 0,55 (Kieselgel, Methylenchlorid/Methanol 30:1)
   C₂₁H₁₇NO₆ (MG = 379,366)
   Massenspektrum: m/z = 380 (M+H)⁺
(2) 1,5-Diacetyl-3-[(4-nitro-phenyl)-methoxy-methyliden]-2-indolinon 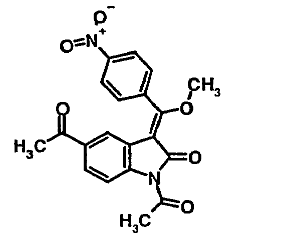 Hergestellt aus 1,5-Diacetyl-3-[(4-nitro-phenyl)-hydroxy-methyliden]-2-indoli non (Bsp. V.2)
   Ausbeute: 82 % der Theorie
   R_{f} = 0,55 (Kieselgel, Methylenchlorid/Methanol 30:1)
   C₂₀H₁₆N₂O₆ (MG = 380,354 )
   Massenspektrum: m/z = 381 (M+H)⁺
(3) 1,5-Diacetyl-3-[(3-nitro-phenyl)-methoxy-methyliden]-2-indolinon 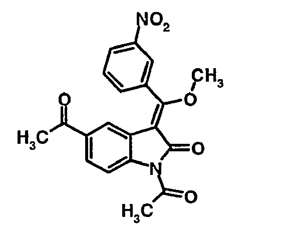 Hergestellt aus 1,5-Diacetyl-3-[(3-nitro-phenyl)-hydroxy-methyliden]-2-indolinon (Bsp. V.3)
   Ausbeute: 43 % der Theorie
   R_{f} = 0,44 (Kieselgel, Methylenchlorid/Methanol 9:1)
   C₂₀H₁₆N₂O₆ (MG = 380,354 )
   Massenspektrum: m/z = 381 (M+H)⁺
(4) 1,5-Diacetyl-3-[(4-methyloxycarbonyl-phenyl)-methoxy-methyliden]-2-indolinon 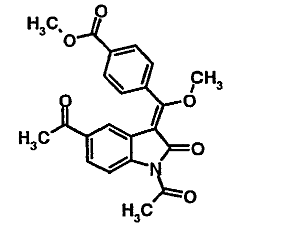 Hergestellt aus 1,5-Diacetyl-3-[(4-methyloxycarbonyl-phenyl)-hydroxy-methyliden]-2-indolinon (Bsp. V.4)
   Ausbeute: 52 % der Theorie
   R_{f} = 0,56 (Kieselgel, Methylenchlorid/Methanol 30:1)
   C₂₂H₁₉NO₆ (MG = 393,393)
   Massenspektrum: m/z = 394 (M+H)⁺
(5) 1,5-Diacetyl-3-[(4-chloro-phenyl)-methoxy-methyliden]-2-indolinon 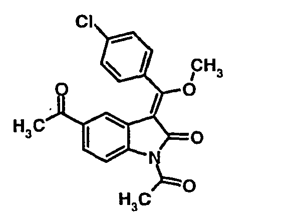 Hergestellt aus 1,5-Diacetyl-3-[(4-chloro-phenyl)-hydroxy-methyliden]-2-indolinon (Bsp. V.5)
   Ausbeute: 65 % der Theorie
   C₂₀H₁₆ClNO₄ (MG = 369,802 )
   Massenspektrum: m/z = 370/372 (M+H)⁺
(6) 1,5-Diacetyl-3-[(3,4-dichlor-phenyl)-methoxy-methyliden]-2-indolinon 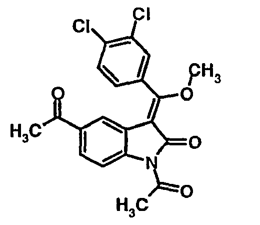 Hergestellt aus 1,5-Diacetyl-3-[(3,4-dichlor-phenyl)-hydroxy-methyliden]-2-indolinon (Bsp. V.6)
   Ausbeute: 72 % der Theorie
   C₂₀H₁₅Cl₂NO₄ (MG = 404,247 )
   Massenspektrum: m/z = 404/406/408 (M+H)⁺
(7) 1,5-Diacetyl-3-[(4-cyano-phenyl)-methoxy-methyliden]-2-indolinon 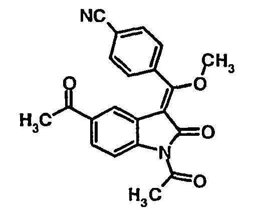 Hergestellt aus 1,5-Diacetyl-3-[(4-cyano-phenyl)-hydroxy-methyliden]-2-indolinon (Bsp. V.7)
   Ausbeute: 53 % der Theorie
   C₂₁H₁₆N₂O₄ (MG = 360,367 )
   Massenspektrum: m/z = 361 (M+H)⁺
(8) 1,5-Diacetyl-3-[(4-trifluoromethyl-phenyl)-methoxy-methyliden]-2-indolinon 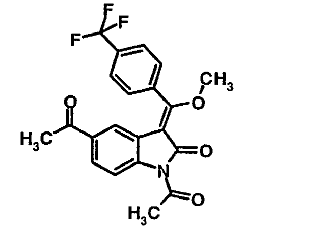 Hergestellt aus 1,5-Diacetyl-3-[(4-trifluoromethyl-phenyl)-hydroxy-methyliden]-2-indolinon (Bsp. V.8)
   Ausbeute: 37 % der Theorie
   C₂₁H₁₆F₃NO₄ (MG = 403,354 )
   Massenspektrum: m/z = 404 (M+H)⁺
(9) 1,5-Diacetyl-3-[(2,3-dihydro-benzo-[1,4]dioxin-6-yl)-methoxy-methyliden]-2-indolinon 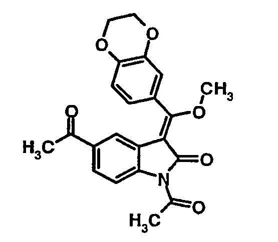 Hergestellt aus 11,5-Diacetyl-3-[(2,3-dihydro-benzo-[1,4]dioxin-6-yl)-hydroxymethyliden]-2-indolinon (Bsp. V.9)
   Ausbeute: 52 % der Theorie
   R_{f} = 0,82 (Kieselgel, Methylenchlorid/Methanol 9:1)
   C₂₂H₁₉NO₆ (MG = 393,393 )
   Massenspektrum: m/z = 394 (M+H)⁺
(10) 1,5-Diacetyl-3-[(3-methoxy-phenyl)-methoxy-methyliden]-2-indolinon 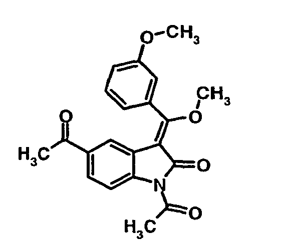 Hergestellt aus 1,5-Diacetyl-3-[(3-methoxy-phenyl)-hydroxy-methyliden]-2-indolinon (Bsp. V.1 0)
   Ausbeute: 48 % der Theorie
   R_{f} = 0,40 (Kieselgel, Methylenchlorid/Methanol 9:1)
   C₂₁H₁₉NO₅ (MG = 365,383 )
   Massenspektrum: m/z = 366 (M+H)⁺
(11) 1,5-Diacetyl-3-[(4-methoxy-phenyl)-methoxy-methyliden]-2-indolinon 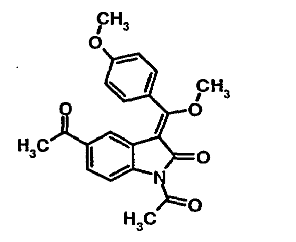 Hergestellt aus 1,5-Diacetyl-3-[(4-methoxy-phenyl)-hydroxy-methyliden]-2-indolinon (Bsp. V.11)
   Ausbeute: 85 % der Theorie
   R_{f} = 0,35 (Kieselgel, Methylenchlorid/Methanol 30:1)
   C₂₁H₁₉NO₅ (MG = 365,383 )
   Massenspektrum: m/z = 366 (M+H)⁺
(12) 1-Diacetyl-5-propionyl-3-[(benzo[1,3]dioxol-5-yl)-methoxy-methyliden]-2-indolinon 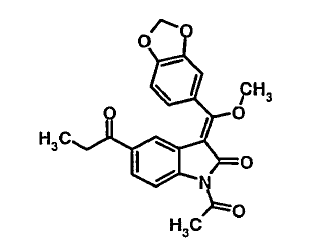 Hergestellt aus 1-Diacetyl-5-propionyl-3-[(benzo[1,3]dioxol-5-yl)-hydroxy-methyliden]-2-indolinon (Bsp. V.12)
   Ausbeute: 98 % der Theorie
   R_{f} = 0,63 (Kieselgel, Methylenchlorid/Methanol 30:1)
   C₂₂H₁₉NO₆ (MG = 393,393 )
   Massenspektrum: m/z = 394 (M+H)⁺
(13) 1,5-Diacetyl-3-[(4-bromophenyl)-methoxy-methyliden]-2-indolinon 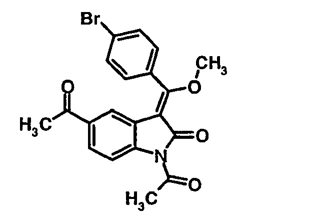 Hergestellt aus 1,5-Diacetyl-3-[(4-bromophenyl)-hydroxy-methyliden]-2-indolinon (Bsp. V.13)
   Ausbeute: 48 % der Theorie
(14) 1,5-Diacetyl-3-[(3,5-dichlor-phenyl)-methoxy-methyliden]-2-indolinon 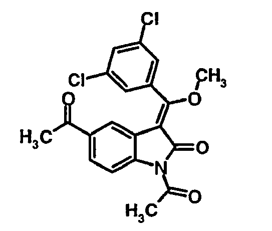 Hergestellt aus 1,5-Diacetyl-3-[(3,5-dichlor-phenyl)-hydroxy-methyliden]-2-indolinon (Bsp. V.14)
   Ausbeute: 44 % der Theorie
   R_{f} = 0,86 (Kieselgel, Methylenchlorid/Methanol 30:1)
   C₁₉H₁₃Cl₂NO₄ (MG = 390,221)
   Massenspektrum: m/z = 388/390/392 (Cl2, M+H)⁺
(15) 1,5-Diacetyl-3-[(3,5-dimethoxy-phenyl)-methoxy-methyliden]-2-indolinon 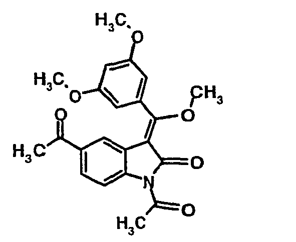 Hergestellt aus 1,5-Diacetyl-3-[(3,5-dimethoxy-phenyl)-hydroxy-methyliden]-2-indolinon (Bsp. V.15)
   Ausbeute: 74 % der Theorie
   R_{f} = 0,65 (Kieselgel, Methylenchlorid/Methanol 30:1)
   C₂₂H₂₁NO₆ (MG = 395,409)
   Massenspektrum: m/z = 396 (M+H)⁺
(16) 1,5-Diacetyl-3-[(2-chloro-phenyl)-methoxy-methyliden]-2-indolinon 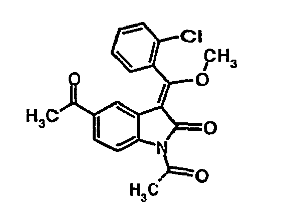 Hergestellt aus 1,5-Diacetyl-3-[(2-chloro-phenyl)-hydroxy-methyliden]-2-indolinon (Bsp. V.16)
   Ausbeute: 54 % der Theorie
   C₂₀H₁₆ClNO₄ (MG = 369,802)
   Massenspektrum: m/z = 370/372 (M+H)⁺
(17) 1,5-Diacetyl-3-[(2-methoxy-phenyl)-methoxy-methyliden]-2-indolinon 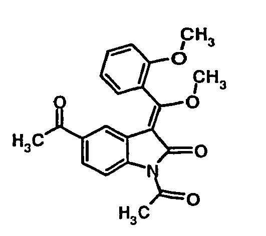 Hergestellt aus 1,5-Diacetyl-3-[(2-methoxy-phenyl)-hydroxy-methyliden]-2-indolinon (Bsp. V.17)
   Ausbeute: 56 % der Theorie
   C₂₁H₁₉NO₅ (MG = 365,383)
   Massenspektrum: m/z = 366 (M+H)⁺
(18) 1,5-Diacetyl-3-[(2,6-difluoro-phenyl)-methoxy-methyliden]-2-indolinon 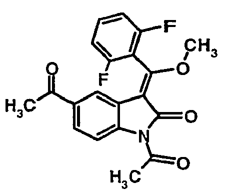 Hergestellt aus 1,5-Diacetyl-3-[(2,6-difluoro-phenyl)-hydroxy-methyliden]-2-indolinon (Bsp. V.18)
   Ausbeute: 59 % der Theorie
   C₂₀H₁₅F₂NO₄ (MG = 3371,337)
   Massenspektrum: m/z = 372 (M+H)⁺
(19) 1,5-Diacetyl-3-[(4-fluorphenyl)-methoxy-methyliden]-2-indolinon 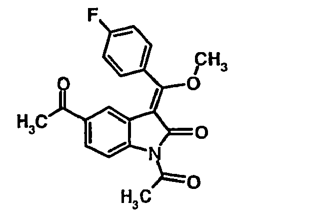 Hergestellt aus 1,5-Diacetyl-3-[(4-fluorphenyl)-hydroxy-methyliden]-2-indolinon (Bsp. V.19)
   Ausbeute: 88 % der Theorie
   C₂₀H₁₆FNO₄ (MG = 353,347)
   Massenspektrum: m/z = 354 (M+H)⁺
(20) 1,5-Diacetyl-3-[(3,4-difluor-phenyl)-methoxy-methyliden]-2-indolinon 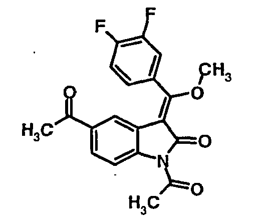 Hergestellt aus 1,5-Diacetyl-3-[(3,4-difluor-phenyl)-hydroxy-methyliden]-2-indolinon (Bsp. V.20)
   Ausbeute: 23 % der Theorie
   C₂₀H₁₅F₂NO₄ (MG = 371,334)
   Massenspektrum: m/z = 372 (M+H)⁺
(21) 1,5-Diacetyl-3-[(2,2-difluor-benzo[1,3]dioxol-5-yl)-methoxy-methyliden]-2-indolinon 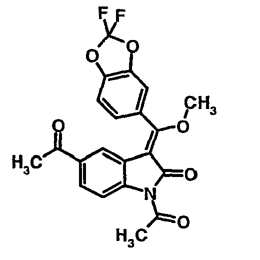 Hergestellt aus 1,5-Diacetyl-3-[(2,2-difluor-benzo[1,3]dioxol-5-yl)-hydroxy-methyliden]-2-indolinon (Bsp. V.21)
   Ausbeute: 6 % der Theorie
   C₂₁H₁₅F₂NO₆ (MG = 415,346)
   Massenspektrum: m/z = 416 (M+H)⁺
(22) 1,5-Diacetyl-3-[(4-(2-methoxycarbonyl-ethyl)-phenyl)-methoxy-methyliden]-2-indolinon 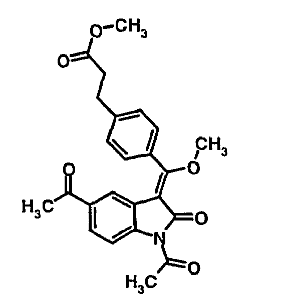 Hergestellt aus 1,5-Diacetyl-3-[(4-(2-methoxycarbonyl-ethyl)-phenyl)-hydroxy-methyliden]-2-indolinon (Bsp. V.22)
   Ausbeute: 63 % der Theorie
   C₂₄H₂₃NO₆ (MG = 421,447)
   Massenspektrum: m/z = 422 (M+H)⁺
(23) 1,5-Diacetyl-3-[furan-3-yl-methoxy-methyliden]-2-indolinon 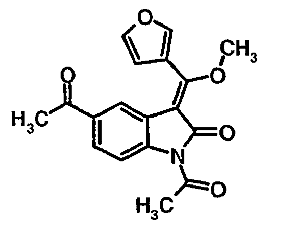 Hergestellt aus 1,5-Diacetyl-3-[furan-3-yl-hydroxy-methyliden]-2-indolinon (Bsp. V.25)
   Ausbeute: 59 % der Theorie
   C₁₈H₁₅NO₅ (MG = 325,324)
   Massenspektrum: m/z = 326 (M+H)⁺
(24) 1,5-Diacetyl-3-[(4-methoxycarbonylmethoxy-phenyl)-methoxy-methyliden]-2-indolinon 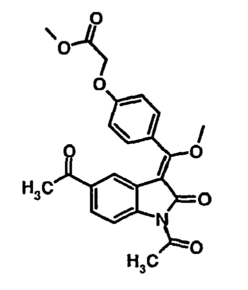 Hergestellt aus 1,5-Diacetyl-3-[(4-methoxycarbonyl methoxy-phenyl)-hydroxy-methyliden]-2-indolinon
   Ausbeute: 24 % der Theorie
   C₂₃H₂₁NO₇ (MG = 423,415)
   Massenspektrum: m/z = 424 (M+H)⁺
(25) 1,5-Diacetyl-3-[(4-methylsulfonyl-phenyl)-methoxy-methyliden]-2-indolinon 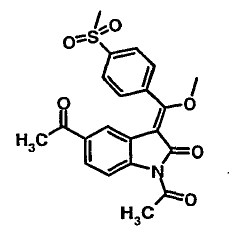 Hergestellt aus 1,5-Diacetyl-3-[(4-methylsulfonyl-phenyl)-hydroxy-methyliden]-2-indolinon (Bsp. V.28)
   Ausbeute: 20 % der Theorie
   C₂₁H₁₉NO₆S (MG = 413,445)
   Massenspektrum: m/z = 414 (M+H)⁺
(26) 1,5-Diacetyl-3-(1-methoxy-octyliden)-2-indolinon 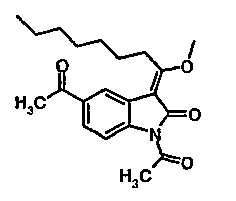 Hergestellt aus 1,5-Diacetyl-3-(1-hydroxyl-octyliden)-2-indolinon (Bsp. VIII) Ausbeute: 82 % der Theorie
   C₂₁H₂₇NO₄S (MG = 357,443)
   Massenspektrum: m/z = 358 (M+H)⁺
(27) 1,5-Diacetyl-3-(1-methoxy-heptyliden)-2-indolinon 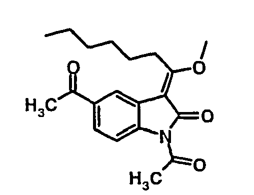 Hergestellt aus 1,5-Diacetyl-3- (1-hydroxy-heptyliden)-2-indolinon (Bsp. V.32)
(28) 1,5-Diacetyl-3-(1-methoxy-hexyliden)-2-indolinon 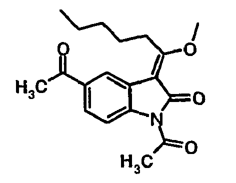 Hergestellt aus 1,5-Diacetyl-3- (1-hydroxy-hexyliden)-2-indolinon (Bsp. V.33)
(29) 1,5-Diacetyl-3-(1-methoxy-3-methyl-butyliden)-2-indolinon 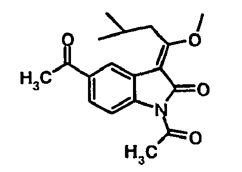 Hergestellt aus 1,5-Diacetyl-3- (1-hydroxy-3-methyl-butyliden)-2-indolinon (Bsp. V.34)

### Beispiel VII

### 1,5-Diacetyl-3-[chloro-(pyrazin-2-yl)-methyliden]-2-indolinon

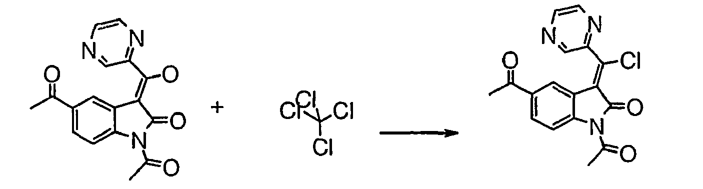

1,2 g (3,7 mmol) 1,5-Diacetyl-3-[(pyrazin-2-yl)-hydroxy-methyliden]-2-indolinon (Bsp. V.23) werden in 50 ml Dioxan gelöst und mit 2 ml Tetrachlorkohlenstoff und 2 g Triphenylphosphin 5 h am Rückfluß gekocht. Anschließend lässt man abkühlen und engt ein. Der Rückstand wird über eine Kieselgelsäule mit Methylenchlorid/Methanol 25:1 chromatographiert, die entsprechenden Fraktionen vereinigt und einrotiert.
Ausbeute: 400 mg (40 % der Theorie)
R_{f} = 0,70 (Kieselgel, Methylenchlorid/Methanol 30:1)
C₁₇H₁₂ClN₃O₃ (MG = 341,756)
Massenspektrum: m/z = 342/344 (M+H)⁺(CL)

Analog Beispiel VII wird folgende Verbindung hergestellt:

### (1)1,5-Diacetyl-3-[chloro-(4-(2-dimethylamino-ethoxy)-phenyl)-methyliden]-2-indolinon

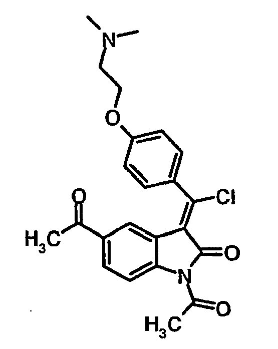

### Beispiel VIII

### 1,5-Diacetyl-3-(1-hydroxy-octyliden)-2-indolinon

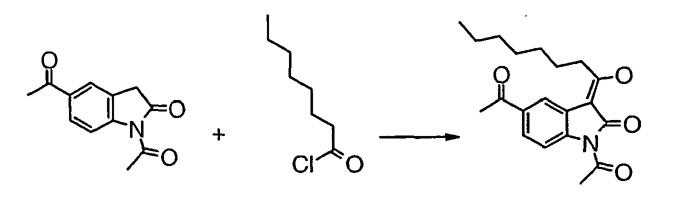

4,3 g (20 mmol) 1,5-Diacetyl-2-indolinon (Bsp. II) werden in 20 ml Dimethylformamid gelöst und 490 mg Dimethylaminopyridin (DMAP) und 6 ml Triethylamin zugegeben und im Eisbad gekühlt. Zu dieser Lösung gibt man 3,8 ml (22 mmol) Octansäurechlorid in 20 ml dimethylformamid und rührt weitere 10 min. Anschließend gibt man das Reaktionsgemisch in 150 ml Methylenchlorid und 150 ml 1 N Salzsäure. Die organische Phase wird abgetrennt, über Natriumsulfat getrocknet und einrotiert. Der Rückstand wird über eine Kieselgelsäule mit Methylenchlorid/Methanol 95:5 chromatographiert.

Ausbeute: 740 mg (11 % der Theorie)
C₂₀H₂₅NO₄ (MG = 343,417)
Massenspektrum: m/z = 344 (M)⁺

### Herstellung der Endverbindungen:

### Fließmittel:

A: Methylenchlorid/Methanol 9:1
B: Methalenchlorid/Methanol 4:1
C: Methylenchlorid/Methanol/konz. Ammoniak 9:1:0,1
D: Methylenchlorid/Methanol 30:1
E: Methylenchlorid/Methanol/Triethylamin 9:1:0,1

Bei den Formeln in der Tabelle stellt die freie gezeichnete Bindung stets die Bindung des jeweiligen Restes zum Anknüpfungspunkt im Molekül dar. Der Tabelleneintrag "-CH₃" bedeutet also einen Methylrest und der Eintrag 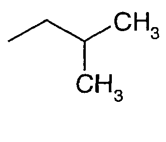 eine Isobutylgruppe, d.h. -CH₂-CH(CH₃)₂.

### Beispiel 1

### 5-Acetyl-3-[(3-dimethylamino-propylamino)-phenyl-methyliden]-2-indolinon

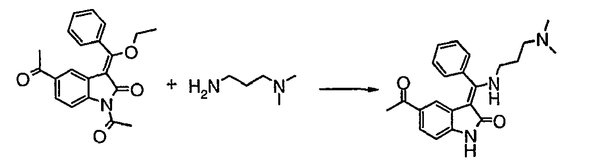

0,2 g (0,57 mmol) 1,5-Diacetyl-3-(phenyl-ethoxy-methyliden)-2-indolinon (Bsp. III) werden in 5 ml Dimethylformamid suspendiert und mit 0,07 ml 3-Dimethylaminopropylamin bei 70 °C 12 h gerührt. Dann lässt man abkühlen, gibt 2 ml Methanol und 1 ml 1 N Natronlauge zu und rührt bei Raumtemperatur 30 min. Anschließend wird Wasser zugegeben, und der entstehende Niederschlag abfiltriert, gewaschen und getrocknet. Falls notwendig, kann die Verbindung über Kieselgel chromatographisch gereinigt werden. Als Fließmittel eignet sich Methylenchlorid/Methanol 30:1
Ausbeute: 0,1 g (82 % der Theorie)
R_{f} = 0,39 (Kieselgel, Methylenchlorid/Methanol/konz. Ammoniak 9:1:0,1)
C₂₂H₂₅N₃O₂ (MG = 363,458)
Massenspektrum: m/z = 364 (M+H)⁺

Analog Beispiel 1 werden folgende Verbindungen der Formel I hergestellt:

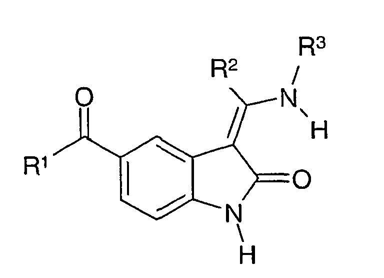

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 1.001 | Me | Ph | 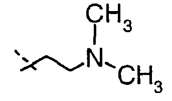 | III 85,9 | (M+H)⁺ = 350 | 0,43 (A) |
| 1.002 | Me | Ph | 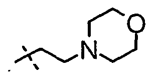 | III 74,1 | (M+H)⁺ = 391 | 0,51 (A) |
| 1.003 | Me | Ph | 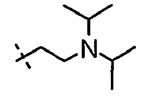 | III 88,8 | (M+H)⁺ = 406 | 0,58 (A) |
| 1.004 | Me | Ph | 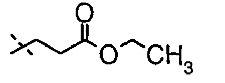 | III 81,9 | (M+H)⁺ = 379 | 0,23 (D) |
| 1.005 | Me | Ph | 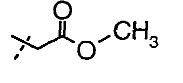 | III 88,5 | (M+H)⁺ = 351 | 0,22 (D) |
| 1.006 | Me | Ph | 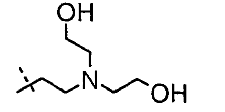 | III 80,6 | (M+H)⁺ = 410 | 0,35 (C) |
| 1.007 | Me | Ph | 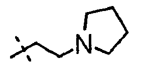 | III 79,0 | (M+H)⁺ = 376 | 0.39 (C) |
| 1.008 | Me | Ph | 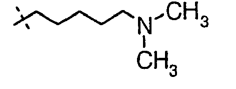 | III 66,4 | (M+H)⁺ = 392 | 0,27 (C) |
| 1.009 | Me | Ph | 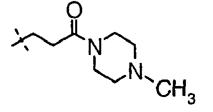 | III 64,9 | (M+H)⁺ = 433 | 0,52 (C) |
| 1.010 | Me | Ph | 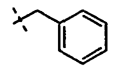 | III 80,7 | (M-H)⁻ = 367 | 0,42 (A) |
| 1.011 | Me | Ph | 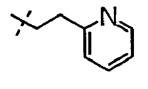 | III 54,7 | (M-H)⁻ = 382 | 0,38 (A) |
| 1.012 | Me | Ph | 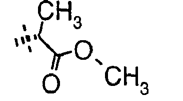 | III 67,1 | (M+H)⁺ = 365 | 0,48 (A) |
| 1.013 | Me | Ph | 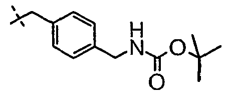 | III 32,8 | (M+H)⁺ = 498 | 0,59 (A) |
| 1.014 | Me | Ph | 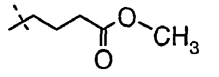 | III 92,3 | (M+H)⁺ = 379 | 0,34 (A) |
| 1.015 | Me | Ph | 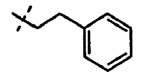 | III 73,1 | (M+H)⁺ = 383 | 0,33 (A) |
| 1.016 | Me | Ph | 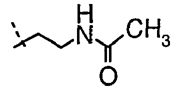 | III 57,7 | (M+H)⁺ = 364 | 0,29 (A) |
| 1.017 | Me | Ph | 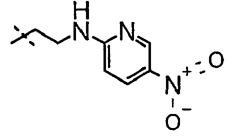 | III 22,9 | (M+H)⁺ = 444 | 0,42 (A) |
| 1.018 | Me | 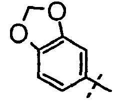 | 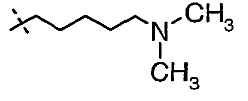 | VI.1 53,2 | (M+H)⁺ = 436 | 0,46 (B) |
| 1.019 | Me | 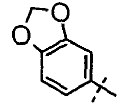 | 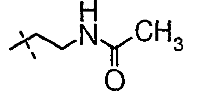 | VI.1 65,2 | (M+H)⁺ = 408 | 0,43 (A) |
| 1.020 | Me | Ph | 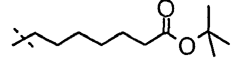 | III 11,7 | (M+H)⁺ = 463 | 0,56 (A) |
| 1.021 | Me | 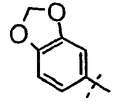 | 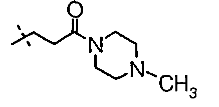 | VI.1 28,7 | (M+H)⁺ = 477 | 0,31 (A) |
| 1.022 | Me | Ph | 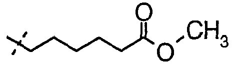 | III 66,6 | (M+H)⁺ = 407 | 0,38 (A) |
| 1.023 | Me | 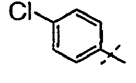 | 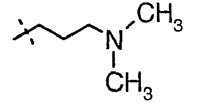 | VI.5 70,2 | (M+H)⁺ = 398/400 (Cl) | 0,18 (A) |
| 1.024 | Me | 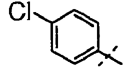 | 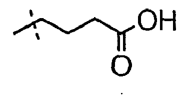 | VI.5 57,0 | (M+H)⁺ = 399/401 (Cl) | 0,25 (A) |
| 1.025 | Me | 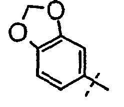 | 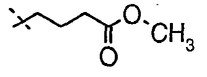 | VI.1 44,9 | (M+H)⁺ = 423 | 0,32 (A) |
| 1.026 | Me | 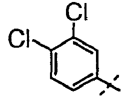 | 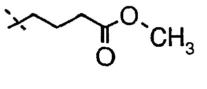 | VI.6 51,9 | (M+H)⁺ = 447/449/ 451 (Cl2) | 0,30 (A) |
| 1.027 | Me | 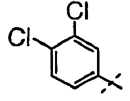 | 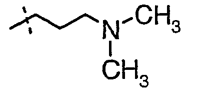 | IV.6 24,5 | (M+H)⁺ = 432/434/ 436 (Cl2) | 0,37 (C) |
| 1.028 | Me | 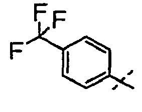 | 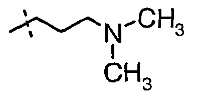 | VI.8 35,1 | (M+H)⁺ = 432 | 0,25 (A) |
| 1.029 | Me | Ph | Et | III 98 | (M+H)⁺ = 307 | 0,44 (A) |
| 1.030 | Me | Ph | 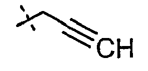 | III 90,6 | (M+H)⁺ = 317 | 0,31 (A) |
| 1.031 | Me | Ph | 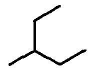 | III 90,3 | (M+H)⁺ = 349 | 0,48 (A) |
| 1.032 | Me | Ph | i-Pr | III 77,5 | (M+H)⁺ = 321 | 0,46 (A) |
| 1.033 | Me | Ph | 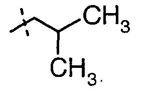 | III 78,4 | (M+H)⁺ = 335 | 0,33 (A) |
| 1.034 | Me | Ph | n-Pr | III 56,7 | (M+H)⁺ = 321 | 0,31 (A) |
| 1.035 | Me | Ph | 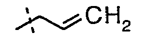 | III 93,3 | (M+H)⁺ = 319 | 0,32 (A) |
| 1.036 | Me | 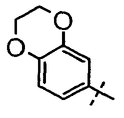 | 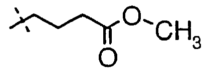 | VI.9 72,1 | (M+H)⁺ = 437 | 0,38 (A) |
| 1.037 | Me | 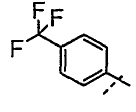 | 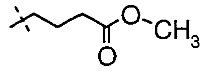 | VI.8 40,2 | (M+H)⁺ = 447 | 0,75 (A) |
| 1.038 | Me | 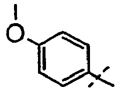 | 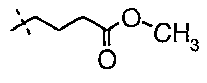 | VI.11 76,9 | (M+H)⁺ = 409 | 0,36 (A) |
| 1.039 | Me | 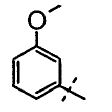 | 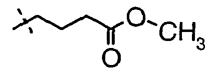 | VI.10 80,3 | (M+H)⁺ = 409 | 0,36 (A) |
| 1.040 | Me | 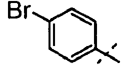 | 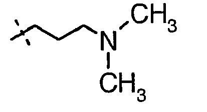 | VI.13 67,7 | (M-H)⁻ = 440/442 (Br) | 0,26 (A) |
| 1.041 | Me | Ph | 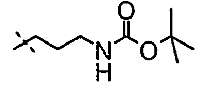 | III 85,0 | (M+H)⁺ = 436 | 0,28 (A) |
| 1.042 | Me | 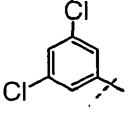 | 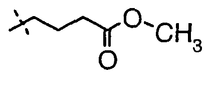 | VI.14 67,8 | (M-H)⁻ = 445/447/ 449 (Cl2) | 0,25 (A) |
| 1.043 | Me | 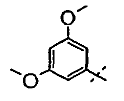 | 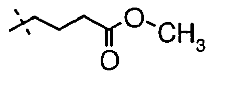 | VI.15 71,0 | (M+H)⁺ = 439 | 0,27 (A) |
| 1.044 | Me | 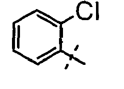 | 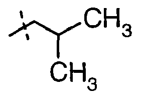 | VI.16 75,5 | (M+H)⁺ = 369/371 (Cl) | 0,33 (A) |
| 1.045 | Me | 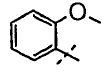 | 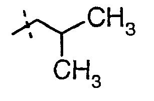 | VI.17 62,2 | (M+H)⁺ = 365 | 0,05 (CH₂Cl₂/ MeOH 50:1) |
| 1.046 | Me | 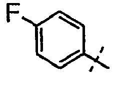 | 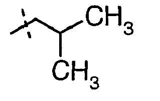 | VI.19 68,1 | (M+H)⁺ = 353 | 0,46 (A) |
| 1.047 | Me | 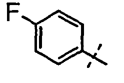 | 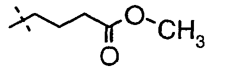 | VI.19 59,4 | (M+H)⁺ = 397 | 0,45 (A) |
| 1.048 | Me | 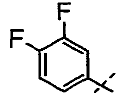 | 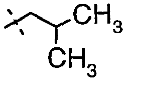 | VI.20 13,8 | (M+H)⁺ = 371 | 0,54 (A) |
| 1.049 | Me | 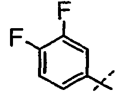 | 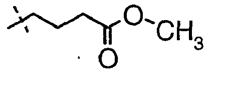 | VI.20 51,4 | (M+H)⁺ = 415 | 0,56 (A) |
| 1.050 | Me | 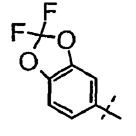 | 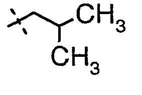 | VI.21 38,5 | (M+H)⁺ = 415 | 0,31 (A) |
| 1.051 | Me | 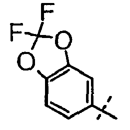 | 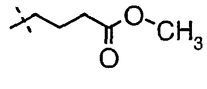 | VI.21 33,8 | (M-H)⁻ = 457 | 0,46 (A) |
| 1.052 | Me | 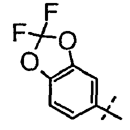 | 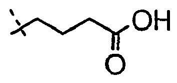 | VI.21 22,4 | (M-H)⁻ = 443 | 0,37 (B) |
| 1.053 | Me | 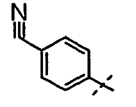 | 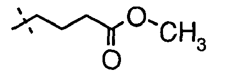 | VI.7 78,6 | (M+H)⁺ = 404 | 0,53 (A) |
| 1.054 | Me | 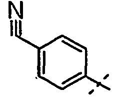 | 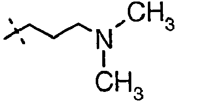 | VI.7 90,3 | (M+H)⁺ = 389 | 0,53 (C) |
| 1.055 | Me | 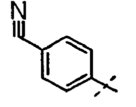 | 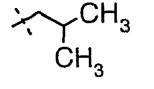 | VI.7 83,8 | (M+H)⁺ = 360 | 0,36 (A) |
| 1.056 | Me | 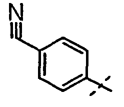 | n-Pr | VI.7 87,6 | (M+H)⁺ = 346 | 0,34 (A) |
| 1.057 | Me | 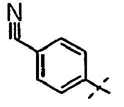 | 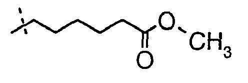 | VI.7 86,0 | (M+H)⁺ = 432 | 0,37 (A) |
| 1.058 | Me | 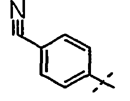 | 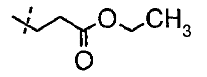 | VI.7 63,4 | (M+H)⁺ = 404 | 0,49 (A) |
| 1.059 | Me | 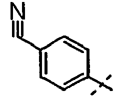 | 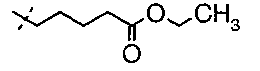 | VI.7 78,0 | (M-H)⁻ = 430 | 0,33 (A) |
| 1.060 | Me | Ph | t-Bu | III 68,0 | (M+H)⁺ = 335 | 0,52 (A) |
| 1.061 | Me | 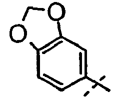 | 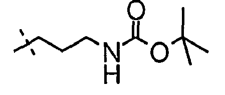 | VI.1 57,0 | (M+H)⁺ = 480 | 0,44 (A) |
| 1.062 | Me | 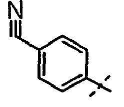 | 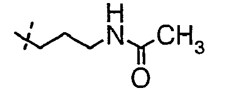 | VI.7 89,5 | (M+H)⁺ = 403 | 0,35 (A) |
| 1.063 | Me | 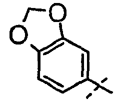 | 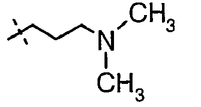 | VI.1 63,3 | (M+H)⁺ = 408 | 0,18 (A) |
| 1.064 | Me | 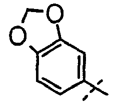 | 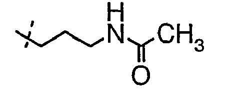 | VI.1 65,7 | (M+H)⁺ = 422 | 0,38 (A) |
| 1.065 | Me | 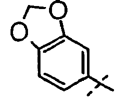 | 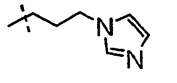 | VI.1 74,5 | (M+H)⁺ = 431 | 0,41 (A) |
| 1.066 | Me | 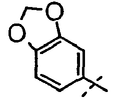 | 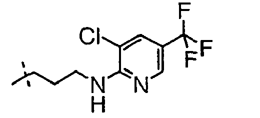 | IV.1 67,2 | (M+H)⁺ = 559/561 (Cl) | 0,55 (A) |
| 1.067 | Me | 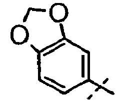 | 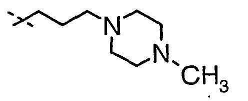 | VI.1 57,4 | (M+H)⁺ = 463 | 0,26 (A) |
| 1.068 | Me | 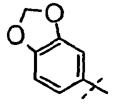 | 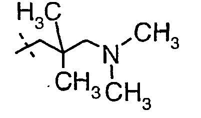 | VI.1 61,0 | (M+H)⁺ = 436 | 0,36 (A) |
| 1.069 | Me | Ph | 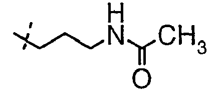 | III 61,1 | (M+H)⁺ = 378 | 0,29 (A) |
| 1.070 | Me | 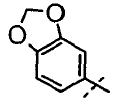 | 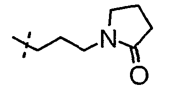 | VI.1 84,8 | (M+H)⁺ = 448 | 0,42 (A) |
| 1.071 | Me | 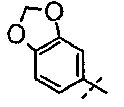 | 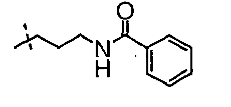 | VI.1 62,0 | (M+H)⁺ = 484 | 0,44 (A) |
| 1.072 | Me | 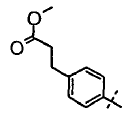 | 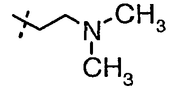 | VI.22 58,0 | (M+H)⁺ = 436 | 0,46 (A) |
| 1.073 | Me | 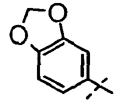 | 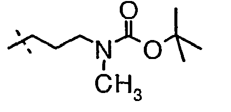 | VI.1 99,9 | (M+H)⁺ = 494 | 0,46 (A) |
| 1.074 | Me | 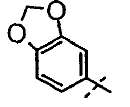 | 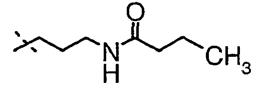 | VI.1 71,8 | (M+H)⁺ = 450 | 0,48 (A) |
| 1.075 | Me | 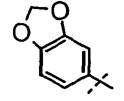 | 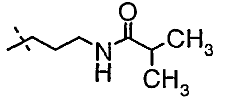 | VI.1 65,4 | (M+H)⁺ = 450 | 0,44 (A) |
| 1.076 | Me | 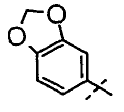 | 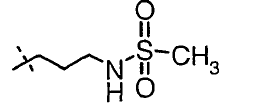 | VI.1 58,1 | (M+H)⁺ = 458 | 0,55 (A) |
| 1.077 | Me | 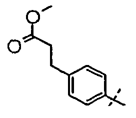 | 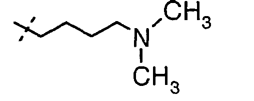 | VI.22 84,8 | (M+H)⁺ = 464 | 0,51 (C) |
| 1.078 | Me | 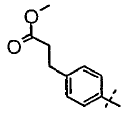 | 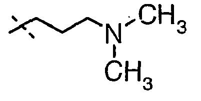 | VI.22 68,7 | (M+H)⁺ = 450 | 0,33 (C) |
| 1.079 | Me | 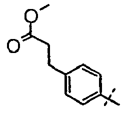 | 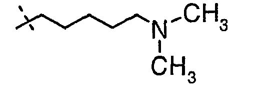 | VI.22 58,8 | (M+H)⁺ = 478 | 0,31 (C) |
| 1.080 | Me | 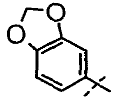 | 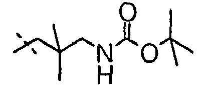 | VI.1 93,5 | (M+H)⁺ = 508 | 0,4 (A) |
| 1.081 | Me | 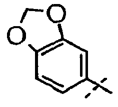 | 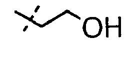 | VI.1 76,0 | (M+H)⁺ = 367 | 0,46 (A) |
| 1.082 | Me | 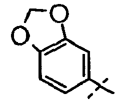 | 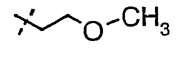 | VI.1 66,6 | (M+H)⁺ = 381 | 0,44 (A) |
| 1.083 | Me | 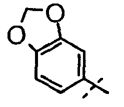 | 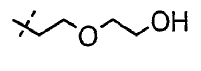 | VI.1 69,7 | (M+H)⁺ = 411 | 0,43 (A) |
| 1.084 | Me | 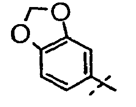 | 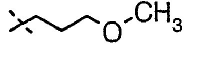 | VI.1 55,2 | (M+H)⁺ = 395 | 0,47 (A) |
| 1.085 | Me | Ph | H | III 97,0 | (M+H)⁺ = 279 | 0,39 (C) |
| 1.086 | Me | 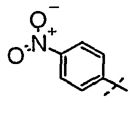 | H | VI.3 85,1 | (M+H)⁺ = 324 | 0,53 (C) |
| 1.087 | Et | 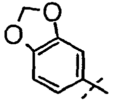 | 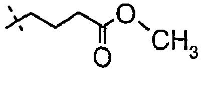 | VI.12 51,5 | (M+H)⁺ = 437 | 0,39 (A) |
| 1.088 | Me | 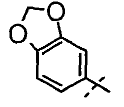 | 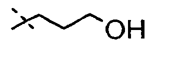 | VI.12 66,6 | (M+H)⁺ = 381 | 0,43 (A) |
| 1.089 | Me | 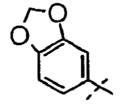 | 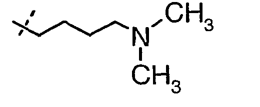 | VI.12 69,1 | (M+H)⁺ = 422 | 0,09 (A) |
| 1.090 | Me | 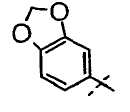 | 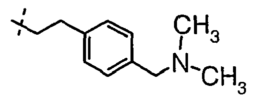 | VI.1 28,8 | (M+H)⁺ = 484 | 0,40 (A) |
| 1.091 | Me | 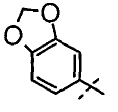 | 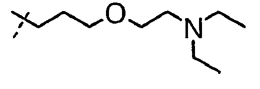 | VI.1 92,4 | (M+H)⁺ = 480 | 0,12 (A) |
| 1.092 | Me | 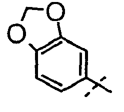 | 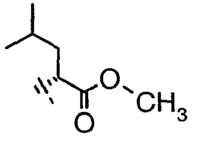 | VI.1 67,0 | (M+H)⁺ = 451 | 0,45 (A) |
| 1.093 | Me | 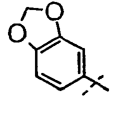 | 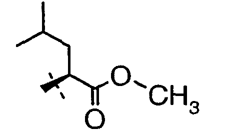 | VI.1 55,0 | (M+H)⁺ = 451 | 0,45 (A) |
| 1.094 | Me | 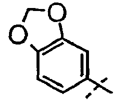 | 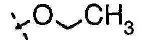 | VI.1 72,0 | (M+H)⁺ = 367 | 0,47 (A) |
| 1.095 | Me | 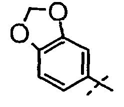 | 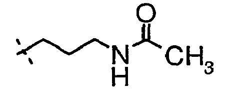 | VI.1 84,0 | (M+H)⁺ = 316 | 0,41 (A) |
| 1.096 | Me | 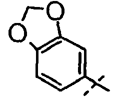 | 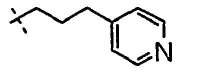 | VI.1 66,0 | (M-H)- = 440 | 0,53 (A) |
| 1.097 | Me | Ph | 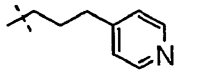 | III 60,0 | (M-H)- = 396 | 0,52 (A) |
| 1.098 | Me | Ph | 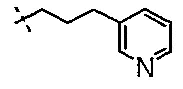 | III 59,0 | (M+H)⁺ = 398 | 0,60 (C) |
| 1.099 | Me | Ph | 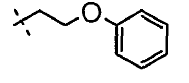 | III 85,0 | (M+H)⁺ = 399 | 0,59 (C) |
| 1.100 | Me | Ph | 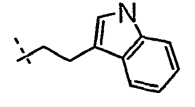 | III 84,0 | (M+H)⁺ = 422 | 0,52 (C) |
| 1.101 | Me | Ph | 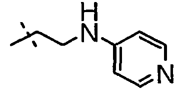 | III 70,0 | (M+H)⁺ = 399 | 0,25 (C) |
| 1.102 | Me | Ph | 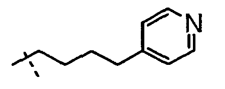 | III 73,0 | (M+H)⁺ = 412 | 0,42 (C) |
| 1.103 | Me | Ph | 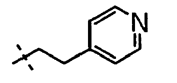 | III 89,0 | (M+H)⁺ = 384 | 0,41 (C) |
| 1.104 | Me | Ph | 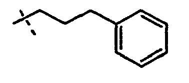 | III 86,0 | (M+H)⁺ = 397 | 0,58 (C) |
| 1.105 | Me | Ph | 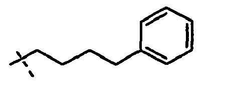 | III 81,0 | (M+H)⁺ = 411 | 0,44 (A) |
| 1.106 | Me | Ph | 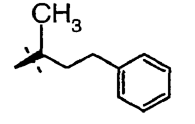 | III 49,0 | (M+H)⁺ = 411 | 0,43 (A) |
| 1.107 | Me | Ph | 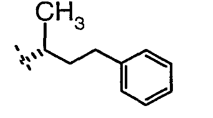 | III 40,0 | (M+H)⁺ = 411 | 0,41 (A) |
| 1.108 | Me | Ph | 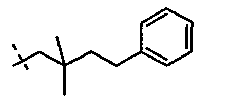 | III 73,0 | (M+H)⁺ = 439 | 0,46 (A) |
| 1.109 | Me | Ph | 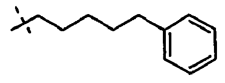 | III 90,0 | (M+H)⁺ = 425 | 0,49 (A) |
| 1.110 | Me | Ph | 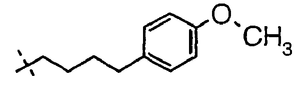 | III 90,0 | (M+H)⁺ = 441 | 0,40 (A) |
| 1.111 | Me | Ph | 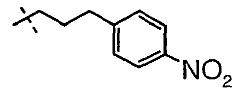 | III 93,0 | (M+H)⁺ = 442 | 0,32 (A) |
| 1.112 | Me | Ph | 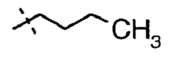 | III 78,0 | (M+H)⁺ = 335 | 0,50 (A) |
| 1.113 | Me | Ph | 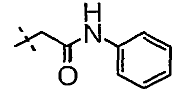 | III 89,0 | (M+H)⁺ = 412 | 0,50 (C) |
| 1.114 | Me | Ph | 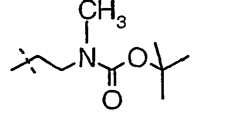 | III 87,0 | (M+H)⁺ = 436 | n.d. |
| 1.115 | Me | 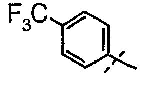 | 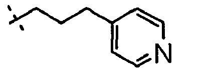 | VI.8 61,0 | (M+H)⁺ = 466 | 0,35 (A) |
| 1.116 | Me | 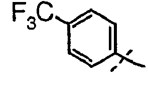 | 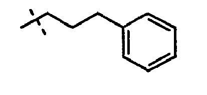 | VI.8 80,0 | (M+H)⁺ = 465 | 0,37 (A) |
| 1.117 | Me | 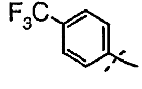 | 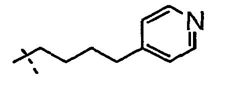 | VI.8 77,0 | (M+H)⁺ = 480 | 0,31 (A) |
| 1.118 | Me | 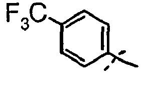 | 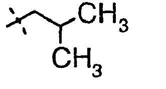 | VI.8 56,0 | (M+H)⁺ = 403 | 0,50 (A) |
| 1.119 | Me | Ph | 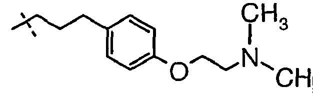 | III 80,0 | (M+H)⁺ = 484 | 0,45 (A) |
| 1.120 | Me | Ph | 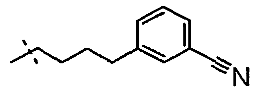 | III 10,0 | (M+H)⁺ = 436 | 0,60 (A) |
| 1.121 | Me | Ph | 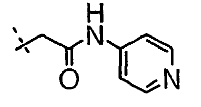 | III 75,0 | (M+H)⁺ = 413 | 0,43 (A) |
| 1.122 | Me | Ph | 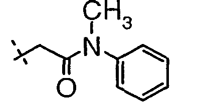 | III 30,0 | (M+H)⁺ = 426 | 0,48 (A) |
| 1.123 | Me | Ph | 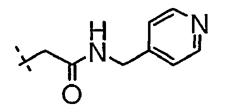 | III 66,0 | (M+H)⁺ = 427 | 0,55 (A) |
| 1.124 | Me | 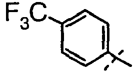 | 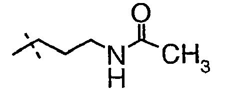 | VI.8 54,0 | (M+H)⁺ = 446 | 0,28 (A) |
| 1.125 | Me | 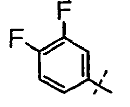 | 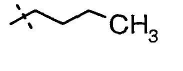 | VI.20 79,0 | (M+H)⁺ = 371 | 0,21 (A) |
| 1.126 | Me | 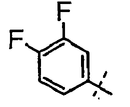 | 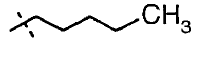 | VI.20 77,0 | (M+H)⁺ = 385 | 0,20 (A) |
| 1.127 | Me | Et | 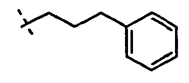 | III.3 73,0 | (M+H)⁺ = 349 | 0,44 (E) |
| 1.128 | Me | Et | 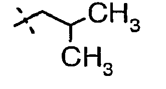 | III.3 69,0 | (M+H)⁺ = 287 | 0,48 (E) |
| 1.129 | Me | n-Pr | 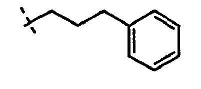 | III.4 94,0 | (M-H)-= 361 | 0,47 (A) |
| 1.130 | Me | n-Pr | 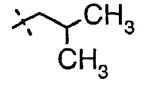 | III.4 93,0 | (M+H)⁺ = 301 | 0,51 (A) |
| 1.131 | Me | n-Pr | 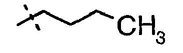 | III.4 74,0 | (M+H)⁺ = 301 | 0,63 (A) |
| 1.132 | Me | n-Pr | 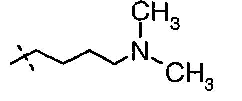 | III.4 47,0 | (M+H)⁺ = 344 | 0,31 (C) |
| 1.133 | Me | n-Pr | 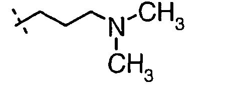 | III.4 38,0 | (M+H)⁺ = 330 | 0,62 (C) |
| 1.134 | Me | n-Pr | 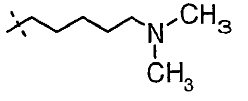 | III.4 45,0 | (M+H)⁺ = 358 | 0,51 (C) |
| 1.135 | Me | n-Bu | 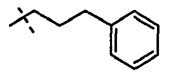 | III.5 94,0 | (M+H)⁺= 377 | 0,54 (E) |
| 1.136 | Me | n-Bu | 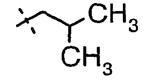 | III.5 86,0 | (M+H)⁺ = 315 | 0,69 (E) |
| 1.137 | Me | n-Bu | 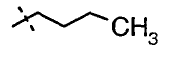 | III.5 81,0 | (M+H)⁺ = 315 | 0,69 (E) |
| 1.138 | Me | n-Bu | 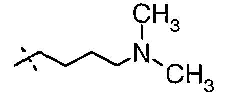 | III.5 59,0 | (M+H)⁺ = 358 | 0,22 (A) |
| 1.139 | Me | n-Bu | 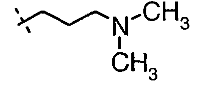 | III.5 55,0 | (M+H)⁺ = 344 | 0,22 (A) |
| 1.140 | Me | n-Bu | 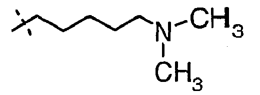 | III.5 42,0 | (M+H)⁺ = 372 | 0,12 (A) |
| 1.141 | Me | 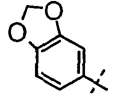 | 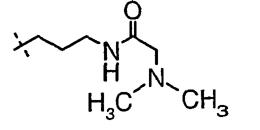 | VI.1 69,8 | (M+H)⁺ = 465 | 0,36 (A) |
| 1.142 | Me | 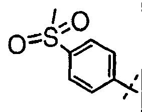 | 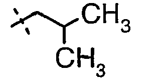 | VI.25 68,0 | (M+H)⁺ = 413 | 0,52 (A) |
| 1.143 | Me | 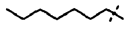 | 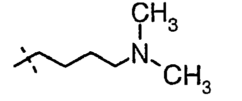 | VI.26 79,6 | (M+H)⁺ = 400 | 0,08 (A) |
| 1.144 | Me | 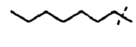 | 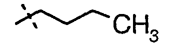 | VI.26 62,1 | (M+H)⁺ = 357 | 0,51 (A) |
| 1.145 | Me | 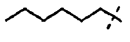 | 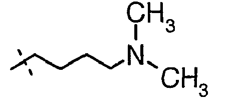 | VI.27 76,4 | (M+H)⁺ = 386 | 0,11 (A) |
| 1.146 | Me | 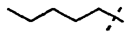 | 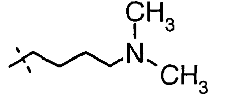 | VI.28 22,0 | (M+H)⁺ = 372 | 0,23 (C) |
| 1.147 | Me | 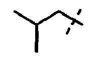 | 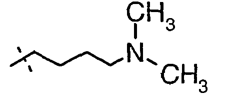 | VI.29 12,0 | (M+H)⁺ = 358 | 0,16 (C) |
| 1.148 | Et | Et | 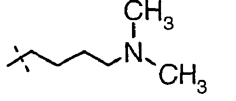 | III.7 70,0 | (M+H)⁺ = 344 | 0,11 (A) |
| 1.149 | Et | Et | 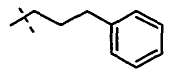 | III.7 87,0 | (M+H)⁺ = 363 | 0,45 (A) |
| 1.150 | n-C₅H₁₁ | Et | 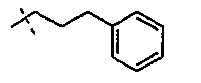 | III.8 73,0 | (M+H)⁺ = 405 | 0,47 (A) |
| 1.151 | Me | 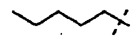 | 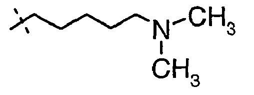 | VI.28 75,0 | (M+H)⁺ = 486 | 0,27 (C) |
| 1.152 | Me | 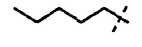 | 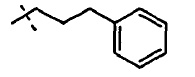 | VI.28 58,0 | (M+H)⁺ = 391 | 0,46 (A) |
| 1.153 | Me | 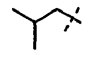 | 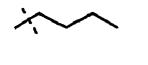 | VI.29 37,0 | (M+H)⁺ = 315 | 0,66 (A) |
| 1.154 | Me | 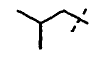 | 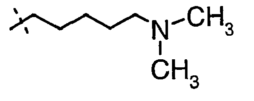 | VI.29 51,0 | (M+H)⁺ = 372 | 0,24 (A) |
| 1.155 | Me | 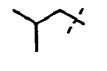 | 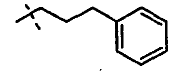 | VI.29 50,0 | (M+H)⁺ = 377 | 0,47 (C) |

### Beispiel 2

### 5-Acetyl-3-[benzo[1,3]dioxol-5-yl-(3-methylamino-prooylamino)-methyliden]-2-indolinon

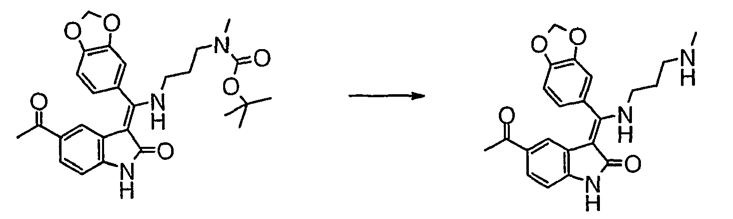

680 mg (1,38 mmol) 5-Acetyl-3-[benzo[1,3]dioxol-5-yl-(3-(N-tert-butoxycarbonyl-N-methyl-amino)-propylamino)-methyliden]-2-indolinon (Beispiel 1.073) werden portionsweise zu einer Lösung von 2 ml Trifluoressigsäure in 20 ml Methylenchlorid gegeben und 5 h bei Raumtemperatur gerührt. Anschließend wird eingeengt, der Rückstand in Methylenchlorid ausgenommen, mit 1 N Natronlauge alkalisch gestellt und dann die organische Phase abgetrennt, über Natriumsulfat getrocknet und eingeengt.
Ausbeute: 210 mg (38 % der Theorie)
R_{f} = 0,05 (Kieselgel, Methylenchlorid/Methanol 9:1)
C₂₂H₂₃N₃O₄ (MG = 393,441)
Massenspektrum: m/z = 394 (M+H)⁺

Analog Beispiel 2 werden folgende Verbindungen der Formel I hergestellt:

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 2.001 | Me | Ph | 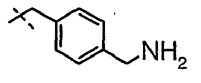 | 1.013 65,1 | (M+H)⁺ = 398 | 0,14 (B) |
| 2.002 | Me | Ph | 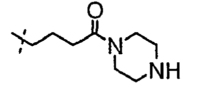 | 4.014 52,9 | (M+H)⁺ = 433 | 0,48 (C) |
| 2.003 | Me | Ph | 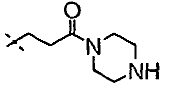 | 4.016 56,7 | (M+H)⁺ = 419 | 0,38 (C) |
| 2.004 | Me | Ph | 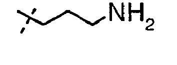 | 1.041 53,2 | (M+H)⁺ = 336 | 0,40 (C) |
| 2.005 | Me | 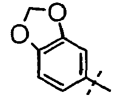 | 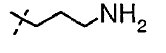 | 1.061 97,3 | (M+H)⁺ = 380 | 0,08 (A) |
| 2.006 | Me | 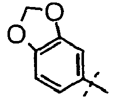 | 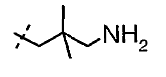 | 1.080 81,1 | (M+H)⁺ = 408 | 0,1 (A) |
| 2.007 | Me | Ph | 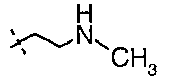 | 1.114 82,0 | (M+H)⁺ = 336 | 0,32 (C) |

### Beispiel 3

### 5-Acetyl-3-[(carboxymethyl-amino)-phenyl-methylidenl-2-indolinon

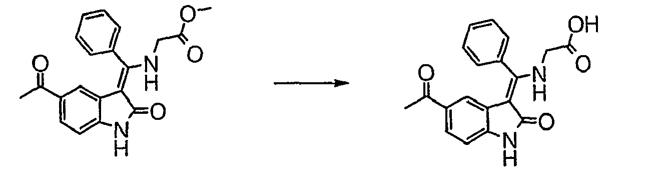

88 mg (0,25 mmol) 5-Acetyl-3-[(methoxycarboxymethyl-amino)-phenyl-methyliden]-2-indolinon (Beispiel 1.005) werden in 0,5 ml 1 N Natronllauge und 5 ml Methanol suspendiert und 4 h unter Rückfluß gekocht. Anschließend läßt man abkühlen, gibt 0,5 ml 1 N Salzsäure zu und saugt den Niederschlag ab.
Ausbeute: 81 mg (95 % der Theorie)
C₁₉H₁₆N₂O₄ (MG = 336,345)
Massenspektrum: m/z = 337 (M+H)⁺

Analog Beispiel 3 werden folgende Verbindungen der Formel I hergestellt:

| Beispiel | R¹ | R² | R³ | Edukt Ausbeute [%] | Massenspektrum (ES) m/z | R*_{f}*-Wert (Kieselgel) (Fließmittel) |
|---|---|---|---|---|---|---|
| 3.001 | Me | Ph | 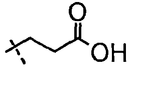 | 1.004 91,3 | (M+H)⁺ = 351 | nicht bestimmt |
| 3.002 | Me | Ph | 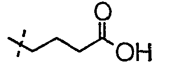 | 1.014 34,6 | (M+H)⁺ = 365 | 0,19 (A) |
| 3.003 | Me | Ph | 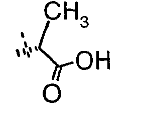 | 1.012 88,5 | (M-H)⁻ = 349 | 0,47 (A + Eisessig) |
| 3.004 | Me | Ph | 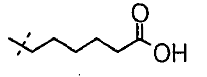 | 1.022 57,0 | (M+H)⁺ = 393 | 0,26 (A) |
| 3.005 | Me | 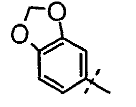 | 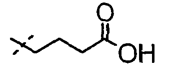 | 1.025 85,5 | (M+H)⁺ = 409 | 0,31 (A) |
| 3.006 | Me | 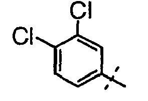 | 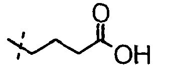 | 1.026 42,5 | (M-H)⁻ = 431/433/ 435 (Cl2) | 0.31 (A) |
| 3.007 | Me | 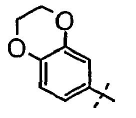 | 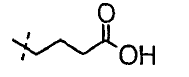 | 1.036 82,7 | (M-H)⁻ = 421 | 0,33 (A) |
| 3.008 | Me | 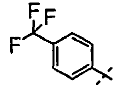 | 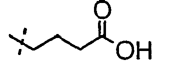 | 1.037 41,3 | (M-H)⁻ = 431 | 0,22 (A) |
| 3.009 | Et | 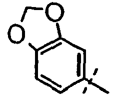 | 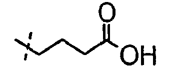 | 1.087 82,6 | (M+H)⁺ = 423 | 0,78 (B) |
| 3.010 | Me | 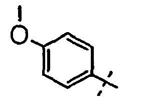 | 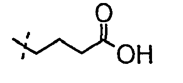 | 1.038 93,1 | (M-H)⁻ = 393 | 0,7 (B) |
| 3.011 | Me | 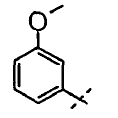 | 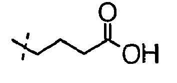 | 1.039 79,2 | (M-H)⁻ = 393 | 0,33 (A) |
| 3.012 | Me | 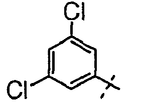 | 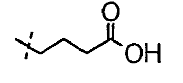 | 1.042 90,9 | (M+H)⁺ = 433/435/ 437 (Cl2) | 0,32 (A) |
| 3.013 | Me | 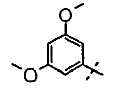 | 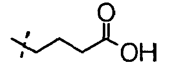 | 1.043 77,5 | (M+H)⁺ = 425 | 0,27 (A) |
| 3.014 | Me | 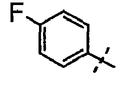 | 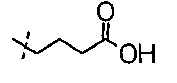 | 1.047 79,4 | (M+H)⁺ = 383 | 0,38 (A) |
| 3.015 | Me | 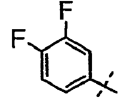 | 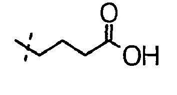 | 1.049 78,4 | (M+H)⁺ = 401 | 0,36 (A) |
| 3.016 | Me | 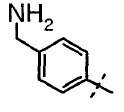 | 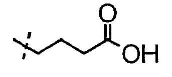 | 7.004 19,5 | (M+H)⁺ = 394 | 0,09 (A + Eisessig) |
| 3.017 | Me | 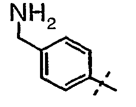 | 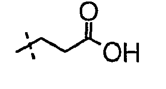 | 7.012 95,5 | (M+H)⁺ = 380 | 0,05 (A + Eisessig) |
| 3.018 | Me | 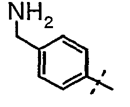 | 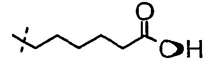 | 7.010 56,3 | (M+H)⁺ = 442 | 0,02 (C) |
| 3.019 | Me | 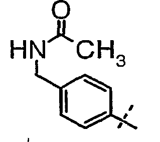 | 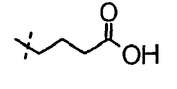 | 6.006 98 | (M+H)⁺ = 436 | 0,39 (A + Eisessig) |
| 3.020 | Me | 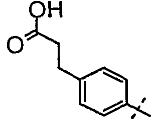 | 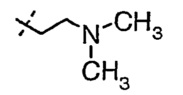 | 1.072 82,5 | (M+H)⁺ = 422 | 0,45 (MeOH) |
| 3.021 | Me | 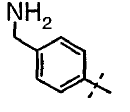 | 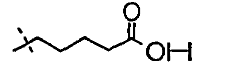 | 5.014 93,4 | (M+H)⁺ = 408 | 0,39 (MeOH) |
| 3.022 | Me | 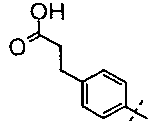 | 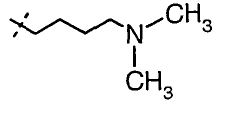 | 1.077 85,9 | (M+H)⁺ = 450 | 0,24 (MeOH) |
| 3.023 | Me | 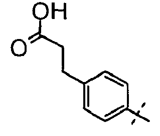 | 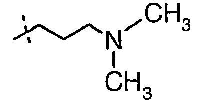 | 1.078 72,4 | (M+H)⁺ = 436 | 0,22 (MeOH) |
| 3.024 | Me | 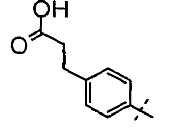 | 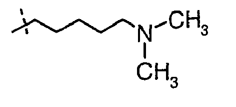 | 1.079 57,4 | (M-H)⁻ = 462 | 0,15 (MeOH) |
| 3.025 | Me | 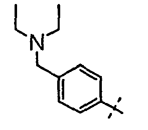 | 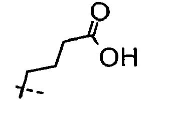 | 8.004 89,0 | (M+H)⁺ = 450 | 0,56 (A) |

### Beispiel 4

### 5-Acetyl-3-{[3-(N,N-bis-(2-hydroxyethyl)-carbamoyl)-propylamino]-phenyl-methyliden}-2-indolinon

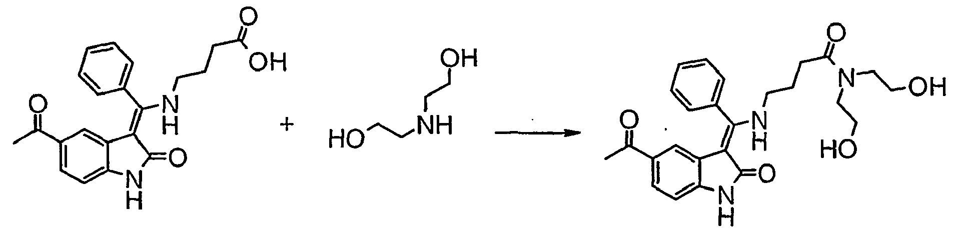

100 mg (0,27 mmol) 5-Acetyl-3-[3-(carboxy-propylamino)-phenyl-methyliden]-2-indolinon (Bsp. 3.002) werden mit 45 mg CDI (Carbonyldiimidazol) in 3 ml Tetrahydrofuran 3 h lang bei 60 °C gerührt. Anschließend werden 30 mg Diethanolamin (0,28 mmol) zugegeben und bei Raumtemperatur über Nacht gerührt. Dann wird der Ansatz eingeengt, der Rückstand in Essigsäureethylester aufgenommen, mit Wasser gewaschen und die organische Phase über Natriumsulfat getrocknet. Anschließend wird einrotiert.
Ausbeute: 42 mg (34 % der Theorie)
R_{f} = 0,23 (Kieselgel, Methylenchlorid/Methanol 9:1)
C₂₅H₂₉N₃O₅ (MG = 451,52)
Massenspektrum: m/z = 452 (M+H)⁺

Analog Beispiel 4 werden folgende Verbindungen der Formel I hergestellt:

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 4.001 | Me | Ph | 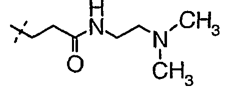 | 3.001 29,2 | (M+H)⁺ = 421 | 0,12 (CH₂Cl₂ /MeOH 1:1) |
| 4.002 | Me | Ph | 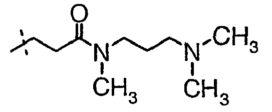 | 3.001 54,8 | (M+H)⁺ = 449 | 0,27 (C) |
| 4.003 | Me | Ph | 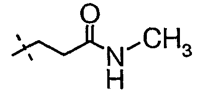 | 3.001 29,0 | (M+H)⁺ = 364 | 0,24 (A) |
| 4.004 | Me | Ph | 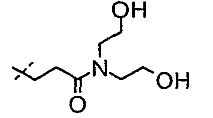 | 3.001 16,0 | (M+H)⁺ = 438 | 0,16 (A) |
| 4.005 | Me | Ph | 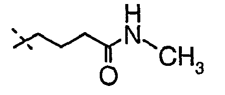 | 3.002 38,7 | (M+H)⁺ = 378 | 0,35 (C) |
| 4.006 | Me | Ph | 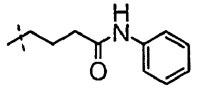 | 3.002 24,9 | (M+H)⁺ = 440 | 0,33 (A) |
| 4.007 | Me | Ph | 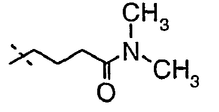 | 3.002 25,9 | (M+H)⁺ = 392 | 0,54 (A) |
| 4.008 | Me | Ph | 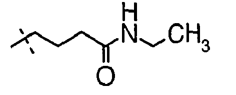 | 3.002 56,9 | (M-H)⁻ = 390 | 0,57 (A) |
| 4.009 | Me | Ph | 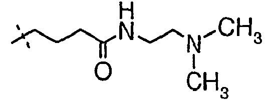 | 3.002 59,0 | (M+H)⁺ = 435 | 0,53 (C) |
| 4.010 | Me | Ph | 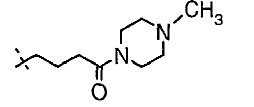 | 3.002 90,2 | (M+H)⁺ = 447 | 0,28 (A) |
| 4.011 | Me | Ph | 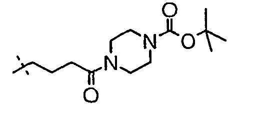 | 3.002 26,2 | (M+H)⁺ = 533 | 0,47 (A) |
| 4.012 | Me | Ph | 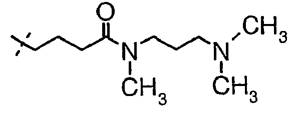 | 3.002 47,5 | (M+H)⁺ = 463 | 0,34 (C) |
| 4.013 | Me | Ph | 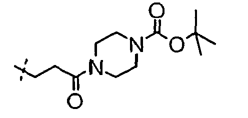 | 3.001 14,8 | (M+H)⁺ = 519 | 0,47 (A) |
| 4.014 | Me | Ph | 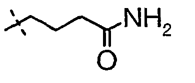 | 3.002 51,0 | (M+H)⁺ = 364 | 0,72 (A) |

### Beispiel 5

### 5-Acetyl-3-[(4-aminomethyl-phenyl)-(3-dimethylamino-propylylamino)-methyliden]-2-indolinon

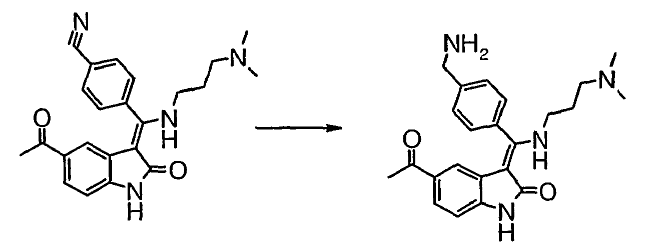

200 mg (0,51 mmol) 5-Acetyl-3-[(4-cyano-phenyl)-(3-dimethylamino-propylamino)-methyliden]-2-indolinon (Beispiel 1.054) werden in 13 ml methanolischem Ammoniak gelöst, mit 80 mg Raneynickel versetzt und bei Raumtemperatur 6 h bei einem Druck von 50 psi hydriert. Anschließend wird der Katalysator abfiltriert und die Lösung eingeengt. Der Rückstand wird über eine Kieselgelsäule mit Methylenchlorid:Methanol 30:1 chromatographiert. Die gewünschte Fraktion wird gesammelt und eingeengt.
Ausbeute: 180 mg (89 % der Theorie)
R_{f} = 0,19 (Kieselgel, Methylenchlorid/Methanol/konz. Ammoniak 9:1:0,1)
C₂₃H₂₈N₄O₂ (MG = 392,5)
Massenspektrum: m/z = 393 (M+H)⁺

Analog Beispiel 5 werden folgende Verbindungen der Formel I hergestellt:

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 5.001 | Me | 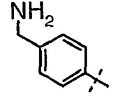 | 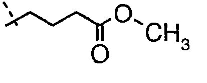 | 1.053 68,9 | (M+H)⁺ = 408 | 0,39 (C) |
| 5.002 | Me | 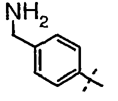 | 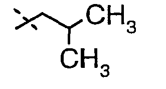 | 1.055 45,0 | (M+H)⁺ = 364 | 0,5 (C) |
| 5.003 | Me | 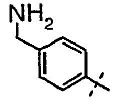 | n-Pr | 1.056 49,4 | (M+H)⁺ = 350 | 0,44 (C) |
| 5.004 | Me | 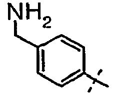 | 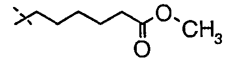 | 1.057 84,9 | nicht bestimmt | 0,43 (C) |
| 5.005 | Me | 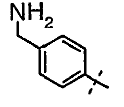 | 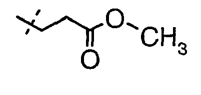 | 1.058 57,4 | (M+H)⁺ = 394 | 0,23 (C) |
| 5.006 | Me | 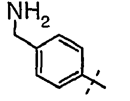 | 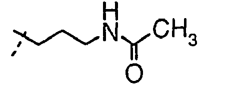 | 1.062 88,6 | (M+H)⁺ = 407 | 0,26 (C) |
| 5.007 | Me | 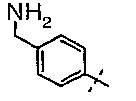 | 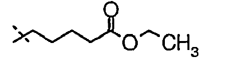 | 1.059 68,7 | (M+H)⁺ = 436 | 0,53 (C) |

### Beispiel 6

### 5-Acetyl-3-{benzo[1,3]dioxol-5-yl-[3-(N-methyl-N-acetyl-amino)-propylamino]-methyliden}-2-indolinon

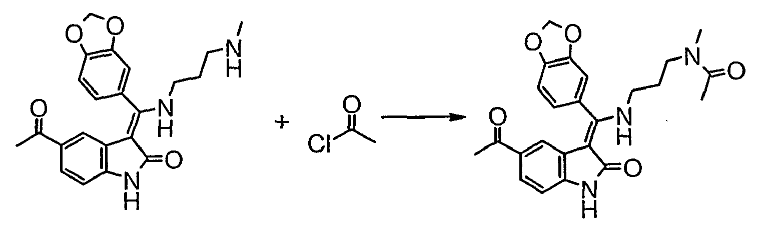

150 mg (0,38 mmol) 5-Acetyl-3-[benzo[1,3]dioxol-5-yl-(3-methylamino-propylamino)-methyliden]-2-indolinon (Beispiel 2) werden in 4 ml Methylenchlorid vorgelegt und mit 54 µl Triethylamin versetzt. Zu dieser Lösung tropft man unter Eiskühlung 28 µl (0,39 mmol) Acetylchlorid und läßt 10 min nachrühren. Anschließend lässt man den Ansatz auf Raumtemperatur erwärmen und rührt 1 h nach. Dann wird die Lösung mit Wasser gewaschen, die organsiche Phase über Natriumsulfat getrocknet, abgesaugt und einrotiert. Der Rückstand wird über eine Kieselgelsäule mit Essigester/Cyclohexan/Methanol 9:9:2 eluiert. Die gewünschte Fraktion wird gesammelt und eingeengt.
Ausbeute: 45 mg (27 % der Theorie)
R_{f} = 0,51 (Kieselgel, Methylenchlorid/Methanol 9:1)
C₂₄H₂₅N₃O₅ (MG = 435,478)
Massenspektrum: m/z = 436 (M+H)⁺

Analog Beispiel 6 werden folgende Verbindungen hergestellt:

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 6.001 | Me | Ph | 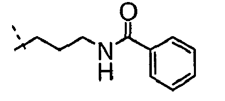 | 2.004 24,8 | (M+H)⁺ = 440 | 0,30 (C) |
| 6.002 | Me | Ph | 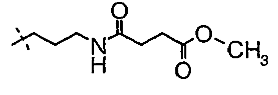 | 2.004 10,1 | (M+H)⁺ = 450 | 0,45 (Essigester/ MeOH 80:1) |
| 6.003 | Me | 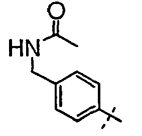 | 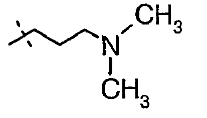 | 5.000 53,4 | (M+H)⁺ = 435 | 0,30 (C) |
| 6.004 | Me | 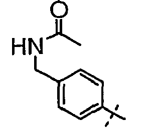 | 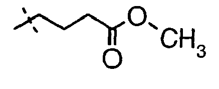 | 5.001 45,4 | (M+H)⁺ = 450 | 0,54 (C) |
| 6.005 | Me | 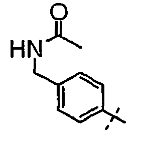 | 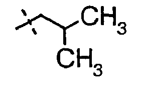 | 5.002 19,2 | (M+H)⁺ = 406 | 0,41 (C) |
| 6.006 | Me | 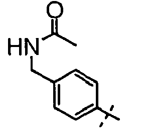 | n-Pr | 5.003 71,4 | (M+H)⁺ = 392 | 0,53 (C) |
| 6.007 | Me | 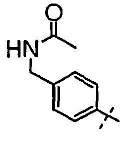 | 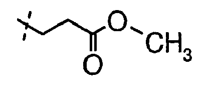 | 5.005 81,3 | (M+H)⁺ = 436 | 0,35 (C) |
| 6.008 | Me | 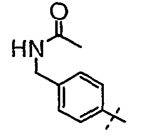 | 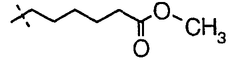 | 5.004 39,6 | (M+H)⁺ = 478 | 0,56 (C) |
| 6.009 | Me | 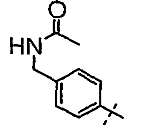 | 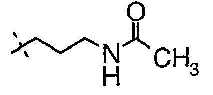 | 5.006 24,2 | (M+H)⁺ = 449 | 0,21 (A) |
| 6.010 | Me | 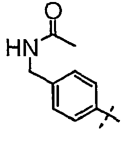 | 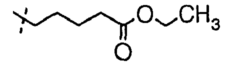 | 5.007 56,9 | (M+H)⁺ = 478 | 0,50 (A) |
| 6.011 | Me | Ph | 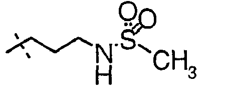 | 2.004 25,3 | (M+H)⁺ = 414 | 0,28 (C) |
| 6.012 | Me | Ph | 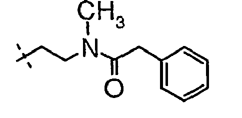 | 2.007 67,0 | (M+H)⁺ = 454 | 0,41 (A) |

### Beispiel 7

### 5-Acetyl-3-[(pyrazin-2-yl)-isobutylamino-methyliden]-2-indolinon

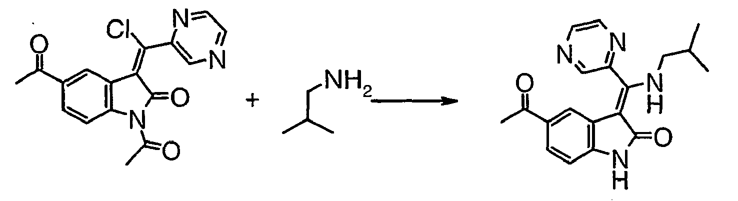

80 mg (0,23 mmol) 1,5-Diacetyl-3-[chloro-(pyrazin-2-yl)-methyliden]-2-indolinon (Bsp. VII) werden in 4 ml Tetrahydrofuran mit 0,05 ml Triethylamin und 0,022 g Isobutylamin bei Raumtemperatur 2 h gerührt. Das acetylgeschützte Zwischenprodukt wird ohne Reinigung mit 0,8 ml konz. Ammoniak versetzt eine halbe Stunde bei Raumtemperatur gerührt. Anschließend wird eingeengt und der Rückstand über eine Kieselgelsäule mit dem Fließmittel Methylenchlorid/Methanol 40:1 chromatographiert.
Ausbeute: 29 mg (36 % der Theorie)
R_{f} = 0,56 (Kieselgel, Methylenchlorid/Methanol 9:1)
C₁₉H₂₀N₄O₂ (MG = 336,393)
Massenspektrum: m/z = 337 (M+H)⁺

### Beispiel 8

### 5-Acetyl-3-[(pyridin-4-yl)-(isobutylamino)-methyliden]-2-indolinon

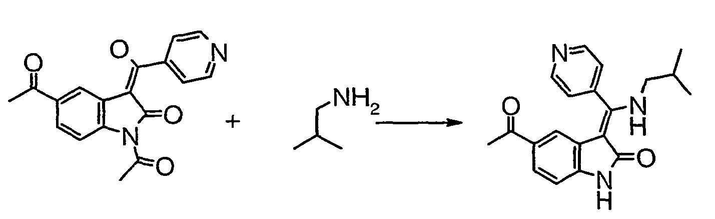

500 mg (1,55 mmol) 1,5-Diacetyl-3-[(pyridin-4-yl)-hydroxy-methyliden]-2-indolinon (Bsp. V.24) werden in 2,4 ml Hexamethyldisilazan mit 0,23 g 4-Amino-1-methylpiperidin 3 h bei 120 °C erhitzt. Anschließend lässt man abkühlen und gibt 10 ml Methanol und 32 mg Natriummethylat zu und rührt 1 h bei Raumtemperatur. Dann wird eingeengt und der Rückstand über eine Kieselgelsäule mit dem Fließmittel Methylenchlorid/ Methanol 12:1 chromatographiert.
Ausbeute: 160 mg (31 % der Theorie)
R_{f} = 0,56 (Kieselgel, Methylenchlorid/Methanol 9:1)
C₂₀H₂₁N₃O₂ (MG = 335,405)
Massenspektrum: m/z = 336 (M+H)⁺

Analog Beispiel 8 wird folgende Verbindung der Formel I hergestellt:

| Bei-spiel | R¹ | R² | R³ | Edukt Ausbeute [%] | Massenspektrum (ES) m/z | R_{f}-Wert (Kieselgel) (Fließmittel) |
|---|---|---|---|---|---|---|
| 8.001 | Me | 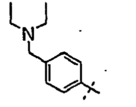 | 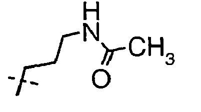 | V.26 26,4 | (M+H)⁺ = 463 | 0,58 (C) |
| 8.002 | Me | 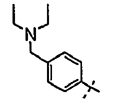 | 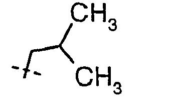 | V.26 38,8 | (M+H)⁺ = 420 | 0,60 (A) |
| 8.003 | Me | 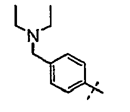 | 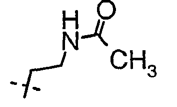 | V.26 13,6 | (M+H)⁺ = 449 | 0,49 (C) |
| 8.004 | Me | 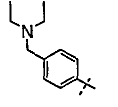 | 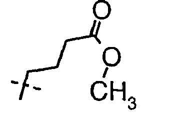 | V.26 43,8 | (M+H)⁺ = 464 | 0,54 (C) |
| 8.005 | Me | 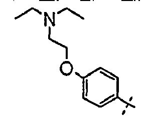 | 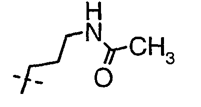 | V.29 21,0 | (M+H)⁺ = 493 | 0,41 (B) |
| 8.006 | Me | 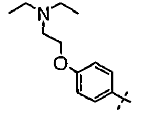 | 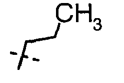 | V.29 12,0 | (M+H)⁺ = 436 | 0,85 (B) |
| 8.007 | Me | 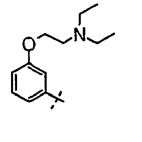 | 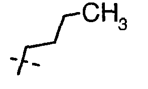 | V.30 Trimet hylimi dazol 70,0 | (M+H)⁺ = 450 | 0,68 (C) |
| 8.008 | Me | 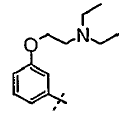 | 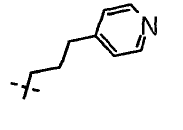 | V.30 Trimet hylimi dazol 54,0 | (M+H)⁺ = 513 | 0,68 (C) |

### Beispiel 9

### 5-Acetyl-3-[furan-3-yl-isobutylamino-methyliden]-2-indolinon

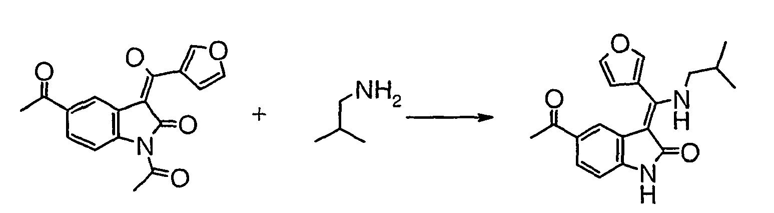

200 mg (0,65 mmol) 1,5-Diacetyl-3-[furan-3-yl-methoxy-methyliden]-2-indolinon (Bsp. VI.23) werden in 5 ml Dimethylformamid suspendiert und mit 61 mg Isobutylamin bei Raumtemperatur 2 h lang gerührt. Das acetylgeschützte Zwischenprodukt wird ohne Reinigung mit 1 ml konz. Ammoniak versetzt und man rührt bei Raumtemperatur 30 min. Anschließend wird eingeengt und der Rückstand über eine Kieselgelsäule mit dem Fließmittel Methylenchlorid/Methanol 4:1 chromatographiert.
Ausbeute: 78 mg (37 % der Theorie)
R_{f} = 0,2 (Kieselgel, Methylenchlorid/Methanol 30:1)
C₁₉H₂₀N₂O₃ (MG = 324,383)
Massenspektrum: m/z = 325 (M+H)⁺

### Beispiel 10

### 5-Acetyl-3-[1-(N',N'-Dimethylhydrazino)-1-(4-trifluormethyl-phenyl)-methyliden]-2-indolinon

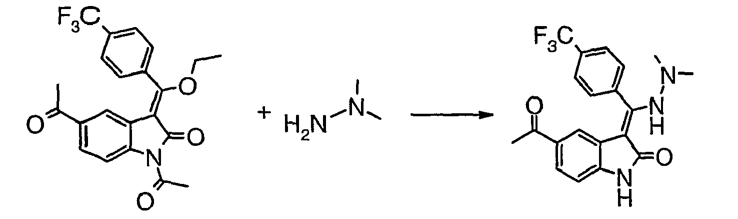

0,2 g (0,4 mmol) 1,5-Diacetyl-3-[(4-trifluormethylphenyl)-ethoxy-methyliden]-2-indolinon (Bsp. VI.8) werden in 4 ml Dimethylformamid suspendiert und mit 0,038 ml Dimethylhydrazin bei Raumtemperatur 5 h gerührt. Dann gibt 2 ml 1 N Natronlauge zu und rührt bei Raumtemperatur 30 min. Das Reaktionsgemisch wird auf 10 ml Wasser gegossen, der Niederachlag abfiltiert und im Exsikkator getrocknet. Gegebenenfalls kann das Produkt über eine Kieselgelsäule mit dem Laufmittel Methylenchlorid/Methanol 30:1 gereinigt werden.
Ausbeute: 0,11 g (57 % der Theorie)
R_{f} = 0,55 (Kieselgel, Methylenchlorid/Methanol 9:1)
C₂₀H₁₈F₃N₃O₂ (MG = 389,371)
Massenspektrum: m/z = 390 (M+H)⁺

Analog Beispiel 10 werden folgende Verbindungen hergestellt:

| Beispiel | R¹ | R² | R³ | Edukt Ausbeute [%] | Massenspektrum (ES) m/z | R_{f}-Wert (Kieselgel) (Fließmittel) |
|---|---|---|---|---|---|---|
| 10.001 | Me | 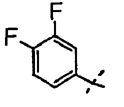 | 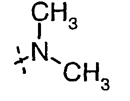 | VI.20 42,0 | (M+H)⁺ = 358 | 0,59 (A) |
| 10.002 | Me | 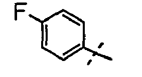 | 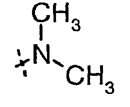 | VI.19 36,0 | (M+H)⁺ = 340 | 0,24 (A) |
| 10.003 | Me | 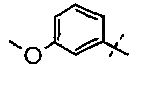 | 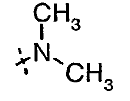 | VI.10 16,0 | (M+H)⁺ = 352 | 0,48 (A) |
| 10.004 | Et | 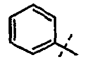 | 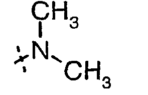 | IV.3 81,0 | (M+H)⁺ = 336 | 0,34 (A) |
| 10.005 | Me | 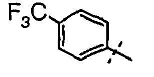 | 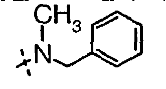 | VI.8 35,0 | (M+H)⁺ = 466 | 0,54 (A) |
| 10.006 | Me | Et | 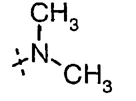 | III.3 63,0 | (M+H)⁺ = 274 | 0,79 (A) |
| 10.007 | Me | 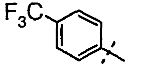 | 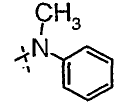 | VI.8 14,0 | (M+H)⁺ = 438 | 0,50 (A) |
| 10.008 | Me | 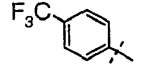 | 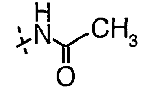 | VI.8 45,0 | (M+H)⁺ = 404 | 0,41 (A) |
| 10.009 | Me | 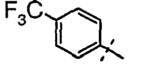 | 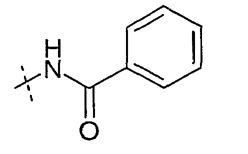 | VI.8 43,0 | (M+H)⁺ = 466 | 0,43 (A) |
| 10.010 | Me | 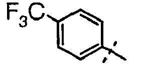 | 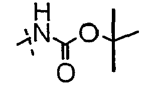 | VI.8 29,0 | (M+H)⁺ = 460 | 0,43 (A) |
| 10.011 | Me | 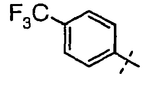 | 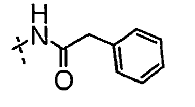 | VI.8 67,0 | (M+H)⁺ = 480 | 0,54 (A) |
| 10.012 | Me | Et | 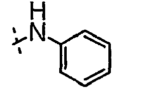 | III.3 53,0 | (M+H)⁺ = 322 | 0,53 (A) |
| 10.013 | Me | Et | 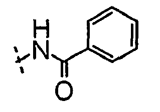 | III.3 58,0 | (M+H)⁺ = 350 | 0,40 (A) |
| 10.014 | Me | Et | 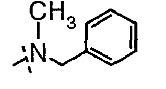 | III.3 62,0 | (M+H)⁺ = 350 | 0,47 (A) |
| 10.015 | Me | 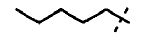 | 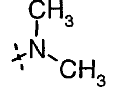 | VI.28 72,0 | (M+H)⁺ = 316 | 0,67 (C) |
| 10.016 | Me | 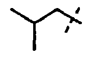 | 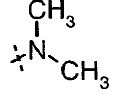 | VI.29 42,0 | (M+H)⁺ = 302 | 0,47 (C) |

### Beispiel 11

### Dragees mit 75 mg Wirksubstanz

### 1 Drageekern enthält:

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1.5 mg |
| | 230,0 mg |

### Herstellung:

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb mit 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

| | |
|---|---|
| Kerngewicht: | 230 mg |
| Stempel: | 9 mm, gewölbt |

Die so hergestellten Dragéekerne werden mit einem Film überzogen, der im wesentlichen aus Hydroxypropylmethylcellulose besteht. Die fertigen Filmdragées werden mit Bienenwachs geglänzt.
Dragéegewicht: 245 mg.

### Beispiel 12

### Tabletten mit 100 mg Wirksubstanz

### Zusammensetzung:

### 1 Tablette enthält:

| | |
|---|---|
| Wirksubstanz | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

### Herstellungverfahren:

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50°C wird erneut gesiebt (1,5 mm-Maschenweite) und das Schmiermittel zugemischt. Die preßfertige Mischung wird zu Tabletten verarbeitet.
Tablettengewicht: 220 mg
Durchmesser: 10 mm, biplan mit beidseitiger Facette und einseitiger Teilkerbe.

### Beispiel 13

### Tabletten mit 150 mg Wirksubstanz

### Zusammensetzung:

### 1 Tablette enthält:

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

### Herstellung:

Die mit Milchzucker, Maisstärke und Kieselsäure gemischte Wirksubstanz wird mit einer 20%igen wäßrigen Polyvinylpyrrolidonlösung befeuchtet und durch ein Sieb mit 1,5 mm-Maschenweite geschlagen.
Das bei 45°C getrocknete Granulat wird nochmals durch dasselbe Sieb gerieben und mit der angegebenen Menge Magnesiumstearat gemischt. Aus der Mischung werden Tabletten gepreßt.

| | |
|---|---|
| Tablettengewicht: | 300 mg |
| Stempel: | 10 mm, flach |

### Beispiel 14

### Hartgelatine-Kapseln mit 150 mg Wirksubstanz

### 1 Kapsel enthält:

| | |
|---|---|
| Wirkstoff | 150,0 mg |
| Maisstärke getr. | ca. 180,0 mg |
| Milchzucker pulv. | ca. 87,0 mg |
| Magnesiumstearat | 3,0 mg |
| | ca. 420,0 mg |

### Herstellung:

Der Wirkstoff wird mit den Hilfsstoffen vermengt, durch ein Sieb von 0,75 mm-Maschenweite gegeben und in einem geeigneten Gerät homogen gemischt. Die Endmischung wird in Hartgelatine-Kapseln der Größe 1 abgefüllt.

| | |
|---|---|
| Kapselfüllung: | ca. 320 mg |
| Kapselhülle: | Hartgelatine-Kapsel Größe 1. |

### Beispiel 15

### Suppositorien mit 150 mg Wirksubstanz

### 1 Zäpfchen enthält:

| | |
|---|---|
| Wirkstoff | 150,0 mg |
| Polyethylenglykol 1500 | 550,0 mg |
| Polyethylenglykol 6000 | 460,0 mg |
| Polyoxyethylensorbitanmonostearat | 840,0 mg |
| | 2000,0 mg |

### Herstellung:

Nach dem Aufschmelzen der Suppositorienmasse wird der Wirkstoff darin homogen verteilt und die Schmelze in vorgekühlte Formen gegossen.

### Beispiel 16

### Suspension mit 50 mg Wirksubstanz

### 100 ml Suspension enthalten:

| | |
|---|---|
| Wirkstoff | 1,00 g |
| Carboxymethylcellulose-Na-Salz | 0,10 g |
| p-Hydroxybenzoesäuremethylester | 0,05 g |
| p-Hydroxybenzoesäurepropylester | 0,01 g |
| Rohrzucker | 10,00 g |
| Glycerin | 5,00 g |
| Sorbitlösung 70%ig | 20,00 g |
| Aroma | 0,30 g |
| Wasser dest. | ad 100 ml |

### Herstellung:

Dest. Wasser wird auf 70°C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

### Beispiel 17

### Ampullen mit 10 mg Wirksubstanz

### Zusammensetzung:

| | |
|---|---|
| Wirkstoff | 10,0 mg |
| 0,01 n Salzsäure s.q. | |
| Aqua bidest | ad 2,0 ml |

### Herstellung:

Die Wirksubstanz wird in der erforderlichen Menge 0,01 n HCl gelöst, mit Kochsalz isotonisch gestellt, sterilfiltriert und in 2 ml Ampullen abgefüllt.

### Beispiel 18

### Ampullen mit 50 mg Wirksubstanz

### Zusammensetzung:

| | |
|---|---|
| Wirkstoff | 50,0 mg |
| 0,01 n Salzsäure s.q. | |
| Aqua bidest | ad 10,0 ml |

### Herstellung:

Die Wirksubstanz wird in der erforderlichen Menge 0,01 n HCl gelöst, mit Kochsalz isotonisch gestellt, sterilfiltriert und in 10 ml Ampullen abgefüllt.

## Patentansprüche

1. Verbindungen der allgemeinen Formel 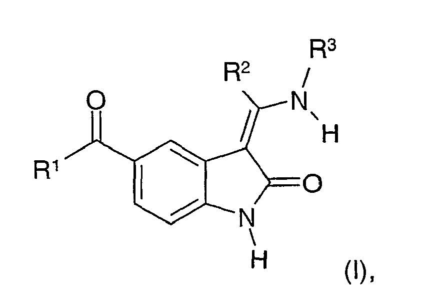 in der
R¹ eine lineare oder verzweigte C₁₋₅-Alkylgruppe, in der die Wasserstoffatome ganz oder teilweise durch Fluoratome ersetzt sein können, oder
eine gegebenenfalls durch ein Fluor-, Chlor- oder Bromatom substiuierte Arylgruppe,
wobei unter einer Arylgruppe eine Phenyl- oder Naphthylgruppe zu verstehen ist,
R² eine C₁₋₇-Alkyl- oder C₃₋₇-Cycloalkylgruppe,
eine gegebenenfalls durch ein oder zwei Fluor-, Chlor-, Brom- oder lodatome oder ein oder zwei Nitro-, Cyano-, Amino-, C₁₋₃-Alkyl- oder C₁₋₃-Alkoxygruppen substituierte 5- oder 6-gliedrige Heteroarylgruppe mit ein bis drei Heteroatomen ausgewählt aus der Gruppe N, S und O, wobei sowohl die Heteroatome als auch die Substituenten gleich oder verschieden sein können,
eine Phenylgruppe, in der zwei benachbarte Kohlenstoffatome über eine Methylendioxy-, Ethylendioxy- oder Difluormethylendioxygruppe miteinander verknüpft sind, eine Phenylgruppe, an die ein weiterer Phenylring oder ein 5- oder 6-gliedriger heteroaromatischer Ring mit ein bis drei Heteroatomen ausgewählt aus der Gruppe N, S und O, wobei die Heteroatome gleich oder verschieden sein können, und wobei der Bicyclus durch ein oder zwei Fluor-, Chlor-, Brom- oder Iodatome oder ein oder zwei Nitro-, Cyano-, Amino-, C₁₋₃-Alkyl- oder C₁₋₃-Alkoxygruppen substituiert sein kann und die Substituenten gleich oder verschieden sein können, anneliert ist, oder
eine Phenylgruppe, die durch ein bis drei Fluor-, Chlor-, Brom- oder Iodatome oder durch eine bis drei C₁₋₃-Alkyl-, Nitro-, Cyano-, Amino-, Di-(C₁₋₃-alkyl)-amino-, C₁₋₃-Alkyl-carbonylamino-, Phenylcarbonylamino-, C₁₋₃-Alkylsulfonylamino-, Arylsulfonylamino-, Trifluormethyl-, C₁₋₃Alkylsulfonyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylaminocarbonyl-, Hydroxy-carbonyl-C₁₋₃-alkyl-aminocarbonyl-, C₁₋₃-Alkoxycarbonyl-C₁₋₃-alkyl-aminocarbonyl-, Di-(C₁₋₃-alkyl)-amino-C₁₋₃-alkylaminocarbonyl-, Di-(C₁₋₃-alkyl)-amino-carbonyl-C₁₋₃-alkoxy-, C₁₋₃-Alkyl-amino-carbonyl-C₁₋₃-alkoxy-, Carboxy-C₁₋₃-alkoxy-, C₁₋₃-Alkyloxy-carbonyl-C₁₋₃-alkoxy-, Carboxy-C₁₋₃-alkyl-, C₁₋₃-Alkoxy-carbonyl-C₁₋₃-alkyl-, C₁₋₃-Alkoxy-carbonylamino-C₁₋₃-alkyl-, Amino-C₁₋₃-alkyl-, Di-(C₁₋₃-alkyl)-amino-C₁₋₃-alkyl-, C₁₋₃-Alkyl-carbonylamino-C₁₋₃-alkyl-, Phthalimido-, Pyrrolyl- oder Mono- oder Di-(C₁₋₃-alkyl)-pyrrolylgruppen substituiert sein kann, wobei die Substituenten gleich oder verschieden sind, und
R³ ein Wasserstoffatom,
eine lineare oder verzweigte C₁₋₆-Alkylgruppe, die durch eine bis drei Carboxy-, C₁₋₄-Alkoxy-carbonyl-, Aminocarbonyl-, C₁₋₃-Alkylaminocarbonyl-, Di-(C₁₋₃-alkyl)-aminocarbonyl-, Di-(C₁₋₃-alkyl)-amino-(C₁₋₃-alkyl)-amino-carbonyl-, N-[Di-(C₁₋₃-alkyl)-amino-(C₁₋₃-alkyl)]-N-(C₁₋₃-alkyl)-amino-carbonyl-, Arylaminocarbonyl-, Heteroarylaminocarbonyl-, Aryl-C₁₋₃-alkyl-aminocarbonyl-, Heteroaryl-C₁₋₃-alkyl-aminocarbonyl-, N-(Aryl)-N-(C₁₋₃-alkyl)-aminocarbonyl-, N-(Heteroaryl)-N-(C₁₋₃-alkyl)-aminocarbonyl-, Heteroarylaminocarbonyl-, Di-(ω-hydroxy-C₂₋₃-alkyl)-aminocarbonyl-, Aryl- oder Heteroarylgruppen oder durch eine 5 bis 7-gliedrige Cycloalkylenimino- oder Cycloalkyleniminocarbonylgruppe substituiert sein kann,
wobei in der oben erwähnten Cycloalkylenimino- oder Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
eine lineare oder verzweigte C₂₋₆-Alkylgruppe, die ab Position 2 durch eine bis drei Hydroxy-, C₁₋₃-Alkoxy-, Aryloxy-, Heteroaryloxy-, Amino-C₁₋₃-alkyloxy-, C₁₋₃-Alkylamino-C₁₋₃-alkyloxy-, Di-(C₁₋₃-alkyl)-amino-C₁₋₃-alkyloxy-, ω-Hydroxy-C₂₋₃-alkoxy-, Amino-, C₁₋₃-Alkylamino-, Di-(C₁₋₃-alkyl)-amino-, C₁₋₃-Alkyl-carbonylamino-, Heteroaryl-carbonylamino-, N-(C₁₋₃-Alkyl)-N-(C₁₋₃-alkyl-carbonyl)-amino-, N-(C₁₋₃-Alkyl)-N-(heteroaryl-carbonyl)-amino-, C₁₋₄-Alkoxy-carbonylamino-, N-(C₁₋₃-Alkyl)-N-(C₁₋₄-alkoxy-carbonyl)-amino-, Arylcarbonylamino-, N-(C₁₋₃-Alkyl)-N-(arylcarbonyl)-amino-, (ω-Hydroxy-C₂₋₃-alkyl)-amino-, Di-(ω-hydroxy-C₂₋₃-alkyl)-amino-, Arylamino-, Heteroarylamino-, C₁₋₃-Alkylsulfonylamino-, N-(C₁₋₃-Alkyl)-N-(C₁₋₃-alkylsulfonyl)-amino-, C₁₋₃-Alkoxy-carbonyl-C₁₋₃-alkyl-carbonylamino-, (C₁₋₃-Alkyl-amino)-carbonyl-amino-, [Di-(C₁₋₃-alkyl)-amino]-carbonyl-amino-, (C₁₋₃-Alkyl-amino)-C₁₋₃-alkyl-carbonyl-amino-, [Di-(C₁₋₃-alkyl)-amino]-C₁₋₃-alkyl-carbonyl-amino-, N-(Aryl-C₁₋₃-alkyl-carbonyl)-N-(C₁₋₃-alkyl)-amino-, N-(C₁₋₃-Alkyl)-N-[(C₁₋₃-alkyl-amino)-carbonyl]-amino- oder N-(C₁₋₃-Alkyl)-N-{[di-(C₁₋₃-alkyl)-amino]-carbonyl}-aminogruppe substituiert ist und gegebenenfalls zusätzlich ab Position 1 durch eine Carboxy-, C₁₋₄-Alkoxy-carbonyl-, C₁₋₃-Alkylaminocarbonyl-, Di-(C₁₋₃-alkyl)-aminocarbonyl-, Arylaminocarbonyl-, Heteroarylaminocarbonyl-, Di-(ω-hydroxy-C₂₋₃-alkyl)-aminocarbonyl-, Aryl- oder Heteroarylgruppen oder durch eine 5 bis 7-gliedrige Cycloalkylenimino- oder Cycloalkyleniminocarbonylgruppe substituiert sein kann,
wobei in der oben erwähnten Cycloalkylenimino- oder Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
eine C₂₋₄-Alkenyl- oder C₂₋₄-Alkinylgruppe, wobei sich zwischen dem Stickstoffatom, an das R³ gebunden ist, und der Mehrfachbindung mindestens ein sp³-hybridisiertes Kohlenstoffatom befindet und die Alkenyl- oder Alkinylgruppe durch eine bis drei C₁₋₃-Alkylgruppen substituiert sein kann, oder
eine C₁₋₅-Alkylgruppe, in der Methylengruppe, die dem Stickstoffatom, an das R³ gebunden ist, benachbart ist, durch eine -NH-, oder eine -N(C₁₋₃-Alkyl)- Gruppe oder durch ein Sauerstoffatom ersetzt sein können,
eine C₁₋₄-Alkyloxygruppe oder
eine Aminogruppe, die durch eine oder zwei C₁₋₃-Alkyl-, Aryl-, Aryl-C₁₋₃-alkyl-, C₁₋₃-Alkyl-carbonyl-, C₁₋₄-Alkyloxy-carbonyl-,Arylcarbonyl- oder Aryl-C₁₋₃-alkyl-carbonylgruppen substituiert sein kann, wobei die Substituenten gleich oder verschieden sein können, bedeuten,
wobei die vorstehend erwähnten Alkylgruppen geradkettig oder verzweigt sein können,
deren Tautomere, Enantiomere, Diastereomere, deren Gemische und deren Salze.

2. Verbindungen der allgemeinen Formel I gemäß Anspruch 1, in denen
R² und R³ wie in Anspruch 1 erwähnt definiert sind und
R¹ eine Methyl-, Ethyl-, n-Propyl-, Isopropyl-, n-Pentyl-, Trifluormethyl- oder Phenylgruppe bedeutet,
deren Tautomere, Enantiomere, Diastereomere, deren Gemische und deren Salze.

3. Verbindungen der allgemeinen Formel I gemäß Anspruch 2, in denen
R¹ eine Methyl- oder Ethylgruppe,
R² eine Ethyl-, Propyl-, Butyl- oder Pentylgruppe,
eine Pyridinyl-, Furanyl- oder Pyrazinylgruppe,
eine Phenylgruppe, in der zwei benachbarte Kohlenstoffatome über eine Methylendioxy-, Ethylendioxy- oder Difluormethylendioxygruppe miteinander verknüpft sind, oder
eine Phenylgruppe, die durch ein oder zwei Fluor-, Chlor-, Brom- oder lodatome oder durch eine oder zwei C₁₋₃-Alkyl-, Nitro-, Cyano-, Amino-, C₁₋₃-Alkylcarbonylamino-, Phenylcarbonylamino-, C₁₋₃-Alkylsulfonylamino-, Trifluormethyl-, Carboxy-, C₁₋₃-Alkoxy-, Di-(C₁₋₃-alkyl)-amino-C₁₋₃-alkyloxy-, C₁₋₃-Alkoxy-carbonyl-, C₁₋₃-Alkylamino-carbonyl-, Hydroxycarbonyl-C₁₋₃-alkyl-aminocarbonyl-, C₁₋₃-Alkoxycarbonyl-C₁₋₃-alkylaminocarbonyl-, Di-(C₁₋₃-alkyl)-amino-C₁₋₃-alkylaminocarbonyl-, Carboxy-C₁₋₃-alkyl-, C₁₋₃-Alkoxy-carbonyl-C₁₋₃-alkyl-, Amino-C₁₋₃-alkyl- oder C₁₋₃-Alkyl-carbonylamino-C₁₋₃-alkylgruppen substituiert sein kann, wobei die Substituenten gleich oder verschieden sind, und
R³ ein Wasserstoffatom,
eine lineare oder verzweigte C₁₋₆-Alkylgruppe, die durch eine Carboxy-, C₁₋₄-Alkoxycarbonyl-, Phenyl-, Pyridinyl-, Indolyl-, Imidazolyl-, C₁₋₃-Alkylaminocarbonyl-, Di-(C₁₋₃-alkyl)-aminocarbonyl-, Phenylaminocarbonyl-, Pyridinylaminocarbonyl-, Di-(ω-hydroxy-C₂₋₃-alkyl)-aminocarbonyl- oder durch eine 5 bis 7-gliedrige Cycloalkylenimino- oder Cycloalkyleniminocarbonylgruppe substituiert sein kann,
wobei die oben erwähnte Phenylgruppe gegebenenfalls durch eine Nitro-, Cyano-, C₁₋₃-Alkyloxy-, [Di-(C₁₋₃-alkyl)-amino]-C₁₋₃-alkyloxy-, Amino-C₁₋₃-alkyl- oder C₁₋₄-Alkoxy-carbonylamino-C₁₋₃-alkylgruppe substituiert sein kann und
wobei in der oben erwähnten Cycloalkylenimino- und Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
eine lineare oder verzweigte C₂₋₆-Alkylgruppe, die ab Position 2 durch eine Hydroxy-, C₁₋₃-Alkoxy-, ω-Hydroxy-C₂₋₃-alkoxy-, Amino-, C₁₋₃-Alkylamino-, Di-(C₁₋₃-alkyl)-amino-, C₁₋₃-Alkyl-carbonylamino-, N-(C₁₋₃-Alkyl)-N-(C₁₋₃-alkyl-carbonyl)-amino-, C₁₋₄-Alkoxy-carbonylamino-, N-(C₁₋₃-Alkyl)-N-(C₁₋₄-alkoxy-carbonyl)-amino-, Phenylcarbonylamino-, N-(C₁₋₃-Alkyl)-N-(phenylcarbonyl)-amino-, Di-(ω-hydroxy-C₂₋₃-alkyl)-amino-, Pyridinylamino-, Nitro-pyridinyl-amino-, Chlor-trifluormethyl-pyridinylamino-, C₁₋₃-Alkylsulfonylamino-, N-(C₁₋₃-Alkyl)-N-(C₁₋₃-alkylsulfonyl)-amino- oder C₁₋₃-Alkoxycarbonyl-C₁₋₃-alkyl-carbonylaminogruppe substiuiert ist und gegebenenfalls zusätzlich ab Position 1 durch eine Carboxy-, C₁₋₄-Alkoxy-carbonyl-, Phenyl-, Pyridinyl-, Imidazolyl-, C₁₋₃-Alkylaminocarbonyl-, Di-(C₁₋₃-alkyl)-aminocarbonyl-, Phenylamino-carbonyl-, Di-(ω-hydroxy-C₂₋₃-alkyl)-aminocarbonyl- oder durch eine 5 bis 7-gliedrige Cycloalkylenimino- oder Cycloalkyleniminocarbonylgruppe substituiert sein kann,
wobei in der oben erwähnten Cycloalkylenimino- oder Cycloalkyleniminocarbonylgruppe eine Methylengruppe in Position 3 oder 4 durch eine -NH-, -N(C₁₋₃-Alkyl)- oder -N(C₁₋₄-Alkoxy-carbonyl)- Gruppe oder durch ein Sauerstoff- oder Schwefelatom und/oder in Position 2, 3 oder 4 durch eine Carbonylgruppe ersetzt sein kann,
und wobei die oben erwähnten Phenylgruppen gegebenenfalls durch eine Amino-C₁₋₃-alkyl- oder C₁₋₄-Alkoxy-carbonylamino-C₁₋₃-alkylgruppe substituiert sein können, oder
oder eine Propenyl- oder Propinylgruppe bedeuten,
deren Tautomere, Enantiomere, Diastereomere, deren Gemische und deren Salze.

4. Verbindungen der allgemeinen Formel I gemäß Anspruch 3, in denen
R¹ eine Methylgruppe,
R² eine Ethyl-, Propyl-, Butyl- oder Pentylgruppe,
eine Phenylgruppe, in der zwei benachbarte Kohlenstoffatome über eine Methylendioxy-, Ethylendioxy- oder Difluormethylendioxygruppe miteinander verknüpft sind, oder
eine Phenylgruppe, die durch ein oder zwei Fluor-, Chlor- oder Bromatome oder durch eine oder zwei Trifluormethyl-, Nitro-, Cyano-, C₁₋₃-Alkoxy-, Carboxy-C₁₋₃-alkyl-, C₁₋₃-Alkoxy-carbonyl-C₁₋₃-alkyl-, Amino-C₁₋₃-alkyl- oder C₁₋₃-Alkyl-carbonylamino-C₁₋₃-alkyl-gruppen substituiert sein kann, wobei die Substituenten gleich oder verschieden sind, und
R³ eine lineare oder verzweigte C₁₋₅-Alkylgruppe, die durch eine Carboxy-, C₁₋₄-Alkoxy-carbonyl-, C₁₋₃-Alkylaminocarbonyl-, Di-(C₁₋₃-alkyl)-aminocarbonyl-, Phenylaminocarbonyl-, Pyrdinylaminocarbonyl-, Di-(2-hydroxy-ethyl)-aminocarbonyl-, Piperazinylcarbonyl-, 4-(C₁₋₃-Alkyl)-piperazinyl-carbonyl-, 4-(C₁₋₄-Alkoxy-carbonyl)-piperazinyl-carbonyl-, Phenyl-, Pyridinyl- oder Imidazolylgruppe substituiert sein kann,
wobei die Phenylgruppe gegebenenfalls durch eine Nitro-, Cyano-, C₁₋₃-Alkyloxy-, [Di-(C₁₋₃-alkyl)-aminol-C₁₋₃-alkyloxy-, Amino-C₁₋₃-alkyl- oder C₁₋₄-Alkoxy-carbonylamino-C₁₋₃-alkylgruppe substituiert sein kann,
eine C₂₋₅-Alkylgruppe, die endständig durch eine Hydroxy-, C₁₋₃-Alkoxy-, Phenyloxy-, 2-Hydroxy-ethoxy-, Amino-, C₁₋₃-Alkylamino-, Di-(C₁₋₃-alkyl)-amino-, C₁₋₃-Alkyl-carbonylamino-, N-(C₁₋₃-Alkyl)-N-(C₁₋₃-alkyl-carbonyl)-amino-, C₁₋₄-Alkoxy-carbonylamino-, N-(C₁₋₃-Alkyl)-N-(C₁₋₄-alkoxy-carbonyl)-amino-, Phenylcarbonylamino-, N-(C₁₋₃-Alkyl)-N-(phenylcarbonyl)-amino-, Pyridinylamino-, Nitro-pyridinyl-amino-, Di-(2-hydroxy-ethyl)-amino-, 3-Chlor-5-trifluormethyl-pyridin-2-yl-amino-, C₁₋₃-Alkylsulfonylamino-, N-(C₁₋₃-Alkyl)-N-(C₁₋₃-alkylsulfonyl)-amino-, C₁₋₃-Alkoxy-carbonyl-C₁₋₃-alkyl-carbonylamino-, Indolyl-, Pyrrolidinyl-, 2-Oxo-pyrrolidinyl-, Morpholinyl-, Piperazinyl- oder 4-(C₁₋₃-Alkyl)-piperazinylgruppe substituiert ist und gegebenenfalls zusätzlich ab Position 1 durch eine Carboxy-, C₁₋₄-Alkoxy-carbonyl-, C₁₋₃-Alkylaminocarbonyl-, Di-(C₁₋₃-alkyl)-aminocarbonyl-, Phenylaminocarbonyl-, Di-(2-hydroxy-ethyl)-aminocarbonyl-, Piperazinylcarbonyl-, 4-(C₁₋₃-Alkyl)-piperazinyl-carbonyl-, 4-(C₁₋₄-Alkoxy-carbonyl)-piperazinyl-carbonyl-, Phenyl-, Pyridinyl- oder Imidazolylgruppe substituiert sein kann, oder
eine Aminogruppe, die durch eine oder zwei C₁₋₃-Alkyl-, Aryl-, Aryl-C₁₋₃-alkyl-, C₁₋₃-Alkyl-carbonyl-, C₁₋₄-Alkyloxy-carbonyl-,Arylcarbonyl- oder Aryl-C₁₋₃-alkyl-carbonylgruppen substituiert sein kann, wobei die Substituenten gleich oder verschieden sein können, bedeuten,
wobei die vorstehend erwähnten Alkylgruppen geradkettig oder verzweigt sein können,
deren Tautomere, Enantiomere, Diastereomere, deren Gemische und deren Salze.

5. Verbindungen der allgemeinen Formel I gemäß Anspruch 4, in denen
R¹ eine Methylgruppe,
R² eine Ethyl-, Propyl-, Butyl- oder Pentylgruppe,
eine Phenylgruppe, in der zwei benachbarte Kohlenstoffatome über eine Methylendioxy- oder Ethylendioxygruppe miteinander verknüpft sind, oder
eine Phenylgruppe und
R³ eine C₁₋₄-Alkylgruppe, die endständig durch eine C₁₋₄-Alkoxy-carbonylgruppe substituiert sein kann, oder
eine C₂₋₄-Alkylgruppe, die endständig durch eine C₁₋₃-Alkyl-carbonylamino-, Phenylcarbonylamino-, Di-(C₁₋₃-alkyl)-amino-, Phenyl-, Pyridinyl- oder C₁₋₃-Alkylsulfonylaminogruppe substituiert ist, bedeuten,
wobei die vorstehend erwähnten Alkylgruppen geradkettig oder verzweigt sein können, bedeuten,
deren Tautomere, Enantiomere, Diastereomere, deren Gemische und deren Salze.

6. Folgende Verbindungen der allgemeinen Formel I gemäß Anspruch 1:
(a) 5-Acetyl-3-[3-(methoxycarbonylpropylamino)-(benzo-[1,3]dioxyo-5-yl)-methyliden]-2-indolinon 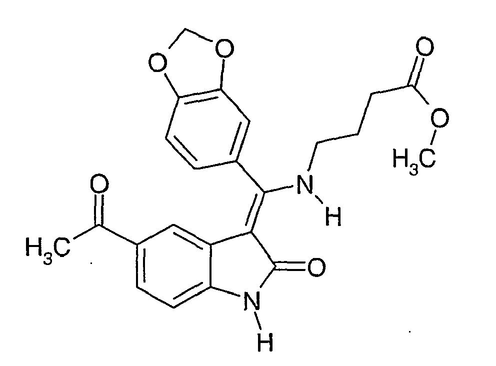
(b) 5-Acetyl-3-[isopropylamino-phenyl-methyliden]-2-indolinon 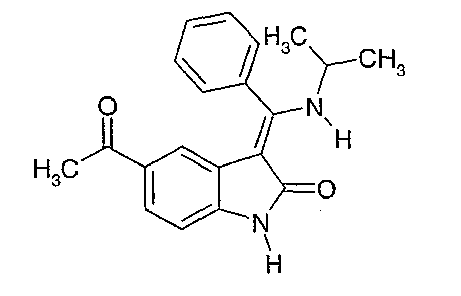
(c) 5-Acetyl-3-[propylamino-phenyl-methyliden]-2-indolinon 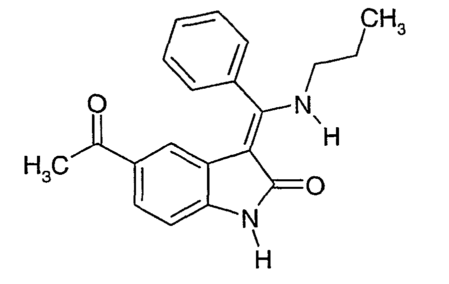
(d) 5-Acetyl-3-[(3-methoxycarbonyl-propylamino)-(2,3-dihydro-benzo[1,4]dioxin-6-yl)-methyliden]-2-indolinon 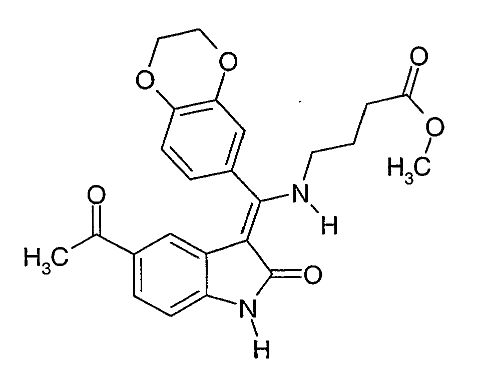
(e) 5-Acetyl-3-[(benzo[1,3]dioxol-5-yl)-(3-(benzoylamino-propylamino)-methyliden]-2-indolinon 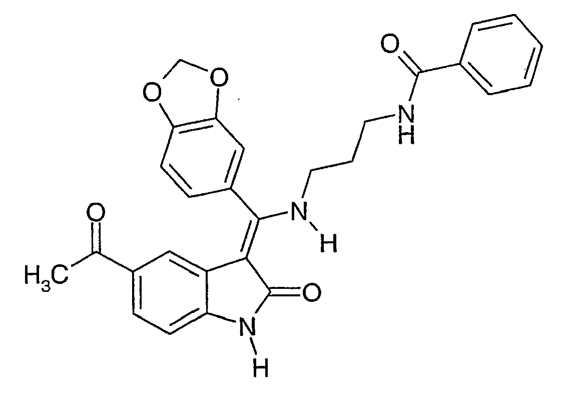
(f) 5-Acetyl-3-[(benzo[1,3]dioxol-5-yl)-(3-(butyrylamino-propylamino)-methyliden]-2-indolinon 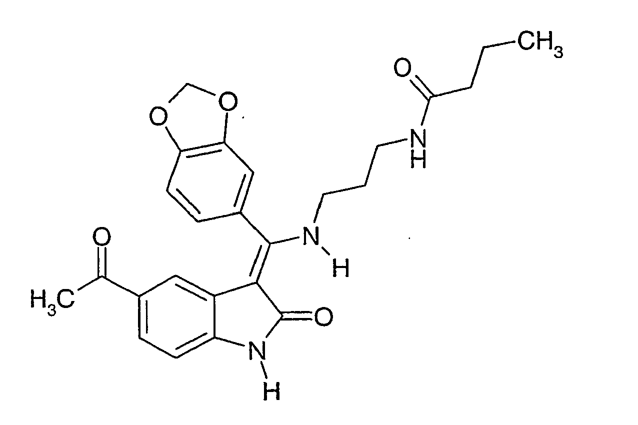
(g) 5-Acetyl-3-[(3-methansulfonylamino-propylamino)-phenyl-methyliden]-2-indolinon 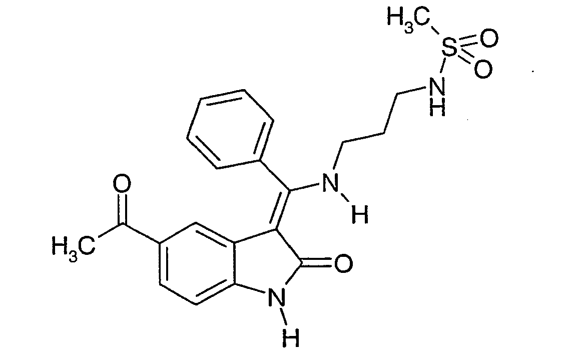
(h) 5-Acetyl-3-[1-(3,4-difluorphenyl)-1-(N',N'-dimethylhydrazino)-methyliden]-2-indolinon 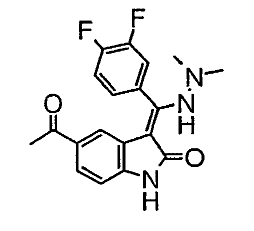
(i) 5-Acetyl-3-[1-(4-dimethylamino-butyl)-butenyliden]-2-indolinon 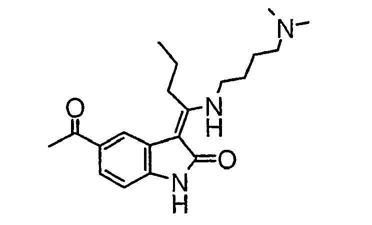
(j) 5-Acetyl-3-[1-phenyl-1-(3-pyridin-3-yl-propylamino)-methyliden]-2-indolinon 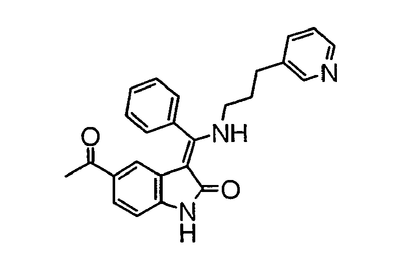
sowie deren Tautomere, Enantiomere, Diastereomere, deren Gemische und deren Salze.

7. Physiologisch verträgliche Salze der Verbindungen nach mindestens einem der Ansprüche 1 bis 6 mit anorganischen oder organischen Säuren oder Basen.

8. Arzneimittel, enthaltend eine Verbindung nach mindestens einem der Ansprüche 1 bis 6 oder ein physiologisch verträgliches Salz gemäß Anspruch 7 neben gegebenenfalls einem oder mehreren inerten Trägerstoffen und/oder Verdünnungsmitteln.

9. Verwendung einer Verbindung nach mindestens einem der Ansprüche 1 bis 7 zur Herstellung eines Arzneimittels, das zur Behandlung von Diabetes mellitus Typ I und Typ II, Diabetes assoziierte Störungen wie diabetische Neuropathie, und degenerativer neurologischer Erkrankungen wie Alzheimers Erkrankung, Schlaganfall, neurotraumatische Verletzungen und bipolare Störungen geeignet ist.

10. Verfahren zur Herstellung eines Arzneimittels gemäß Anspruch 8, **dadurch gekennzeichnet, daß** auf nichtchemischen Weg eine Verbindung nach mindestens einem der Ansprüche 1 bis 7 in einen oder mehrere inerte Trägerstoffe und/oder Verdünnungsmittel eingearbeitet wird.

11. Verfahren zur Herstellung der Verbindungen der allgemeinen Formel I gemäß den Ansprüchen 1 bis 7, **dadurch gekennzeichnet, daß**
a) eine Verbindung der allgemeinen Formel 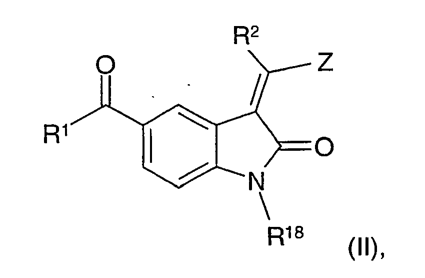 in der R¹ und R² wie in den Ansprüchen 1 bis 6 erwähnt definiert sind,
R¹⁸ ein Wasserstoffatom oder eine Schutzgruppe für das Stickstoffatom der Lactamgruppe und
Z eine Austrittsgruppe wie etwa ein Halogenatom, eine Hydroxy-, Alkoxy-,Alkylsulfonyl-, Aralkylsulfonyl-, oder Aryl-alkoxygruppe, z.B. ein Chlor- oder Bromatom, eine Methoxy-, Ethoxy-, Methansulfonyl-, Toluolsulfonyl- oder Benzyloxygruppe, bedeuten,
mit einem Amin der allgemeinen Formel
R³-NH₂ (III),
in der R³ wie in einem der Ansprüche 1 bis 6 erwähnt definiert ist, wobei eventuell in den Resten R² und/oder R³ enthaltene Hydroxy-, Amino- oder Iminogruppen vorübergehend durch geeignete Schutzgruppen geschützt werden können, umgesetzt wird,
b) zur Herstellung einer Verbindung der Formel I, die eine Aminocarbonylgruppe enthält, eine Verbindung, die eine Carboxygruppe enthält, mit dem entsprechenden Amin umgesetzt wird,
c) zur Herstellung einer Verbindung der Formel I, die eine Carbonylaminogruppe enthält, eine Verbindung, die eine Aminogruppe enthält, mit dem entsprechenden Säurechlorid umgesetzt wird,
d) zur Herstellung einer Verbindung der Formel I, die eine Aminomethylgruppe enthält, eine Verbindung, die eine Cyanogruppe enthält, zu dem entsprechenden Aminomethylderivat hydriert wird,
e) zur Herstellung einer Verbindung der Formel I, die eine Aminogruppe enthält, eine Verbindung, die eine Nitrogruppe enthält, hydriert wird,
und/oder
anschließend gegegebenenfalls während der Umsetzung verwendete Schutzgruppen abgespalten werden und/oder
die so erhaltenen Verbindungen der allgemeinen Formel I in ihre Enantiomeren und/oder Diastereomeren aufgetrennt werden und/oder
die erhaltenen Verbindungen der Formel I in ihre Salze, insbesondere für die pharmazeutische Anwendung in ihre physiologisch verträglichen Salze mit anorganischen oder organischen Säuren oder Basen, übergeführt werden.

## Claims

1. Compounds of general formula 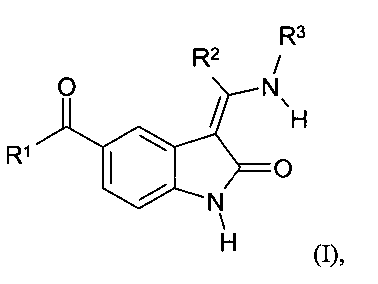 wherein
R¹ denotes a straight-chain or branched C₁₋₅-alkyl group wherein the hydrogen atoms may be wholly or partly replaced by fluorine atoms, or
an aryl group optionally substituted by a fluorine, chlorine or bromine atom,
while by an aryl group is meant a phenyl or naphthyl group,
R² denotes a C₁₋₇-alkyl or C₃₋₇-cycloalkyl group,
a 5- or 6-membered heteroaryl group with one to three heteroatoms selected from the group N, S and O, while both the heteroatoms and the substituents may be identical or different, optionally substituted by one or two fluorine, chlorine, bromine or iodine atoms or one or two nitro, cyano, amino, C₁₋₃-alkyl or C₁₋₃-alkoxy groups,
a phenyl group wherein two adjacent carbon atoms are linked together through a methylenedioxy, ethylenedioxy or difluoromethylenedioxy group,
a phenyl group, to which is anellated another phenyl ring or a 5- or 6-membered heteroaromatic ring with one to three heteroatoms selected from the group N, S and O, while the heteratoms may be identical or different, and the bicyclic group may be substituted by one or two fluorine, chlorine, bromine or iodine atoms or one or two nitro, cyano, amino, C₁₋₃-alkyl or C₁₋₃-alkoxy groups and the substituents may be identical or different, or
a phenyl group which may be substituted by one to three fluorine, chlorine, bromine or iodine atoms or by one to three C₁₋₃-alkyl, nitro, cyano, amino, di-(C₁₋₃-alkyl)-amino, C₁₋₃-alkyl-carbonylamino, phenylcarbonylamino, C₁₋₃-alkylsulphonylamino, arylsulphonylamino, trifluoromethyl, C₁₋₃ alkylsulphonyl, carboxy, C₁₋₃-alkoxy, di-(C₁₋₃-alkyl)-amino-C₁₋₃-alkyloxy, C₁₋₃-alkoxy-carbonyl, C₁₋₃-alkylaminocarbonyl, hydroxycarbonyl-C₁₋₃-alkyl-aminocarbonyl, C₁₋₃-alkoxycarbonyl-C₁₋₃-alkylaminocarbonyl, di-(C₁₋₃-alkyl)-amino-C₁₋₃-alkylaminocarbonyl, di-(C₁₋₃-alkyl)-amino-carbonyl-C₁₋₃-alkoxy, C₁₋₃-alkyl-amino-carbonyl-C₁₋₃-alkoxy, carboxy-C₁₋₃-alkoxy, C₁₋₃-alkyloxy-carbonyl-C₁₋₃-alkoxy, carboxy-C₁₋₃-alkyl, C₁₋₃-alkoxycarbonyl-C₁₋₃-alkyl, C₁₋₃-alkoxy-carbonylamino-C₁₋₃-alkyl, amino-C₁₋₃-alkyl, di-(C₁₋₃-alkyl)-amino-C₁₋₃-alkyl, C₁₋₃-alkyl-carbonylamino-C₁₋₃-alkyl, phthalimido, pyrrolyl or mono- or di-(C₁₋₃-alkyl)-pyrrolyl groups, while the substituents are identical or different, and
R³ denotes a hydrogen atom,
a straight-chain or branched C₁₋₆-alkyl group which may be substituted by one to three carboxy, C₁₋₄-alkoxy-carbonyl, aminocarbonyl, C₁₋₃-alkylaminocarbonyl, di-(C₁₋₃-alkyl)-aminocarbonyl, di-(C₁₋₃-alkyl)-amino-(C₁₋₃-alkyl)-amino-carbonyl, N-[di-(C₁₋₃-alkyl)-amino-(C₁₋₃-alkyl)]-N-(C₁₋₃-alkyl)-amino-carbonyl, arylaminocarbonyl, heteroarylaminocarbonyl, aryl-C₁₋₃-alkyl-aminocarbonyl, heteroaryl-C₁₋₃-alkyl-aminocarbonyl, N-(aryl)-N-(C₁₋₃-alkyl)-aminocarbonyl, N-(heteroaryl)-N-(C₁₋₃-alkyl)-aminocarbonyl, di-(ω-hydroxy-C₂₋₃-alkyl)-aminocarbonyl, aryl or heteroaryl groups or by a 5 to 7-membered cycloalkyleneimino or cycloalkyleneiminocarbonyl group,
while in the above-mentioned cycloalkyleneimino or cycloalkyleneiminocarbonyl group a methylene group may be replaced in position 3 or 4 by an -NH-, -N(C₁₋₃-alkyl)- or -N(C₁₋₄-alkoxy-carbonyl)- group or by an oxygen or sulphur atom and/or in position 2, 3 or 4 by a carbonyl group,
a straight-chain or branched C₂₋₆-alkyl group which is substituted from position 2 onwards by one to three hydroxy, C₁₋₃-alkoxy, aryloxy, heteroaryloxy, amino-C₁₋₃-alkyloxy, C₁₋₃-alkyl-amino-C₁₋₃-alkyloxy, di-(C₁₋₃-alkyl)-amino-C₁₋₃-alkyloxy, ω-hydroxy-C₂₋₃-alkoxy, amino, C₁₋₃-alkylamino, di-(C₁₋₃-alkyl)-amino, C₁₋₃-alkylcarbonylamino, heteroaryl-carbonylamino, N-(C₁₋₃-alkyl)-N-(C₁₋₃-alkyl-carbonyl)-amino, N-(C₁₋₃-alkyl)-N-(heteroaryl-carbonyl)-amino, C₁₋₄-alkoxy-carbonylamino, N-(C₁₋₃-alkyl)-N-(C₁₋₄-alkoxy-carbonyl)-amino, arylcarbonylamino, N-(C₁₋₃-alkyl)-N-(arylcarbonyl)-amino, (ω-hydroxy-C₂₋₃-alkyl)-amino, di-(ω-hydroxy-C₂₋₃-alkyl)-amino, arylamino, heteroarylamino, C₁₋₃-alkylsulphonylamino, N-(C₁₋₃-alkyl)-N-(C₁₋₃-alkylsulphonyl)-amino, C₁₋₃-alkoxy-carbonyl-C₁₋₃-alkyl-carbonylamino, (C₁₋₃-alkylamino)-carbonyl-amino, [di-(C₁₋₃-alkyl)-amino]-carbonyl-amino, (C₁₋₃-alkyl-amino)-C₁₋₃-alkyl-carbonyl-amino, [di-(C₁₋₃-alkyl)-amino]-C₁₋₃-alkyl-carbonyl-amino, N-(aryl-C₁₋₃-alkyl-carbonyl)-N-(C₁₋₃-alkyl)-amino, N-(C₁₋₃-alkyl)-N-[(C₁₋₃-alkylamino)-carbonyl]-amino or N-(C₁₋₃-alkyl)-N-{[di-(C₁₋₃-alkyl)-amino]-carbonyl}-amino group and may optionally additionally be substituted from position 1 onwards by a carboxy, C₁₋₄-alkoxy-carbonyl, C₁₋₃-alkylaminocarbonyl, di-(C₁₋₃-alkyl)-aminocarbonyl, arylaminocarbonyl, heteroarylaminocarbonyl-, di-(ω-hydroxy-C₂₋₃-alkyl)-aminocarbonyl-, aryl or heteroaryl group or by a 5 to 7-membered cycloalkyleneimino or cycloalkyleneiminocarbonyl group,
while in the above-mentioned cycloalkyleneimino or cycloalkyleneimino-carbonyl group a methylene group in position 3 or 4 may be replaced by an -NH-, -N(C₁₋₃-alkyl)- or -N(C₁₋₄-alkoxy-carbonyl)- group or by an oxygen or sulphur atom and/or in position 2, 3 or 4 by a carbonyl group,
a C₂₋₄-alkenyl or C₂₋₄-alkynyl group, while between the nitrogen atom to which R³ is bound and the multiple bond there is at least one sp³-hybridised carbon atom and the alkenyl or alkynyl group may be substituted by one to three C₁₋₃-alkyl groups,
a C₁₋₅-alkyl group in the methylene group which is adjacent to the nitrogen atom to which R³ is bound may be replaced by an -NH-, or a -N(C₁₋₃-alkyl)- group or by an oxygen atom,
a C₁₋₄-alkyloxy group or
an amino group which may be substituted by one or two C₁₋₃-alkyl, aryl, aryl-C₁₋₃-alkyl, C₁₋₃-alkyl-carbonyl, C₁₋₄-alkyloxy-carbonyl, arylcarbonyl or aryl-C₁₋₃-alkylcarbonyl groups, while the substituents may be identical or different,
while the above-mentioned alkyl groups may be straight-chain or branched,
the tautomers, enantiomers, diastereomers, the mixtures thereof and the salts thereof.

2. Compounds of general formula I according to claim 1, wherein
R² and R³ are defined as in claim 1 and
R¹ denotes a methyl, ethyl, n-propyl, isopropyl, n-pentyl, trifluoromethyl or phenyl group,
the tautomers, enantiomers, diastereomers, the mixtures thereof and the salts thereof.

3. Compounds of general formula I according to claim 2, wherein
R¹ denotes a methyl or ethyl group,
R² denotes an ethyl, propyl, butyl or pentyl group,
a pyridinyl, furanyl or pyrazinyl group,
a phenyl group wherein two adjacent carbon atoms are linked together through a methylenedioxy, ethylenedioxy or difluoromethylenedioxy group, or
a phenyl group which may be substituted by one or two fluorine, chlorine, bromine or iodine atoms or by one or two C₁₋₃-alkyl, nitro, cyano, amino, C₁₋₃-alkylcarbonylamino, phenylcarbonylamino, C₁₋₃-alkylsulphonylamino, trifluoromethyl, carboxy, C₁₋₃-alkoxy, di-(C₁₋₃-alkyl)-amino-C₁₋₃-alkyloxy, C₁₋₃-alkoxy-carbonyl, C₁₋₃-alkylaminocarbonyl, hydroxycarbonyl-C₁₋₃-alkylaminocarbonyl, C₁₋₃-alkoxycarbonyl-C₁₋₃-alkyl-aminocarbonyl, di-(C₁₋₃-alkyl)-amino-C₁₋₃-alkylaminocarbonyl, carboxy-C₁₋₃-alkyl, C₁₋₃-alkoxy-carbonyl-C₁₋₃-alkyl, amino-C₁₋₃-alkyl or C₁₋₃-alkyl-carbonylamino-C₁₋₃-alkyl groups, while the substituents are identical or different, and
R³ denotes a hydrogen atom,
a straight-chain or branched C₁₋₆-alkyl group which may be substituted by a carboxy, C₁₋₄-alkoxy-carbonyl, phenyl, pyridinyl, indolyl, imidazolyl, C₁₋₃-alkylaminocarbonyl, di-(C₁₋₃-alkyl)-aminocarbonyl, phenylaminocarbonyl, pyridinylaminocarbonyl, di-(ω-hydroxy-C₂₋₃-alkyl)-aminocarbonyl or by a 5- to 7-membered cycloalkyleneimino or cycloalkyleneiminocarbonyl group,
while the above-mentioned phenyl group may optionally be substituted by a nitro, cyano, C₁₋₃-alkyloxy, [di-(C₁₋₃-alkyl)-amino]-C₁₋₃-alkyloxy, amino-C₁₋₃-alkyl or C₁₋₄-alkoxy-carbonylamino-C₁₋₃-alkyl group and
in the above-mentioned cycloalkyleneimino and cycloalkyleneiminocarbonyl group a methylene group in position 3 or 4 may be replaced by an -NH-, -N(C₁₋₃-alkyl)- or-N(C₁₋₄-alkoxy-carbonyl)- group or by an oxygen or sulphur atom and/or in position 2, 3 or 4 by a carbonyl group,
a straight-chain or branched C₂₋₆-alkyl group which is substituted from position 2 onwards by a hydroxy, C₁₋₃-alkoxy, ω-hydroxy-C₂₋₃-alkoxy, amino, C₁₋₃-alkylamino, di-(C₁₋₃-alkyl)-amino, C₁₋₃-alkyl-carbonylamino, N-(C₁₋₃-alkyl)-N-(C₁₋₃-alkylcarbonyl)-amino, C₁₋₄-alkoxy-carbonylamino, N-(C₁₋₃-alkyl)-N-(C₁₋₄-alkoxy-carbonyl)-amino, phenylcarbonylamino, N-(C₁₋₃-alkyl)-N-(phenylcarbonyl)-amino, di-(ω-hydroxy-C₂₋₃-alkyl)-amino, pyridinylamino, nitro-pyridinyl-amino, chloro-trifluoromethyl-pyridinylamino, C₁₋₃-alkylsulphonylamino, N-(C₁₋₃-alkyl)-N-(C₁₋₃-alkylsulphonyl)-amino or C₁₋₃-alkoxy-carbonyl-C₁₋₃-alkyl-carbonylamino group and may optionally additionally be substituted from position 1 onwards by a carboxy, C₁₋₄-alkoxy-carbonyl, phenyl, pyridinyl, imidazolyl, C₁₋₃-alkylaminocarbonyl, di-(C₁₋₃-alkyl)-aminocarbonyl, phenylaminocarbonyl, di-(ω-hydroxy-C₂₋₃-alkyl)-aminocarbonyl or by a 5 to 7-membered cycloalkyleneimino or cycloalkyleneiminocarbonyl group,
while in the above-mentioned cycloalkyleneimino or cycloalkyleneiminocarbonyl group a methylene group in position 3 or 4 may be replaced by an - NH-, -N(C₁₋₃-alkyl)- or-N(C₁₋₄-alkoxy-carbonyl)- group or by an oxygen or sulphur atom and/or in position 2, 3 or 4 may be replaced by a carbonyl group,
and the above-mentioned phenyl groups may optionally be substituted by an amino-C₁₋₃-alkyl or C₁₋₄-alkoxy-carbonylamino-C₁₋₃-alkyl group, or
or represent a propenyl or propynyl group,
the tautomers, enantiomers, diastereomers, the mixtures thereof and the salts thereof.

4. Compounds of general formula I according to claim 3, wherein
R¹ denotes a methyl group,
R² denotes an ethyl, propyl, butyl or pentyl group,
a phenyl group wherein two adjacent carbon atoms are linked together through a methylenedioxy, ethylenedioxy or difluoromethylenedioxy group, or
a phenyl group which may be substituted by one or two fluorine, chlorine or bromine atoms or by one or two trifluoromethyl, nitro, cyano, C₁₋₃-alkoxy, carboxy-C₁₋₃-alkyl, C₁₋₃-alkoxy-carbonyl-C₁₋₃-alkyl, amino-C₁₋₃-alkyl or C₁₋₃-alkyl-carbonylamino-C₁₋₃-alkyl- groups, while the substituents are identical or different, and
R³ denotes a straight-chain or branched C₁₋₅-alkyl group which may be substituted by a carboxy, C₁₋₄-alkoxy-carbonyl, C₁₋₃-alkylaminocarbonyl, di-(C₁₋₃-alkyl)-aminocarbonyl, phenylaminocarbonyl, pyridinylaminocarbonyl, di-(2-hydroxyethyl)-aminocarbonyl, piperazinylcarbonyl, 4-(C₁₋₃-alkyl)-piperazinyl-carbonyl, 4-(C₁₋₄-alkoxy-carbonyl)-piperazinyl-carbonyl, phenyl, pyridinyl or imidazolyl group,
while the phenyl group may optionally be substituted by a nitro, cyano, C₁₋₃-alkyloxy, [di-(C₁₋₃-alkyl)-amino]-C₁₋₃-alkyloxy, amino-C₁₋₃-alkyl or C₁₋₄-alkoxy-carbonylamino-C₁₋₃-alkyl group,
a C₂₋₅-alkyl group which is terminally substituted by a hydroxy, C₁₋₃-alkoxy, phenyloxy, 2-hydroxy-ethoxy, amino, C₁₋₃-alkylamino, di-(C₁₋₃-alkyl)-amino, C₁₋₃-alkyl-carbonylamino, N-(C₁₋₃-alkyl)-N-(C₁₋₃-alkyl-carbonyl)-amino, C₁₋₄-alkoxycarbonylamino, N-(C₁₋₃-alkyl)-N-(C₁₋₄-alkoxy-carbonyl)-amino, phenylcarbonylamino, N-(C₁₋₃-alkyl)-N-(phenylcarbonyl)-amino, pyridinylamino, nitro-pyridinylamino, di-(2-hydroxy-ethyl)-amino, 3-chloro-5-trifluoromethyl-pyridin-2-yl-amino, C₁₋₃-alkylsulphonylamino, N-(C₁₋₃-alkyl)-N-(C₁₋₃-alkylsulphonyl)-amino, C₁₋₃-alkoxy-carbonyl-C₁₋₃-alkyl-carbonylamino, indolyl, pyrrolidinyl, 2-oxo-pyrrolidinyl, morpholinyl, piperazinyl or 4-(C₁₋₃-alkyl)-piperazinyl group and may optionally additionally be substituted from position 1 onwards by a carboxy, C₁₋₄-alkoxycarbonyl, C₁₋₃-alkylaminocarbonyl, di-(C₁₋₃-alkyl)-aminocarbonyl, phenylaminocarbonyl, di-(2-hydroxy-ethyl)-aminocarbonyl, piperazinylcarbonyl, 4-(C₁₋₃-alkyl)-piperazinyl-carbonyl, 4-(C₁₋₄-alkoxy-carbonyl)-piperazinyl-carbonyl, phenyl, pyridinyl or imidazolyl group, or
an amino group which may be substituted by one or two C₁₋₃-alkyl, aryl, aryl-C₁₋₃-alkyl, C₁₋₃-alkyl-carbonyl, C₁₋₄-alkyloxy-carbonyl, arylcarbonyl or aryl-C₁₋₃-alkylcarbonyl groups, while the substituents may be identical or different,
while the above-mentioned alkyl groups may be straight-chain or branched,
the tautomers, enantiomers, diastereomers, the mixtures thereof and the salts thereof.

5. Compounds of general formula I according to claim 4, wherein
R¹ denotes a methyl group,
R² denotes an ethyl, propyl, butyl or pentyl group,
a phenyl group wherein two adjacent carbon atoms are linked together through a methylenedioxy or ethylenedioxy group, or
a phenyl group and
R³ denotes a C₁₋₄-alkyl group which may be terminally substituted by a C₁₋₄-alkoxycarbonyl group, or
a C₂₋₄-alkyl group which is terminally substituted by a C₁₋₃-alkyl-carbonylamino, phenylcarbonylamino, di-(C₁₋₃-alkyl)-amino, phenyl, pyridinyl or C₁₋₃-alkylsulphonylamino group,
while the above-mentioned alkyl groups may be straight-chain or branched,
the tautomers, enantiomers, diastereomers, the mixtures thereof and the salts thereof.

6. The following compounds of general formula I according to claim 1:
(a) 5-acetyl-3-[3-(methoxycarbonylpropylamino)-(benzo-[1,3]dioxyo-5-yl)-methylidene]-2-indolinone 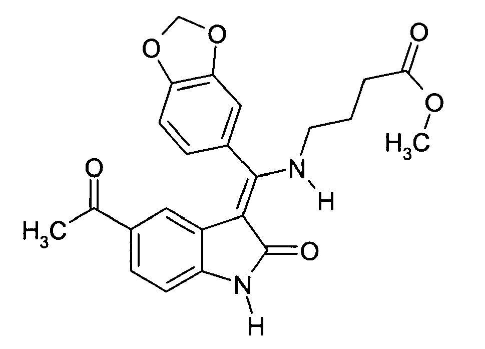
(b) 5-acetyl-3-[isopropylamino-phenyl-methylidene]-2-indolinone 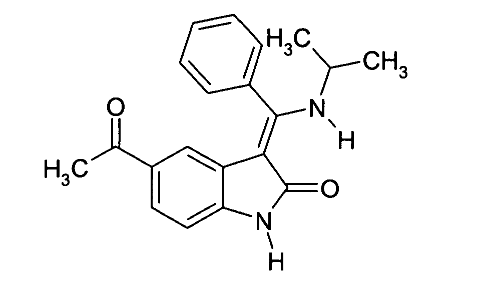
(c) 5-acetyl-3-[propylamino-phenyl-methylidene]-2-indolinone 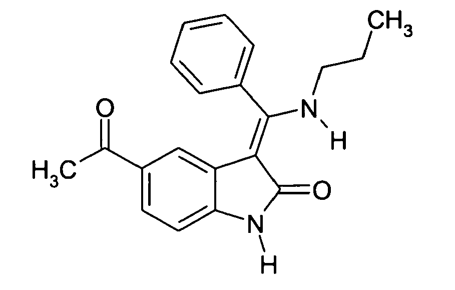
(d) 5-acetyl-3-[(3-methoxycarbonyl-propylamino)-(2,3-dihydro-benzo[1,4]dioxin-6-yl)-methylidene]-2-indolinone 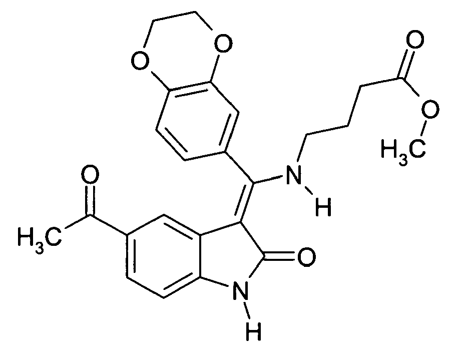
(e) 5-acetyl-3-[(benzo[1,3]dioxol-5-yl)-(3-(benzoylamino-propylamino)-methylidene]-2-indolinone 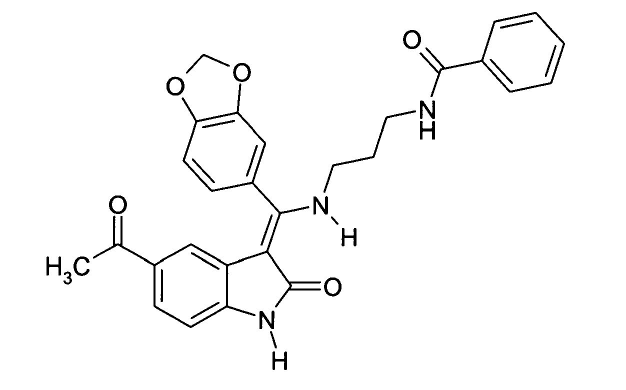
(f) 5-acetyl-3-[(benzo[1,3]dioxol-5-yl)-(3-(butyrylamino-propylamino)-methylidene]-2-indolinone 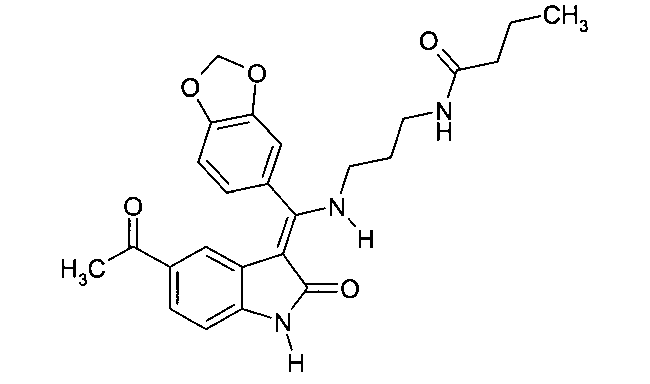
(g) 5-acetyl-3-[(3-methanesulphonylamino-propylamino)-phenyl-methylidene]-2-indolinone 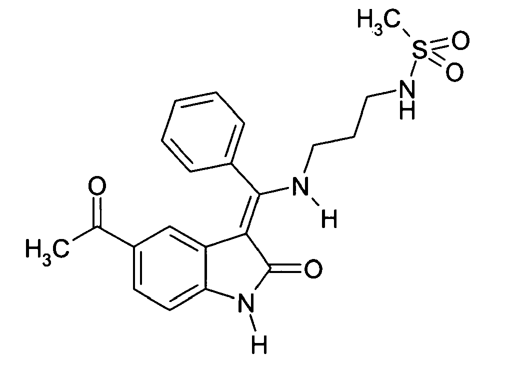
(h) 5-acetyl-3-[1-(3,4-difluorophenyl)-1-(N',N'-dimethylhydrazino)-methylidene]-2-indolinone 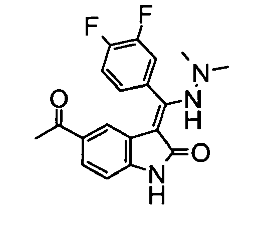
(i) 5-acetyl-3-[1-(4-dimethylamino-butyl)-butenylidene]-2-indolinone 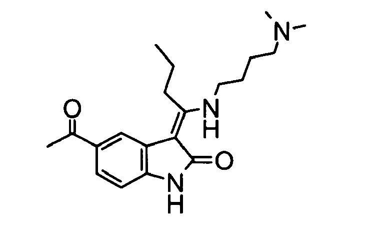
(j) 5-acetyl-3-[1-phenyl-1-(3-pyridin-3-yl-propylamino)-methylidene]-2-indolinone 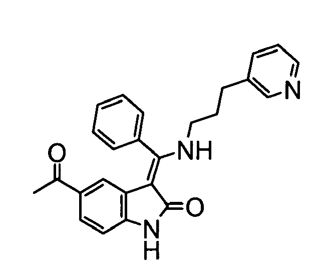
and the tautomers, enantiomers, diastereomers, the mixtures thereof and the salts thereof.

7. Physiologically acceptable salts of the compounds according to at least one of claims 1 to 6 with inorganic or organic acids or bases.

8. Pharmaceutical compositions containing a compound according to at least one of claims 1 to 6 or a physiologically acceptable salt according to claim 7 optionally together with one or more inert carriers and/or diluents.

9. Use of a compound according to at least one of claims 1 to 7 for preparing a pharmaceutical composition which is suitable for the treatment of type I and type II diabetes mellitus, diabetes associated disorders such as diabetic neuropathy and degenerative neurological diseases such as Alzheimer's disease, stroke, neurotraumatic injuries and bipolar disorders.

10. Process for preparing a pharmaceutical composition according to claim 8, **characterised in that** a compound according to at least one of claims 1 to 7 is incorporated in one or more inert carriers and/or diluents by a non-chemical method.

11. Process for preparing the compounds of general formula I according to claims 1 to 7, **characterised in that**
a) a compound of general formula 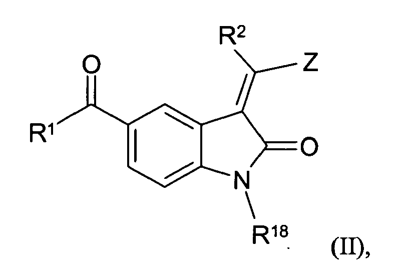 wherein R¹ and R² are defined as in claims 1 to 6,
R¹⁸ denotes a hydrogen atom or a protective group for the nitrogen atom of the lactam group and
Z denotes a leaving group such as for example a halogen atom, a hydroxy, alkoxy,alkylsulphonyl, aralkylsulphonyl, or aryl-alkoxy group, e.g. a chlorine or bromine atom, a methoxy, ethoxy, methanesulphonyl, toluenesulphonyl or benzyloxy group,
is reacted with an amine of general formula
R³-NH₂ (III),
wherein R³ is defined as in one of claims 1 to 6, while any hydroxy, amino or imino groups contained in the groups R² and/or R³ may temporarily be protected by suitable protective groups,
b) in order to prepare a compound of formula I which contains an aminocarbonyl group, a compound which contains a carboxy group is reacted with the corresponding amine,
c) in order to prepare a compound of formula I which contains a carbonylamino group, a compound which contains an amino group is reacted with the corresponding acid chloride,
d) in order to prepare a compound of formula I which contains an aminomethyl group, a compound which contains a cyano group is hydrogenated to form the corresponding aminomethyl derivative,
e) in order to prepare a compound of formula I which contains an amino group, a compound which contains a nitro group is hydrogenated,
and/or
any protective groups which may be used during the reaction are then cleaved and/or
the compounds of general formula I thus obtained are resolved into their enantiomers and/or diastereomers and/or
the compounds of general formula I thus obtained are converted into their salts, particularly for pharmaceutical use into the physiologically acceptable salts thereof with inorganic or organic acids or bases.

## Revendications

1. Composés de formule générale 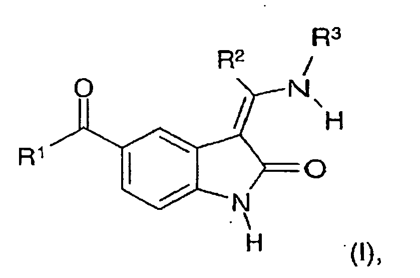 où
R¹ représente un groupe C₁₋₅-alkyle linéaire ou ramifié où les atomes d'hydrogène peuvent être remplacés totalement ou partiellement par des atomes de fluor, ou un groupe aryle éventuellement substitué par un atome de fluor, de chlore ou de brome,
où, par groupe aryle, on doit entendre un groupe phényle ou naphtyle, R² représente un groupe C₁₋₇-alkyle ou C₃₋₇-cycloalkyle, un groupe hétéroaryle à 5 ou 6 chaînons éventuellement substitué par un ou deux atomes de fluor, de chlore, de brome ou d'iode ou un ou deux groupes nitro, cyano, amino, C₁₋₃-alkyle ou C₁₋₃-alcoxy avec un à trois hétéroatomes choisis dans le groupe N, S et O, où les hétéroatomes et les substituants peuvent être identiques ou différents,
un groupe phényle où deux atomes de carbone voisins sont liés entre eux via un groupe méthylènedioxy, éthylènedioxy ou difluorométhylènedioxy,
un groupe phényle auquel est condensé un autre cycle phényle ou un cycle hétéroaromatique à 5 ou 6 chaînons avec un à trois hétéroatomes choisis dans le groupe N, S et O, où les hétéroatomes peuvent être identiques ou différents, et où le bicycle peut être substitué par un ou deux atomes de fluor, de chlore, de brome ou d'iode ou par un ou deux groupes nitro, cyano, amino, C₁₋₃-alkyle ou C₁₋₃-alcoxy et les substituants peuvent être identiques ou différents, ou un groupe phényle qui peut être substitué par un à trois atomes de fluor, de chlore, de brome ou d'iode ou par un à trois groupes C₁₋₃-alkyle, nitro, cyano, amino, di-(C₁₋₃-alkyl)-amino, C₁₋₃-alkyl-carbonylamino, phénylcarbonylamino, C₁₋₃-alkylsulfonylamino, arylsulfonylamino, trifluorométhyle, C₁₋₃-alkylsulfonyle, carboxy, C₁₋₃-alcoxy, di-(C₁₋₃-alkyl)-amino-C₁₋₃-alkyloxy, C₁₋₃-alcoxy-carbonyle, C₁₋₃-alkylaminocarbonyle, hydroxycarbonyl-C₁₋₃-alkyl-aminocarbonyle, C₁₋₃-alcoxy-carbonyl-C₁₋₃-alkyl-aminocarbonyle, di-(C₁₋₃-alkyl)-amino-C₁₋₃-alkylaminocarbonyle, di-(C₁₋₃-alkyl)-amino-carbonyl-C₁₋₃-alcoxy, C₁₋₃-alkylamino-carbonyl-C₁₋₃-alcoxy, carboxy-C₁₋₃-alcoxy, C₁₋₃-alkyloxy-carbonyl-C₁₋₃-alcoxy, carboxy-C₁₋₃-alkyle, C₁₋₃-alcoxy-carbonyl-C₁₋₃-alkyle, C₁₋₃-alcoxy-carbonylamino-C₁₋₃-alkyle, amino-C₁₋₃-alkyle, di-(C₁₋₃-alkyl)-amino-C₁₋₃-alkyle, C₁₋₃-alkyl-carbonylamino-C₁₋₃-alkyle, phtalimido, pyrrolyle ou mono- ou di-(C₁₋₃-alkyl)-pyrrolyle, où les substituants sont identiques ou différents, et
R³ représente un atome d'hydrogène,
un groupe C₁₋₃-alkyle linéaire ou ramifié qui peut être substitué par un à trois groupes carboxy, C₁₋₄-alcoxy-carbonyle, aminocarbonyle, C₁₋₃-alkylaminocarbonyle, di-(C₁₋₃-alkyl)-aminocarbonyle, di-(C₁₋₃-alkyl)-amino-(C₁₋₃-alkyl)aminocarbonyle, N-[di-(C₁₋₃-alkyl)-amino-(C₁₋₃-alkyl)]-N-(C₁₋₃-alkyl)-aminocarbonyle, arylaminocarbonyle, hétéroarylaminocarbonyle, aryl-C₁₋₃-alkylaminocarbonyle, hétéroaryl-C₁₋₃-alkyl-aminocarbonyle, N-(aryl)-N-(C₁₋₃-alkyl)-aminocarbonyle, N-(hétéroaryl)-N-(C₁₋₃-alkyl)-aminocarbonyle, hétéroarylaminocarbonyle, di-(ω-hydroxy-C₂₋₃-alkyl)-aminocarbonyle, aryle ou hétéroaryle ou par un groupe cycloalkylèneimino ou cycloalkylèneiminocarbonyle à 5 à 7 chaînons,
où dans le groupe cycloalkylèneimino ou cycloalkylèneiminocarbonyle cité ci-dessus, un groupe méthylène en position 3 ou 4 peut être remplacé par un groupe -NH-, -N(C₁₋₃-alkyl)- ou -N(C₁₋₃-alcoxy-carbonyl)- ou par un atome d'oxygène ou de soufre et/ou en position 2, 3 ou 4 par un groupe carbonyle, un groupe C₂₋₆-alkyle linéaire ou ramifié, qui peut être substitué à partir de la position 2 par un à trois groupes hydroxy, C₁₋₃-alcoxy, aryloxy, hétéroaryloxy, amino-C₁₋₃-alkyloxy-, C₁₋₃-alkylamino-C₁₋₃-alkyloxy, di-(C₁₋₃-alkyl)-amino-C₁₋₃-alkyloxy, ω-hydroxy-C₂₋₃-alcoxy, amino, C₁₋₃-alkylamino, di-(C₁₋₃-alkyl)-amino, C₁₋₃-alkyl-carbonylamino, hétéroaryl-carbonylamino, N-(C₁₋₃-alkyl)-N-(C₁₋₃-alkyl-carbonyl)-amino, N-(C₁₋₃-alkyl)-N-(hétéroaryl-carbonyl)-amino, C₁₋₄-alcoxy-carbonylamino, N-(C₁₋₃-alkyl)-N-(C₁₋₄-alcoxy-carbonyl)-amino, arylcarbonylamino, N-(C₁₋₃-alkyl)-N-(arylcarbonyl)-amino, (ω-hydroxy-C₂₋₃-alkyl)-amino, di-(ω-hydroxy-C₂₋₃-alkyl)-amino, arylamino, hétéroarylamino, C₁₋₃-alkylsulfonylamino, N-(C₁₋₃-alkyl)-N-(C₁₋₃-alkylsulfonyl)-amino, C₁₋₃-alcoxy-carbonyl-C₁₋₃-alkyl-carbonylamino, (C₁₋₃-alkyl-amino)-carbonyl-amino, [di-(C₁₋₃-alkyl)-amino]-carbonyl-amino, (C₁₋₃-alkyl-amino)-C₁₋₃-alkyl-carbonylamino, [di-(C₁₋₃-alkyl)-amino]-C₁₋₃-alkyl-carbonyl-amino, N-(aryl-C₁₋₃-alkyl-carbonyl)-N-(C₁₋₃-alkyl)-amino, N-(C₁₋₃-alkyl)-N-[(C₁₋₃-alkyl-amino)-carbonyl]-amino ou N-(C₁₋₃-alkyl)-N-{[di-(C₁₋₃-alkyl)-amino]-carbonyl}-amino et éventuellement en outre à partir de la position 1 par un groupe carboxy, C₁₋₄-alcoxy-carbonyle, C₁₋₃-alkylaminocarbonyle, di-(C₁₋₃-alkyl)-aminocarbonyle, arylaminocarbonyle, hétéroarylaminocarbonyle, di-(ω-hydroxy-C₂₋₃-alkyl)-aminocarbonyle, aryle ou hétéroaryle ou par un groupe cycloalkylèneimino ou cycloalkylèneiminocarbonyle à 5 à 7 chaînons,
où, dans le groupe cycloalkylèneimino ou cycloalkylèneiminocarbonyle cité ci-dessus, un groupe méthylène en position 3 ou 4 peut être remplacé par un groupe -NH-, -N(C₁₋₃-alkyl)- ou -N(C₁₋₃-alcoxy-carbonyl)- ou par un atome d'oxygène ou de soufre et/ou en position 2, 3 ou 4 par un groupe carbonyle, un groupe C₂₋₄-alcényle ou C₂₋₄-alcynyle, où au moins un atome de carbone hybridé sp³ se trouve entre l'atome d'azote auquel est lié R³ et la liaison multiple et le groupe alcényle ou alcynyle peut être substitué par un à trois groupes C₁₋₃-alkyle,
un groupe C₁₋₅-alkyle où le groupe méthylène qui est voisin de l'atome d'azote auquel est lié R³ peut être remplacé par un groupe -NH-, ou un groupe -N(C₁₋₃-alkyl)- ou par un atome d'oxygène,
un groupe C₁₋₄-alkyloxy ou
un groupe amino qui peut être substitué par un ou deux groupes C₁₋₃-alkyle, aryle, aryl-C₁₋₃-alkyle, C₁₋₃-alkyl-carbonyle, C₁₋₃-alkyloxy-carbonyle, arylcarbonyle ou aryl-C₁₋₃-alkyl-carbonyle, où les substituants peuvent être identiques ou différents, où les groupes alkyle cités précédemment peuvent être linéaires ou ramifiés, leurs tautomères, énantiomères, diastéréoisomères, leurs mélanges et leurs sels.

2. Composés de formule générale I selon la revendication 1 où
R² et R³ sont définis comme indiqué dans la revendication 1 et
R¹ représente un groupe méthyle, éthyle, n-propyle, isopropyle, n-pentyle, trifluorométhyle ou phényle, leurs tautomères, énantiomères, diastéréoisomères, leurs mélanges et leurs sels.

3. Composés de formule générale I selon la revendication 2 où
R¹ représente un groupe méthyle ou éthyle,
R² représente un groupe éthyle, propyle, butyle ou pentyle,
un groupe pyridinyle, furanyle ou pyrazinyle,
un groupe phényle où deux atomes de carbone voisins sont liés entre eux via un groupe méthylènedioxy, éthylènedioxy ou difluorométhylènedioxy,
ou
un groupe phényle qui peut être substitué par un ou deux atomes de fluor, de chlore, de brome ou d'iode ou par un ou deux groupes C₁₋₃-alkyle, nitro, cyano, amino, C₁₋₃-alkylcarbonylamino, phénylcarbonylamino, C₁₋₃-alkylsulfonylamino, trifluorométhyle, carboxy, C₁₋₃-alcoxy, di-(C₁₋₃-alkyl)-amino-C₁₋₃-alkyloxy, C₁₋₃-alcoxy-carbonyle, C₁₋₃-alkylaminocarbonyle, hydroxycarbonyl-C₁₋₃-alkyl-aminocarbonyle, C₁₋₃-alcoxycarbonyl-C₁₋₃-alkyl-aminocarbonyle, di-(C₁₋₃-alkyl)-amino-C₁₋₃-alkylaminocarbonyle, carboxy-C₁₋₃-alkyle, C₁₋₃-alcoxy-carbonyl-C₁₋₃-alkyle, amino-C₁₋₃-alkyle ou C₁₋₃-alkyl-carbonylamino-C₁₋₃-alkyle, où les substituants sont identiques ou différents, et
R³ représente un atome d'hydrogène,
un groupe C₁₋₆-alkyle linéaire ou ramifié qui peut être substitué par un groupe carboxy, C₁₋₄-alcoxy-carbonyle, phényle, pyridinyle, indolyle, imidazolyle, C₁₋₃-alkylaminocarbonyle, di-(C₁₋₃-alkyl)aminocarbonyle, phénylaminocarbonyle, pyridinylaminocarbonyle, di-(ω-hydroxy-C₂₋₃-alkyl)-aminocarbonyle ou par un groupe cycloalkylèneimino ou cycloalkylèneiminocarbonyle à 5 à 7 chaînons,
où le groupe phényle cité ci-dessus peut être substitué éventuellement par un groupe nitro, cyano, C₁₋₃-alkyloxy, [di-(C₁₋₃-alkyl)-amino]-C₁₋₃-alkyloxy, amino-C₁₋₃-alkyle ou C₁₋₄-alcoxy-carbonylamino-C₁₋₃-alkyle et
où dans le groupe cycloalkylèneimino et cycloalkylèneiminocarbonyle cité ci-dessus, un groupe méthylène en position 3 ou 4 peut être remplacé par un groupe -NH-, -N(C₁₋₃-alkyl)- ou -N(C₁₋₃-alcoxy-carbonyl)- ou par un atome d'oxygène ou de soufre et/ou en position 2, 3 ou 4 par un groupe carbonyle, un groupe C₂₋₆-alkyle linéaire ou ramifié, qui peut être substitué à partir de la position 2 par un groupe hydroxy, C₁₋₃-alcoxy, ω-hydroxy-C₂₋₃-alcoxy, amino, C₁₋₃-alkylamino, di-(C₁₋₃-alkyl)-amino, C₁₋₃-alkyl-carbonylamino, N-(C₁₋₃-alkyl)-N-(C₁₋₃-alkyl-carbonyl)-amino, C₁₋₄-alcoxy-carbonylamino, N-(C₁₋₃-alkyl)-N-(C₁₋₄-alcoxy-carbonyl)-amino, phénylcarbonylamino, N-(C₁₋₃-alkyl)-N-(phénylcarbonyl)-amino, di-(ω-hydroxy-C₂₋₃-alkyl)-amino, pyridinylamino, nitro-pyridinylamino, chloro-trifluorométhyl-pyridinylamino, C₁₋₃-alkylsulfonylamino, N-(C₁₋₃-alkyl)-N-(C₁₋₃-alkylsulfonyl)-amino ou C₁₋₃-alcoxy-carbonyl-C₁₋₃-alkyl-carbonylamino et éventuellement en outre à partir de la position 1 par un groupe carboxy, C₁₋₄-alcoxy-carbonyle, phényle, pyridinyle, imidazolyle, C₁₋₃-alkylaminocarbonyle, di-(C₁₋₃-alkyl)-aminocarbonyle, phénylaminocarbonyle, di-(ω-hydroxy-C₂₋₃-alkyl)-aminocarbonyle ou par un groupe cycloalkylèneimino ou cycloalkylèneiminocarbonyle à 5 à 7 chaînons,
où, dans le groupe cycloalkylèneimino ou cycloalkylèneiminocarbonyle cité ci-dessus, un groupe méthylène en position 3 ou 4 peut être remplacé par un groupe -NH-, -N(C₁₋₃-alkyl)- ou -N(C₁₋₃-alcoxy-carbonyl)- ou par un atome d'oxygène ou de soufre et/ou en position 2, 3 ou 4 par un groupe carbonyle, et où les groupes phényle cités ci-dessus peuvent éventuellement être substitués par un groupe amino-C₁₋₃-alkyle ou C₁₋₄-alcoxy-carbonylamino-C₁₋₃-alkyle, ou
un groupe propényle ou propynyle, leurs tautomères, énantiomères, diastéréoisomères, leurs mélanges et leurs sels.

4. Composés de formule générale I selon la revendication 3 où
R¹ représente un groupe méthyle,
R² représente un groupe éthyle, propyle, butyle ou pentyle,
un groupe phényle où deux atomes de carbone voisins sont liés entre eux via un groupe méthylènedioxy, éthylènedioxy ou difluorométhylènedioxy,
ou
un groupe phényle qui peut être substitué par un ou deux atomes de fluor, de chlore ou de brome ou par un ou deux groupes trifluorométhyle, nitro, cyano, C₁₋₃-alcoxy, carboxy-C₁₋₃-alkyle, C₁₋₃-alcoxy-carbonyl-C₁₋₃-alkyle, amino-C₁₋₃-alkyle
ou C₁₋₃-alkyl-carbonylamino-C₁₋₃-alkyle, où les substituants sont identiques ou différents, et
R³ représente un groupe C₁₋₅-alkyle linéaire ou ramifié qui peut être substitué par un groupe carboxy, C₁₋₄-alcoxy-carbonyle, C₁₋₃-alkylaminocarbonyle, di-(C₁₋₃-alkyl)aminocarbonyle, phénylaminocarbonyle, pyridinylaminocarbonyle, di-(2-hydroxy-éthyl)-aminocarbonyle, pipérazinylcarbonyle, 4-(C₁₋₃-alkyl)-pipérazinylcarbonyle, 4-(C₁₋₄-alcoxy-carbonyl)-pipérazinyl-carbonyle, phényle, pyridinyle ou imidazolyle,
où le groupe phényle peut éventuellement être substitué par un groupe nitro, cyano, C₁₋₃-alkyloxy, [di-(C₁₋₃-alkyl)-amino]-C₁₋₃-alkyloxy, amino-C₁₋₃-alkyle ou C₁₋₄-alcoxy-carbonylamino-C₁₋₃-alkyle,
un groupe C₂₋₅-alkyle, qui peut être substitué en position terminale par un groupe hydroxy, C₁₋₃-alcoxy, phényloxy, 2-hydroxy-éthoxy, amino, C₁₋₃-alkylamino, di-(C₁₋₃-alkyl)-amino, C₁₋₃-alkyl-carbonylamino, N-(C₁₋₃-alkyl)-N-(C₁₋₃-alkyl-carbonyl)-amino, C₁₋₄-alcoxy-carbonylamino, N-(C₁₋₃-alkyl)-N-(C₁₋₄-alcoxy-carbonyl)-amino, phénylcarbonylamino, N-(C₁₋₃-alkyl)-N-(phénylcarbonyl)-amino, pyridinylamino, nitro-pyridinylamino, di-(2-hydroxy-éthyl)-amino, 3-chloro-5-trifluorométhyl-pyridin-2-yl-amino, C₁₋₃-alkylsulfonylamino, N-(C₁₋₃-alkyl)-N-(C₁₋₃-alkylsulfonyl)-amino, C₁₋₃-alcoxy-carbonyl-C₁₋₃-alkyl-carbonylamino, indolyle, pyrrolidinyle, 2-oxo-pyrrolidinyle, morpholinyle, pipérazinyle ou 4-(C₁₋₃-alkyl)-pipérazinyle et éventuellement en outre à partir de la position 1 par un groupe carboxy, C₁₋₄-alcoxy-carbonyle, C₁₋₃-alkylaminocarbonyle, di-(C₁₋₃-alkyl)-aminocarbonyle, phénylaminocarbonyle, di-(2-hydroxy-éthyl)-aminocarbonyle, pipérazinylcarbonyle, 4-(C₁₋₃-alkyl)-pipérazinyl-carbonyle, 4-(C₁₋₄-alcoxy-carbonyl)-pipérazinyl-carbonyle, phényle, pyridinyle ou imidazolyle, ou un groupe amino qui peut être substitué par un ou deux groupes C₁₋₃-alkyle, aryle, aryl-C₁₋₃-alkyle, C₁₋₃-alkyl-carbonyle, C₁₋₄-alkyloxy-carbonyle, arylcarbonyle ou aryl-C₁₋₃-alkyl-carbonyle, où les substituants peuvent être identiques ou différents, où les groupes alkyle cités ci-dessus peuvent être linéaires ou ramifiés, leurs tautomères, énantiomères, diastéréoisomères, leurs mélanges et leurs sels.

5. Composés de formule générale I selon la revendication 4 où
R¹ représente un groupe méthyle,
R² représente un groupe éthyle, propyle, butyle ou pentyle,
un groupe phényle où deux atomes de carbone voisins peuvent être liés entre eux via un groupe méthylènedioxy ou éthylènedioxy, ou
un groupe phényle et
R³ représente un groupe C₁₋₄-alkyle qui peut être substitué en position terminale par un groupe C₁₋₄-alcoxy-carbonyle, ou
un groupe C₂₋₄-alkyle qui est substitué en position terminale par un groupe C₁₋₃-alkyl-carbonylamino, phénylcarbonylamino, di-(C₁₋₃-alkyl)-amino, phényle, pyridinyle ou C₁₋₃-alkylsulfonylamino,
où les groupes alkyle cités ci-dessus peuvent être linéaires ou ramifiés, leurs tautomères, énantiomères, diastéréoisomères, leurs mélanges et leurs sels.

6. Composés de formule générale I selon la revendication 1 suivants :
(a) 5-acétyl-3-[3-(méthoxycarbonylpropylamino)-(benzo[1,3]dioxyo-5-yl)-méthylidène]-2-indolinone 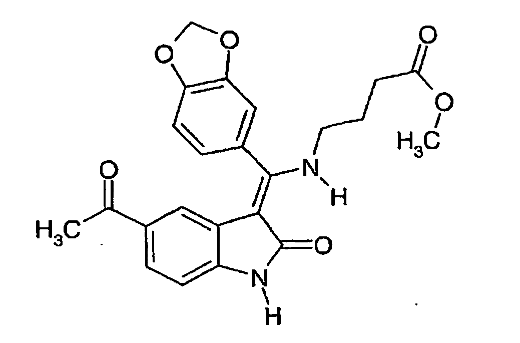
(b) 5-acétyl-3-[isopropylamino-phényl-méthylidène]-2-indolinone 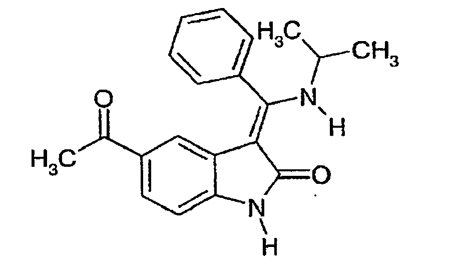
(c) 5-acétyl-3-[propylamino-phényl-méthylidène]-2-indolinone 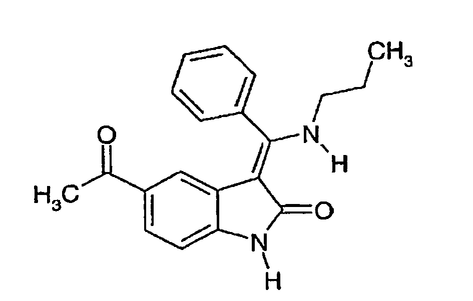
(d) 5-acétyl-3-[(3-méthoxycarbonyl-propylamino)-(2,3-dihydro-benzo[1,4]dioxin-6-yl)-méthylidène]-2-indolinone 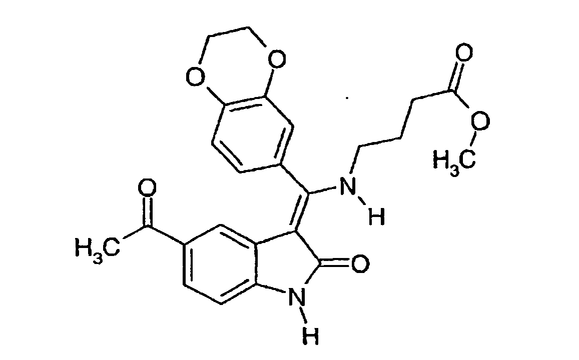
(e) 5-acétyl-3-[(benzo[1,3]dioxol-5-yl)-(3-(benzoylamino-propylamino)-méthylidène]-2-indolinone 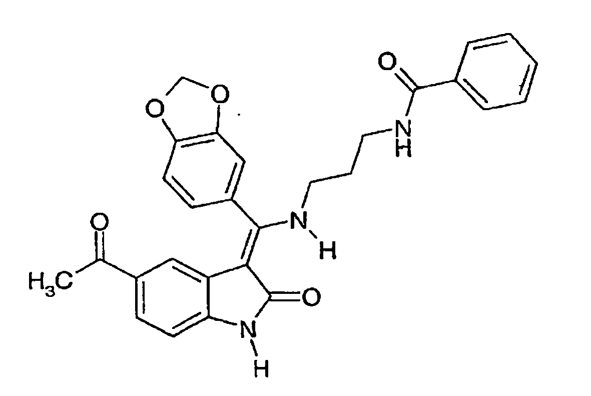
(f) 5-acétyl-3-[(benzo[1,3]dioxol-5-yl)-(3-(butyrylamino-propylamino)-méthylidène]-2-indolinone 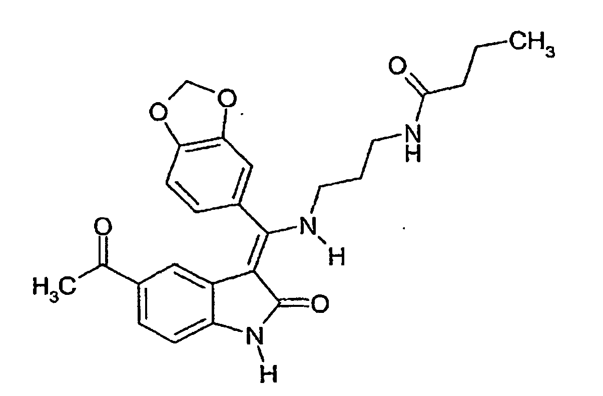
(g) 5-acétyl-3-[(3-méthanesulfonylamino-propylamino)-phényl-méthylidène]-2-indolinone 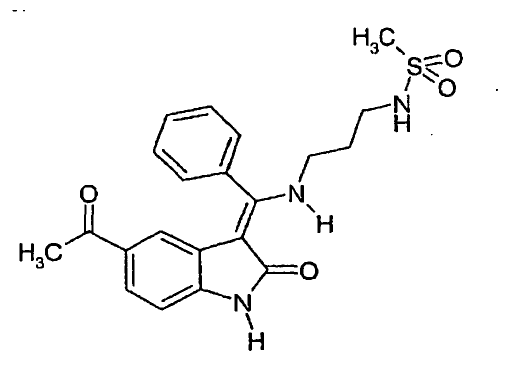
(h) 5-acétyl-3-[1-(3,4-difluorophényl)-1-(N',N'-diméthylhydrazino)-méthylidène]-2-indolinone 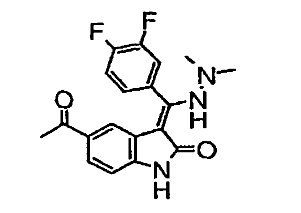
(i) 5-acétyl-3-[1-(4-diméthylamino-butyl)-buténylidène]-2-indolinone 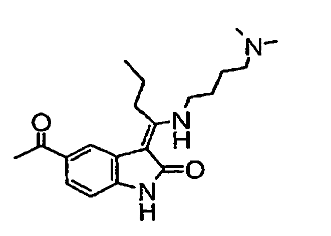
(j) 5-acétyl-3-[1-phényl-1-(3-pyridin-3-yl-propylamino)-méthylidène]-2-indolinone 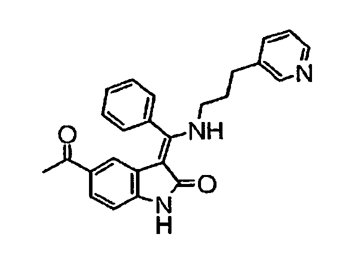 ainsi que leurs tautomères, énantiomères, diastéréoisomères, leurs mélanges et leurs sels.

7. Sels physiologiquement acceptables des composés selon au moins l'une des revendications 1 à 6 avec des acides ou bases inorganiques ou organiques.

8. Médicament contenant un composé selon au moins l'une des revendications 1 à 6 ou un sel physiologiquement acceptable selon la revendication 7 outre éventuellement un ou plusieurs vecteurs et/ou diluants inertes.

9. Utilisation d'un composé selon au moins l'une des revendications 1 à 7 pour la production d'un médicament qui convient pour le traitement du diabète sucré de type I et de type II, des troubles associés au diabète comme la neuropathie diabétique, et des maladies neurologiques dégénératives comme la maladie d'Alzheimer, de l'apoplexie cérébrale, des lésions neurotraumatiques et des troubles bipolaires.

10. Procédé de production d'un médicament selon la revendication 8 **caractérisé en ce que,** par voie non chimique, un composé selon au moins l'une des revendications 1 à 7 est incorporé dans un ou plusieurs vecteurs et/ou diluants inertes.

11. Procédé de production des composés de formule générale I selon les revendications 1 à 7 **caractérisé en ce que**
a) un composé de formule générale 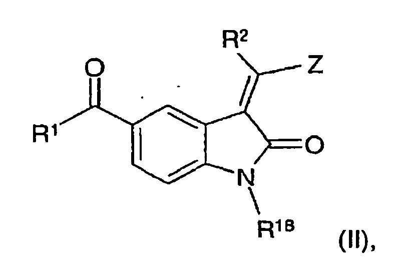 où R¹ et R² sont définis comme indiqué dans les revendications 1 à 6,
R¹⁸ représente un atome d'hydrogène ou un groupe protecteur pour l'atome d'azote du groupe lactame et Z représente un groupe partant comme par exemple un atome d'halogène, un groupe hydroxy, alcoxy, alkylsulfonyle, aralkylsulfonyle ou aryl-alcoxy, par exemple un atome de chlore ou de brome, un groupe méthoxy, éthoxy, méthanesulfonyle, toluènesulfonyle ou benzyloxy,
est mis à réagir avec une amine de formule générale
R³-NH₂ (III)
où R³ est défini comme indiqué dans l'une des revendications 1 à 6, où les groupes hydroxy, amino ou imino éventuellement contenus dans les groupements R² et/ou R³ peuvent être protégés provisoirement par des groupes protecteurs appropriés,
b) pour la production d'un composé de formule I qui contient un groupe aminocarbonyle, un composé qui contient un groupe carboxy est mis à réagir avec l'amine correspondante,
c) pour la production d'un composé de formule I qui contient un groupe carbonylamino, un composé qui contient un groupe amino est mis à réagir avec le chlorure d'acide correspondant,
d) pour la production d'un composé de formule I qui contient un groupe aminométhyle, un composé qui contient un groupe cyano est hydrogéné en le dérivé aminométhyle correspondant,
e) pour la production d'un composé de formule I qui contient un groupe amino, un composé qui contient un groupe nitro est hydrogéné, et/ou
ensuite, les groupes protecteurs éventuellement utilisés pendant la réaction sont clivés et/ou
les composés de formule générale I ainsi obtenus sont séparés en leurs énantiomères et/ou diastéréoisomères et/ou
les composés de formule I obtenus sont convertis en leurs sels, en particulier pour l'utilisation pharmaceutique en leurs sels physiologiquement acceptables avec des acides ou bases inorganiques ou organiques.
